# EUROPEAN PATENT SPECIFICATION

(11) **EP 4 396 227 B1**
(45) Date of publication and mention of the grant of the patent: **09.07.2025**
(21) Application number: 22776885.0
(22) Date of filing: 02.09.2022
(51) Int. Cl.: C07K 16/24, C07K 16/44, C07K 16/46, A61K 39/395, A61K 31/00, A61P 37/06, A61P 35/00, A61P 13/12, A61K 49/00, A61K 51/04, A61K 47/68

(54) **IMPROVED FC SILENCED ANTI-OXMIF ANTIBODIES WITH REDUCED AGGREGATION POTENTIAL AND REDUCED HYDROPHOBICITY**
VERBESSERTE FC-AUSGESCHALTETE ANTI-OXMIF-ANTIKÖRPER MIT REDUZIERTEM AGGREGATIONSPOTENZIAL UND REDUZIERTER HYDROPHOBIZITÄT
ANTICORPS ANTI-OXMIF SILENCIEUX FC AMÉLIORÉS AVEC RÉDUCTION DU POTENTIEL D'AGRÉGATION ET DE L'HYDROPHOBICITÉ

(30) Priority: 03.09.2021 EP 21194701; 22.10.2021 EP 21204108; 07.02.2022 EP 22155394
(43) Date of publication of application: 10.07.2024
(73) Proprietor: OncoOne Research & Development GmbH, 3400 Klosterneuburg (AT)
(72) Inventor: SCHINAGL, Alexander, 1200 Vienna (AT); KERSCHBAUMER, Randolf, 3400 Klosterneuburg (AT); THIELE, Michael Robert, 2201 Seyring (AT)
(74) Representative: Loidl, Manuela Bettina
(86) International application number: PCT/EP2022/074449
(87) International publication number: WO 2023/031397

(56) References cited:
- WO-A1-2009/086920
- WO-A1-2017/178493
- WO-A1-2019/234241
- WO-A1-2022/167474
- US-A1- 2016 287 732
- MOHAMED ALTAI ET AL: "Pretargeted Imaging and Therapy", THE JOURNAL OF NUCLEAR MEDICINE, vol. 58, no. 10, 7 July 2017 (2017-07-07), US, pages 1553 - 1559, XP055764118, ISSN: 0161-5505, DOI: 10.2967/jnumed.117.189944
- H. KARACAY ET AL: "Pretargeted Radioimmunotherapy of Pancreatic Cancer Xenografts: TF10-90Y-IMP-288 Alone and Combined with Gemcitabine", THE JOURNAL OF NUCLEAR MEDICINE, vol. 50, no. 12, 30 November 2009 (2009-11-30), pages 2008 - 2016, XP055186129, ISSN: 0161-5505, DOI: 10.2967/jnumed.109.067686
- RIJ CATHARINA M VAN ET AL: "Pretargeted immuno-PET and radioimmunotherapy of prostate cancer with an anti-TROP-2 x anti-HSG bispecific antibody", EUROPEAN JOURNAL OF NUCLEAR MEDICINE AND MOLECULAR IMAGING, SPRINGER BERLIN HEIDELBERG, BERLIN/HEIDELBERG, vol. 40, no. 9, 15 May 2013 (2013-05-15), pages 1377 - 1383, XP035341747, ISSN: 1619-7070, [retrieved on 20130515], DOI: 10.1007/S00259-013-2434-7
- "CHAPTER 10: Antibody Fc engineering for optimal antibody performance ED - William R Strohl; Lila M Strohl", 1 January 2012, THERAPEUTIC ANTIBODY ENGINEERING (IN: WOODHEAD PUBLISHING SERIES IN BIOMEDICINE), WP, WOODHEAD PUBLISHING, PAGE(S) 225 - 249, ISBN: 978-1-908818-09-6, XP009181736

## Description

### FIELD OF THE INVENTION

The invention refers to Fc silenced anti-oxMIF antibodies with improved properties such as reduced aggregation potential and reduced hydrophobicity due to selected amino acid substitutions in the light and heavy chain variable domains, and their use in the treatment of oxMIF-related conditions.

### BACKGROUND OF THE INVENTION

The cytokine Macrophage Migration Inhibitory Factor (MIF) has been described as early as 1966 (David, J.R., 1966; Bloom B.R. and Bennet, B., 1966). The biochemical properties and physiological role of MIF were elucidated following its cloning and recombinant expression (Bernhagen et al., 1993; Bernhagen et al., 1994). It is now well accepted that MIF is a pivotal regulator of innate immunity, playing a central role in inflammatory responses and cancer. MIF promotes the production of other proinflammatory mediators, such as TNF (Calandra et al., 1994), nitric oxide (Bernhagen et al., 1994), and prostaglandin E2 (Mitchell et al., 1999; Sampey et al., 2001). One of its most striking properties is the ability to override the immunosuppressive effects of glucocorticoids (GCs). In vitro, MIF counteracts the GC-induced inhibition of cytokine secretion in monocytes (TNF, IL-1, IL-6, and IL-8) (Calandra et al., 1995) and T-cells (Bacher et al., 1996) and overrides dexamethasone suppression of TNF-induced arachidonic acid release in fibroblasts (Mitchell et al., 1999). In vivo studies revealed that MIF increases the mortality of endotoxemic mice treated with dexamethasone (Calandra et al., 1995). The most detailed data for the upregulation of MIF serum levels and its association with disease were described in patients with severe sepsis (Emonts et al., 2007; Bozza et al., 2004; Sprong et al., 2007). Plasma levels of MIF correlated with disease severity and a state of shock and were significantly higher in patients who died than in those who survived. MIF concentrations significantly correlated with elevated plasma concentrations of IL-1, IL-6, IL-10, IL-12, and cortisol. Elevated MIF levels in patients have furthermore been determined for numerous inflammatory diseases, e.g. rheumatoid arthritis (Onodera et al., 1999; Morand et al., 2002), Crohn's disease (de Jong et al., 2001), psoriasis (Shimizu et al., 2001), and multiple sclerosis (Niino et al., 2000; Rinta et al., 2008). MIF furthermore contributes to the maintenance of the inflammatory processes by inhibiting p53-dependent cell death and stimulating the survival of monocytes and macrophages (Mitchell et al., 2002).

Mounting evidence suggests that inflammation is closely associated with many types of cancer. Inflammatory pathways designed to defend against infection and injury can promote an environment which facilitates tumor growth and metastasis (Conroy et al., 2010). Thus, inflammation is suggested to be a key driver of tumorigenesis, with many cancers arising as a result of infection or chronic inflammation (Bucala and Donnelly, 2007; Conroy et al., 2010; Karin, 2009). Further, it is well established that the inflammatory nature of the tumor microenvironment promotes angiogenesis and breakdown of the extracellular matrix (ECM), which in turn contribute to the survival, proliferation, and migration of tumor cells (Coussens and Werb, 2002; Hagemann and Balkwill, 2005; Hagemann et al., 2007; Kessenbrock et al., 2010). MIF has been shown to be up-regulated in a large variety of human neoplasms like pancreatic, breast, prostate, colon, brain, skin and lung-derived tumors (Bando et al., 2002; Chen et al., 2010; Kamimura et al., 2000; Meyer-Siegler and Hudson, 1996; Shimizu et al., 1999; Takahashi et al., 1998; Winner et al., 2007). Several studies report that MIF expression closely correlates with tumor aggressiveness and metastatic potential, suggesting that MIF may play an important role in disease severity and cell survival (Rendon et al., 2009). Recent data suggest that extracellular MIF may contribute to a more aggressive tumor phenotype as compared to intracellular MIF (Verjans et al., 2009). MIF contributes to a microenvironment that favors tumor growth, angiogenesis, invasiveness and metastasis. Besides its pro-inflammatory functions, MIF exerts anti-apoptotic and pro-proliferative effects, including inhibition of p53 (Hudson et al., 1999; Mitchell and Bucala, 2000) and activation of the central kinases ERK1/2 (Mitchell et al., 1999) and AKT (Lue et al., 2007). MIF further has been described as a pro-angiogenic factor promoting neo-angiogenesis (Coleman et al., 2008) and tumor vascularization by stabilizing HIF-1α (Winner et al., 2007) and up-regulation of pro-angiogenic factors like VEGF and IL-8 (Ren et al., 2004). MIF also acts as a chemokine and is expected to contribute to the inflammatory cell recruitment within the tumor environment via the chemokine receptors CXCR2 and CXCR4 (Bernhagen et al., 2007; Rendon et al., 2007).

MIF, however, is markedly different from other cytokines and chemokines because it is constitutively expressed and present in the circulation of healthy subjects. Pre-formed MIF is stored in cytoplasmic pools of macrophages, T-cells, and many other cells within the body, including the hypothalamic-pituitary-adrenal axis, allowing for rapid re-lease upon stimulation without de novo synthesis (Bernhagen et al., 1993; Bacher et al., 1997; Fingerle-Rowson et al., 2003).

Due to the ubiquitous nature of this protein, MIF can be considered as an inappropriate target for therapeutic intervention. However, MIF occurs in two immunologically distinct conformational isoforms, termed reduced MIF (redMIF) and oxidized MIF (oxMIF) (Thiele M. et al., 2015). RedMIF was found to be the abundantly expressed isoform of MIF that can be found in the cytoplasm and in the circulation of any subject. RedMIF seems to represent a latent non-active storage form (Schinagl. A. et al., 2018).

In contrast, oxMIF seems to be the physiologic relevant and disease related isoform which can be detected in tumor tissue, specifically in tumor tissue from patients with colorectal, pancreatic, ovarian and lung cancer outlining a high tumor specificity of oxMIF (Schinagl. A. et al., 2016), but also in the circulation and in the inflamed tissue of patients with inflammatory diseases (Thiele et al., 2015).

The number of successful drug targets to treat cancers, like the above mentioned oxMIF positive indications, is restricted. E.g., more than 300 potential immune-oncology targets are described, but many clinical studies focus on anti-PD-1 and anti-PD-L1 antibodies (Tang J., et al. 2018). The scientific and medical community therefore eagerly awaits potential drugs targeting tumor specific antigens to increase the therapeutic options for cancer patients with poor prognosis.

In addition, there is also a high demand on drugs targeting inflammatory diseases. Glucocorticoids (GCs) represent the most important and frequently used class of anti-inflammatory drugs in routine clinical practice (Schäcke et al., 2002). The utility of GC relates largely to their capacity to reduce the recruitment of inflammatory cells and suppress the pro-inflammatory cytokine and mediator synthesis, thereby effectively blocking the inflammatory cascade at many levels (Barnes et al., 2003). The prevalence of oral GC use has been estimated to be 0.5% of the general population and 1.4% of those older than 55 years (Ramsey-Goldman, 2002; Walsh et al., 1996). Although GCs are regarded clearly beneficial, substantial dose-dependent and often irreversible side-effects are recognized, which limit their use (Pisu et al., 2005). Side-effects of most concern include hypertension, obesity, osteoporosis, myopathy, oedema and immune compromise. Premature death related to atherosclerosis is also increasingly recognized in inflammatory diseases including RA and systemic lupus erythematosus (Wallberg-Jonsson et al., 2005; del Rincon et al., 2001; Solomon et al., 2003; EI-Magadmi et al., 2004; Manzi et al., 1999). The substantial burden of toxicity of GC mandates the development of therapies, which would facilitate the action of GC on inflammatory disease and allow dose reduction. This however requires the elucidation of factors that regulate GC sensitivity. In the early 2000s, a unique relationship between the cytokine macrophage migration inhibitory factors (MIFs) and GCs has been emerged, identifying MIF as a candidate factor that could regulate GC sensitivity (Aeberli et al., 2006).

Antibodies targeting oxMIF showed efficacy in *in vitro-* and *in vivo* models of inflammation and cancer (Hussain F. et al., 2013; Schinagl. A. et al., 2016; Thiele et al., 2015).

WO2019/234241A1 discloses anti-oxMIF/anti-CD3 bispecific antibodies.

WO2009/086920A1 describes the anti-oxMIF antibody Bax69 (Imalumab).

Protein aggregation, specifically antibody aggregation is frequently observed at several stages of bioprocessing, including protein expression, purification and storage. Antibody aggregation can affect the overall yield of therapeutic protein manufacturing processes and may contribute to stability and immunogenicity of therapeutic antibodies.

Protein aggregation of antibodies thus continues to be a significant problem in their developability and remains a major area of focus in antibody production. Antibody aggregation can be triggered by partial unfolding of its domains, leading to monomer-monomer association followed by nucleation and aggregate growth. Although the aggregation propensities of antibodies and antibody-based proteins can be affected by the external experimental conditions, they are strongly dependent on the intrinsic antibody properties as determined by their sequences and structures.

For example, resistance to aggregation can be achieved by stabilizing the native state (i.e., resisting unfolding) or by reducing the propensity of the unfolded or partially folded states of the protein to aggregate. A disadvantage of stabilizing the native state is that proteins will likely be exposed to an environment in which they will unfold. Generally, when a protein is denatured or unfolds, amino acid residues that normally mediate intramolecular contacts in the interior of the protein are exposed. Such exposure often makes proteins prone to form intermolecular contacts and aggregate. In contrast to proteins that resist unfolding, a protein having a reduced propensity to aggregate when unfolded will simply refold into a bioactive non-aggregated state after exposure to such an environment.

The aggregation-resistance or aggregation-propensity of antibodies and proteins comprising antigen binding domains thereof is usually limited by the most aggregation prone domain(s) contained therein and by the strength of its interaction with surrounding domains (if present).

This is because once that domain unfolds, if it is incapable of refolding, it may interact with other domains in the same protein or in other proteins and form aggregates. Constant domains of antibodies generally do not aggregate and do not vary considerably. Accordingly, the weakest domains of an antibody with regard to aggregation potential and stability are generally considered to be the variable domains (e.g., heavy chain variable domain (V_{H}) and/or light chain variable domain (V_{L}), Ewert S. et al., 2003). In this regard, incorporation of aggregation prone V_{H} or V_{L} domains into otherwise stable recombinant antibody products often imparts these generally undesirable traits to the new recombinant design. Thus, engineering a variable domain to be aggregation-resistant is most likely to render the entire protein which may comprise that variable domain aggregation-resistant. Various strategies have been proposed for reducing aggregation of variable domains, e.g., rational design of aggregation-resistant proteins, complementarity determining region (CDR) grafting, or introducing disulfide bonds into a variable domain. Rational design of aggregation-resistant proteins generally involves using in silico analysis to predict the effect of a point mutation on the aggregation propensity of a protein. However, there are several difficulties with this approach. For example, it is not sufficient to merely identify a mutation that is likely to reduce aggregation of an unfolded protein. Rather, the mutation must also not increase aggregation of a folded protein or affect the function of the folded protein, especially the binding properties such as affinity or specificity in case of antibodies. Furthermore, rational design requires detailed structural analysis of the specific protein being improved and thus, is difficult to use with a protein that has not been thoroughly characterized and is not readily applicable to a variety of different proteins. CDR grafting involves transplanting CDRs from one variable domain onto framework regions (FRs) of another variable domain. This strategy was shown to be useful in stabilizing an anti-EGP-2 scFv (Willuda J. et al., 1999). Disadvantages of this approach include the reduction in affinity that can occur following CDR grafting. This loss of affinity can be overcome by introducing mutations to the FRs, however such mutations can produce immunogenic epitopes in the protein, thereby making the protein undesirable from a therapeutic point of view. Furthermore, CDR grafting generally requires analysis of crystal structure or homology modeling of the donor and acceptor variable domains to assess suitability for grafting. Such an approach is laborious and requires specialized knowledge. Moreover, since each variable domain has a different structure, the method is not readily applied across a variety of molecules. As for methods involving introducing disulfide bonds into a variable domain, while the bond may assist in the protein correctly refolding, it also introduces rigidity into the variable domain. Such rigidity can reduce the affinity of an antibody for an antigen. Moreover, not all variable domains can support the introduction of the requisite cysteine residues for disulfide bond formation without loss of affinity or without introducing an immunogenic epitope. Furthermore, formation of disulfide bonds under high protein concentrations can lead to protein aggregation, thus negating any potential positive effect of the bond.

Reduction in aggregation propensity, however, has been shown to be accompanied by an increase in expression titer, showing that reducing protein aggregation is beneficial throughout the development process and can lead to a more efficient path to clinical studies. For therapeutic proteins, aggregates are a significant risk factor for deleterious immune responses in patients and can form via a variety of mechanisms. Controlling aggregation can improve protein stability, manufacturability, attrition rates, safety, formulation, titers, immunogenicity, and solubility (Wei Li et al., 2016; Van der Kant R. et al., 2017).

Further intrinsic properties of proteins such as hydrophobicity also play important roles in antibody solubility. Low solubility of these therapeutic proteins due to surface hydrophobicity has been shown to render formulation development more difficult and may lead to poor bio-distribution, undesirable pharmacokinetics behavior and immunogenicity *in vivo.* Decreasing the overall surface hydrophobicity of candidate monoclonal antibodies could thus provide benefits and cost savings relating to purification and dosing regimens.

Modulating antibody effector functions to suit specific therapeutic purposes is also of growing interest. Specifically, Fc-null or Fc-silenced antibodies may be a strategy to abrogate Fc effector function as strong immune effector functions through FcγR and complement interactions may sometimes be detrimental to antibody mechanisms. Likewise, modulating the neonatal Fc receptor (FcRn) binding of IgG antibodies in order to modulate pharmacokinetics has also gained increased notoriety.

Strohl W.R. et al. (2012) describe antibody Fc engineering to increase or decrease FcγR mediated activities of IgG isotypes.

WO2017/178493A1 describes antibodies targeting TIM-3 (T-cell immunoglobulin and mucin domain containing 3) comprising modified CH1-CH3 domains for Fc silencing. A combination of reduced aggregation potential, reduced hydrophobicity and Fc silencing would render the antibody properties highly advantageous. There is still an unmet need in the art for Fc silenced and aggregation-resistant antibodies against oxMIF with reduced hydrophobicity.

### SUMMARY OF THE INVENTION

It is the objective of the present invention to provide improved antibodies targeting oxMIF, with silenced Fc and having reduced aggregation propensity and hydrophobicity.

The objective is solved by the subject matter of the present invention.

The present invention discloses an Fc silenced anti-oxMIF antibody, comprising a variant Fc region of a wild-type human IgG comprising SEQ ID NO:1 (CH2-CH3) with one or more amino acid substitutions or glycosylation modifications, and the following variable domains:
(a1) a light chain variable domain comprising SEQ ID NO:2 with 1, 2, 3, 4, or 5 amino acid substitutions selected from M30L, F49Y, A51G, P80S, W93F, or
(a2) a light chain variable domain comprising SEQ ID NO:2 with 1, 2, 3, 4, or 5 amino acid substitutions selected from M30L, F49Y, A51G, P80S, W93F and with 1, 2, 3, 4, or 5 amino acid substitutions, with the proviso that the conserved tyrosine at position 36 is preserved, and
(b1) a heavy chain variable domain comprising SEQ ID NO:3; or
(b2) a heavy chain variable domain comprising SEQ ID NO:3 with 1 or 2 amino acid substitutions selected from L5Q and W97Y, or
(b3) a heavy chain variable domain comprising SEQ ID NO:3 with 1 or 2 amino acid substitutions selected from L5Q and W97Y, and 1, 2, 3, 4 or 5 further amino acid substitutions;
wherein amino acid positions are numbered according to Kabat,
wherein the variant Fc region exhibits decreased FcγR binding compared to the wildtype IgG1 Fc region, wherein the one or more amino acid substitutions in the variant Fc region are at any one of positions E233, L234, L235, G236, G237, P238, D265, S267, H268, N297, S298, T299, E318, L328, P329, A330, P331 of SEQ ID NO:1, specifically the amino acid substitutions are at positions L234 and L235, according to EU numbering index, and/or wherein the Fc region is aglycosylated, and
wherein said antibody has reduced aggregation potential and reduced hydrophobicity compared to an antibody comprising SEQ ID NO:2 and SEQ ID NO:3 lacking the amino acid substitutions.

Specifically, the recombinant anti-oxMIF antibody comprises the amino acid substitution W93F with reference to SEQ ID 2.

Specifically, the recombinant anti-oxMIF antibody comprises the amino acid substitution W97Y with reference to SEQ ID NO:3.

According to a further specific embodiment, the recombinant anti-oxMIF antibody comprises the amino acid substitutions W93F and W97Y.

More specifically, the amino acid substitutions in the Fc region are L234A and L235A of SEQ ID NO:1 according to EU numbering.

Specifically provided herein is an Fc silenced anti-oxMIF antibody comprising an Fc region comprising an amino acid sequence selected from the group consisting of SEQ ID NOs:4, 5, 6, 7, 8, 9,43, and 85.

More specifically, the Fc-silenced anti-oxMIF antibody described herein comprises the variable heavy and light chains
i.) SEQ ID NOs:3 and 6,
ii.) SEQ ID NOs:9 and 6,
iii.) SEQ ID NOs:4 and 6,
iv.) SEQ ID NOs:4 and 8,
v.) SEQ ID NOs:4 and 5, or
vi.) SEQ ID NOs:43 and any one of 5, 6, 7 or 8.

According to an embodiment, the Fc-silenced anti-oxMIF antibody described herein further comprises CH1 to CH3 domains of SEQ ID NO:14.

According to a specific embodiment of the invention, the anti-oxMIF antibody comprises an amino acid sequence selected from the group consisting of SEQ ID NOs:15 and 16 or 17.

According to a further embodiment, herein provided is an Fc silenced anti-oxMIF antibody, comprising a variant Fc region of a wild-type human IgG comprising SEQ ID NO:1 with one or more amino acid substitutions or glycosylation modifications, comprising
a light chain CDR1 sequence selected from SEQ ID NOs: 72 or 78, and
a light chain CDR2 sequence selected from SEQ ID NOs: 73, 79, 80, or 81, and
a light chain CDR3 sequence selected from SEQ ID NOs: 74 or 82, and
a heavy chain CDR1 sequence selected from SEQ ID NOs: 75 and
a heavy chain CDR2 sequence selected from SEQ ID NOs: 76 or 83, and
a heavy chain CDR3 sequence selected from SEQ ID NOs: 77 or 84, with the provision that SEQ ID 74 and SEQ ID 77 are not comprised together.

In an alternative embodiment, the Fc silenced anti-oxMIF antibody comprises
a light chain CDR1 sequence selected from SEQ ID NOs: 72 or 78, and
a light chain CDR2 sequence selected from SEQ ID NOs: 73, 79, 80, or 81, and
a light chain CDR3 sequence selected from SEQ ID NOs: 82, and
a heavy chain CDR1 sequence selected from SEQ ID NOs: 75 and
a heavy chain CDR2 sequence selected from SEQ ID NOs: 76 or 83, and
a heavy chain CDR3 sequence selected from SEQ ID NOs: 77 or 84.

In an alternative embodiment, the Fc silenced anti-oxMIF antibody comprises
a light chain CDR1 sequence selected from SEQ ID NOs: 72 or 78, and
a light chain CDR2 sequence selected from SEQ ID NOs: 73, 79, 80, or 81, and
a light chain CDR3 sequence selected from SEQ ID NOs: 82, and
a heavy chain CDR1 sequence selected from SEQ ID NOs: 75 and
a heavy chain CDR2 sequence selected from SEQ ID NOs: 76 or 83, and
a heavy chain CDR3 sequence selected from SEQ ID NOs: 84.

In an alternative embodiment, the Fc silenced anti-oxMIF antibody comprises
a light chain CDR1 sequence selected from SEQ ID NOs: 72 or 78, and
a light chain CDR2 sequence selected from SEQ ID NOs: 73, 79, 80, or 81, and
a light chain CDR3 sequence selected from SEQ ID NO: 82 and
a heavy chain CDR1 sequence selected from SEQ ID NOs: 75 or 83, and
a heavy chain CDR2 sequence selected from SEQ ID NOs: 76 or 84, and
a heavy chain CDR3 sequence selected from SEQ ID NO: 85

The Fc-silenced anti-oxMIF antibody described herein can be of any format which contains CH2 and CH3 antibody domains, specifically it is selected from the group consisting of monospecific, bispecific antibodies (e.g., Crossmab), scFv-Fc, (scFv)₂-Fc (two scFv fragments are comprised on one arm), scFv/scFv-Fc (i.e. each arm comprises an scFv fragment), Fab/scFv-Fc, Fab/(scFv)₂-Fc, Fab/Fab-scFv-Fc, IgG-scFv and IgG-(scFv)₂.

In a further embodiment, the Fc-silenced anti-oxMIF antibody described herein is a bispecific antibody further comprising at least one binding site specifically recognizing an epitope of CD3, or histamine-succinyl-glycine (HSG).In a specific embodiment, the Fc-silenced anti-oxMIF antibody is for use in the treatment or detection of solid tumors, wherein said antibody is administered to a subject in a first step and a HSG hapten is administered in a second step, wherein said HSG hapten binds to the antibody and is labelled with a radionuclide.

Herein provided is also the Fc-silenced antibody described herein for use in the preparation of a medicament.

In yet a further embodiment of the invention, herein provided is also a pharmaceutical composition comprising the antibody described herein, optionally together with a pharmaceutical carrier or adjuvant.

Specifically, the composition comprises 10 to 250 mg/ml of the inventive antibody, specifically comprising > 50 mg/ml.

More specifically, said pharmaceutical composition is formulated for subcutaneous administration.

As provided herein, the pharmaceutical composition is for administration as the sole substance, or for administration as combination formulation with a further pharmaceutical composition comprising one or more active substances, preferably selected from the group consisting of antiviral, anti-cancer, anti-inflammatory, and antibiotic substances.

In a further embodiment provided herein is the pharmaceutical composition for use in the treatment of a patient suffering from inflammatory disease, infectious disease, specifically in the treatment of asthma, vasculitis, arthritis, sepsis, septic shock, endotoxic shock, toxic shock syndrome, acquired respiratory distress syndrome, glomerulonephritis, inflammatory bowel disease, Crohn's disease, ulcerative colitis, peritonitis, nephritis, NASH (nonalcoholic steatosis hepatitis), multiple sclerosis, acute and chronic pancreatitis, Type 1 diabetes, IgA nephropathy, interstitial cystitis, post COVID syndrome and psoriasis.

According to an alternative embodiment, the pharmaceutical composition is for use in the treatment of a patient suffering from hyperproliferative disorder or cancer, specifically in the treatment of colorectal cancer, ovarian cancer, breast cancer, prostate cancer, pancreas cancer, gastric cancer, and lung cancer.

Further provided herein is an isolated nucleic acid encoding the antibody of the invention.

Also an expression vector is provided herein, comprising the nucleic acid molecule(s) described herein.

In a further embodiment, a host cell is provided, containing the nucleic acid or an expression vector described herein.

Further provided is a method of producing the antibody of the invention, comprising culturing a host cell and recovering said antibody from the cell culture.

Further provided herein is a method for producing an antibody of the invention, comprising expressing a nucleic acid encoding the antibody in a host cell.

### FIGURES

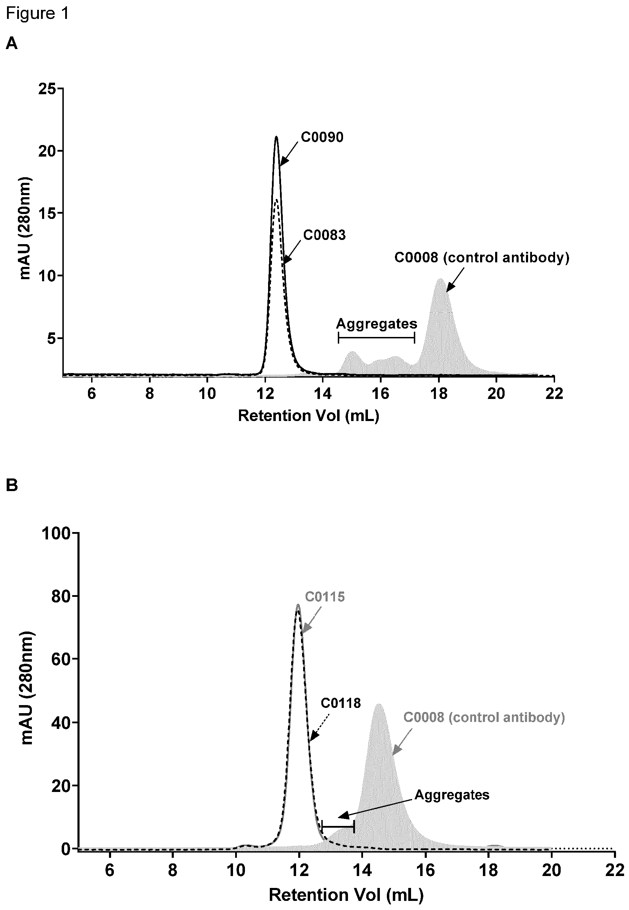

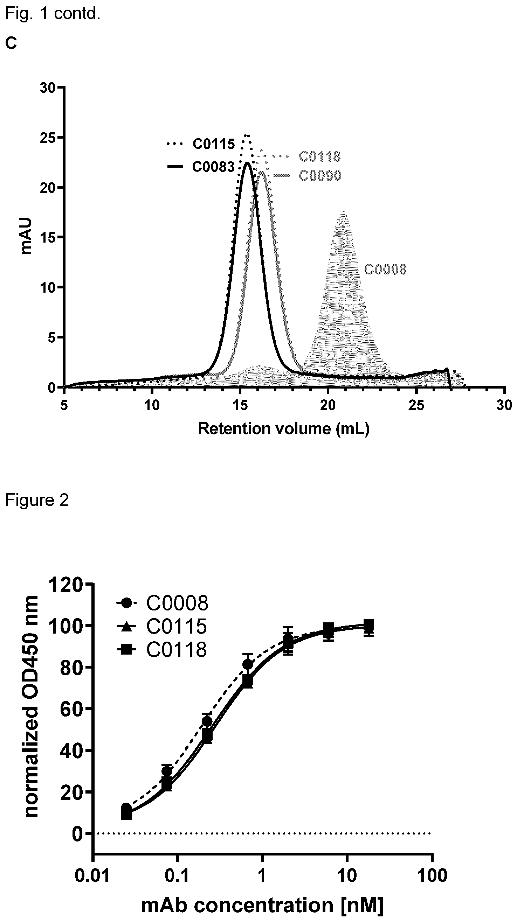

**Figure 1****:** Chromatography profiles demonstrating reduced aggregation and hydrophobicity of the newly designed anti-oxMIF antibodies. (A) Comparison of elution profiles of C0008 (control antibody, gray area) and the parent antibodies (C0083 and C0090, without Fc silencing) of the newly designed antibodies C0115 and C0118 on a Enrich 650 gel filtration column using 1xPBS as mobile phase; (B) Comparison of elution profiles of C0008 (control antibody, gray area) and newly designed antibodies C0115 and C0118 on a Enrich 650 gel filtration column using 1xPBS as mobile phase (C) Comparison of elution profiles of C0008 (control antibody, gray area) and newly designed antibodies C0115 and C0118 and their parent antibodies C0083 and C0090 (without Fc silencing) on a HiTrap Butyl HP HIC column.
**Figure 2****:** Binding curves of newly designed anti-oxMIF antibodies to immobilized oxMIF (K_{D} determination). Anti-oxMIF antibodies were detected by an anti-human-IgG (Fc)-HRP conjugate, C0008 was used as reference antibody. EC₅₀ values were determined by sigmoidal, 4-parameter equation using GraphPad Prism (mean +/- SEM of two experiments is shown)

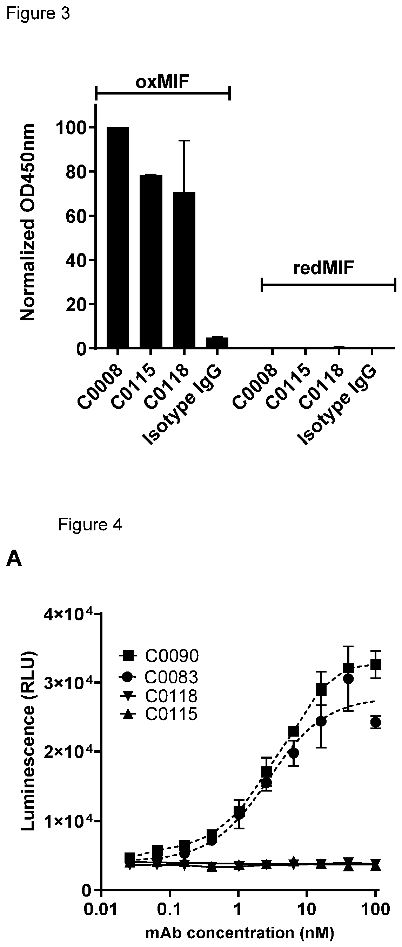

**Figure 3****:** Differential binding of the newly designed antibodies to oxMIF vs. redMIF. C0008 was used as reference antibody and an Isotype IgG as negative control. Mean +/- SEM of two to three experiments is shown.

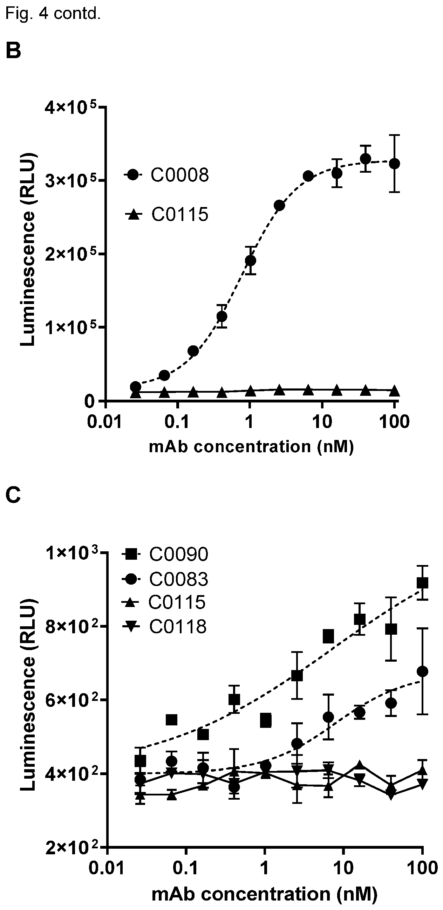

**Figure 4****:** Strongly reduced effector functions of the newly designed Fc silenced antibodies C0115 and C0118 determined by reporter assays. (A-B) ADCC reporter bioassay with the newly designed Fc silenced antibodies C0115 and/or C0118 using engineered Jurkat effector cells stably expressing FcyRllla (V: high responder genotype, F: low responder genotype) and HCT116-pMIF (A) or A2780-pMIF (B) target cells either compared to the anti-oxMIF control antibody C0008 (B) or their parent anti-oxMIF antibodies C0083 and C0090 with wt Fc (A); (C) ADCP reporter bioassay with the newly designed Fc silenced antibodies C0115 and/or C0118 using engineered Jurkat effector cells stably expressing FcyRlla and HCT116-pMIF target cells compared to their parent antibodies C0083 and C0090 with wt Fc. Data were fitted to a sigmoidal, 4-parameter equation using GraphPad Prism (mean +/- SD of two replicates is shown).

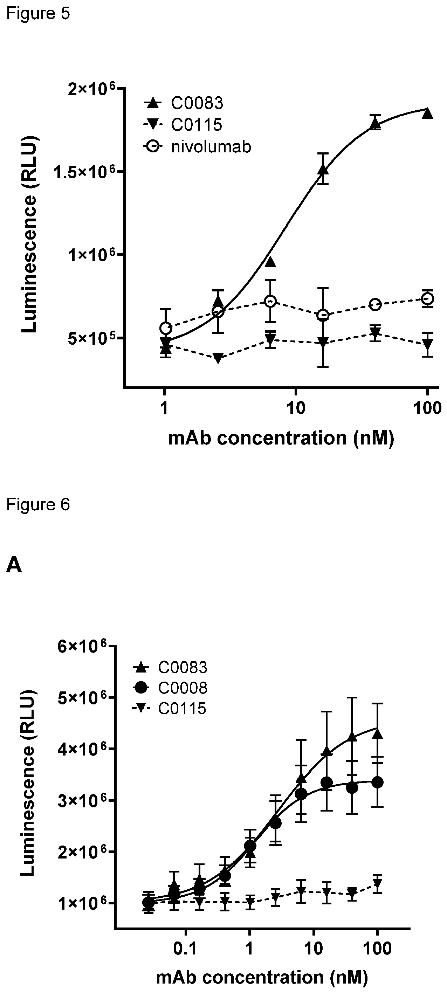

**Figure 5****:** Strongly reduced CDC activity of the newly designed Fc silenced antibody C0115 determined by a complement dependent cell toxicity bioassay. CDC bioassay with the newly designed Fc silenced antibody C0115 using BRC as source of complement and HCT116-HiBiT-pMIF as target cells compared to its parent anti-oxMIF antibody C0083 with a wt Fc and nivolumab as an IgG4 negative control. Data (where appropriate) were fitted to a sigmoidal, 4-parameter equation using GraphPad Prism (mean +/- SD of two replicates is shown).

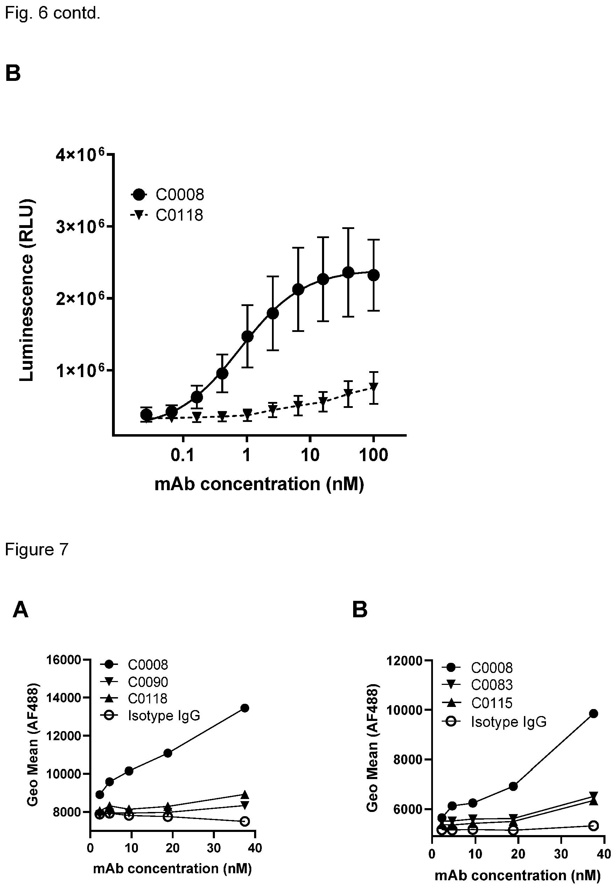

**Figure 6****:** Strongly reduced ADCC activity of the newly designed Fc silenced antibodies C0115 and C0118 determined by a PBMC mediated cell toxicity bioassay. ADCC bioassay with the newly designed Fc silenced antibodies C0115 (A) and C0118 (B) using PBMCs as effector cells and HCT116-HiBiT-pMIF as target cells compared to the anti-oxMIF control antibody C0008 or the parent anti-oxMIF antibody of C0115 (C0083) with wt Fc. Mean and SEM of two replicates using PBMCs from two healthy donors are shown. Data were fitted to a sigmoidal, 4-parameter equation using GraphPad Prism
**Figure 7****:** Reduced unspecific binding of the newly designed anti-oxMIF antibodies on A2780 MIF^{-/-} cells determined by FACS. Staining of A2780 MIF^{-/-} cells with the newly designed anti-oxMIF antibody C0118 and its parent antibody C0090 (A) and C0115 and its parent antibody C0083 (B) and the control antibody C0008 as well as an isotype IgG as negative control; GeoMean (mean fluorescence intensity for AF488) of viable cells is plotted against antibody concentration.

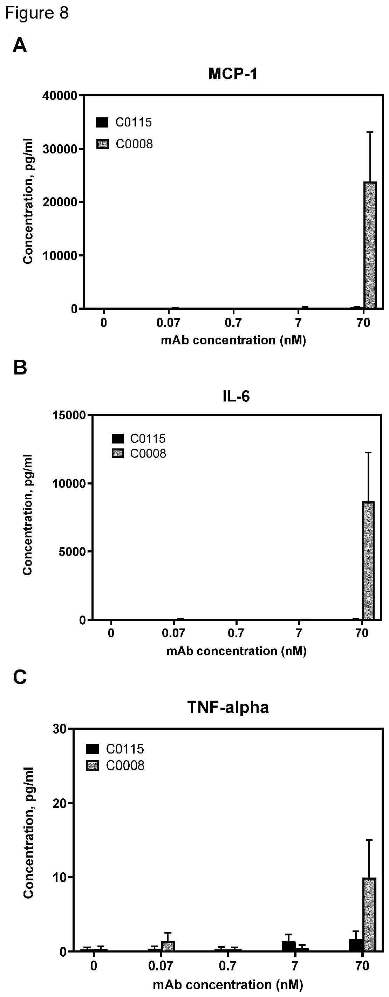

**Figure 8****:** The newly designed Fc silenced anti-oxMIF antibody C0115 shows strongly reduced cytokine release from human PBMCs compared to reference antibody C0008. The anti-oxMIF antibody C0115 carrying Fc-silencing mutations, and the reference antibody C0008 were incubated with human PBMCs overnight, and supernatants were analyzed in LegendPlex cytometric bead assays (BioLegend) for human MCP-1 **(A),** human IL-6 **(B),** and human TNF-α **(C).** Mean +/-SEM for cytokine concentrations (in pg/ml) are shown from 3 different PBMC donors.

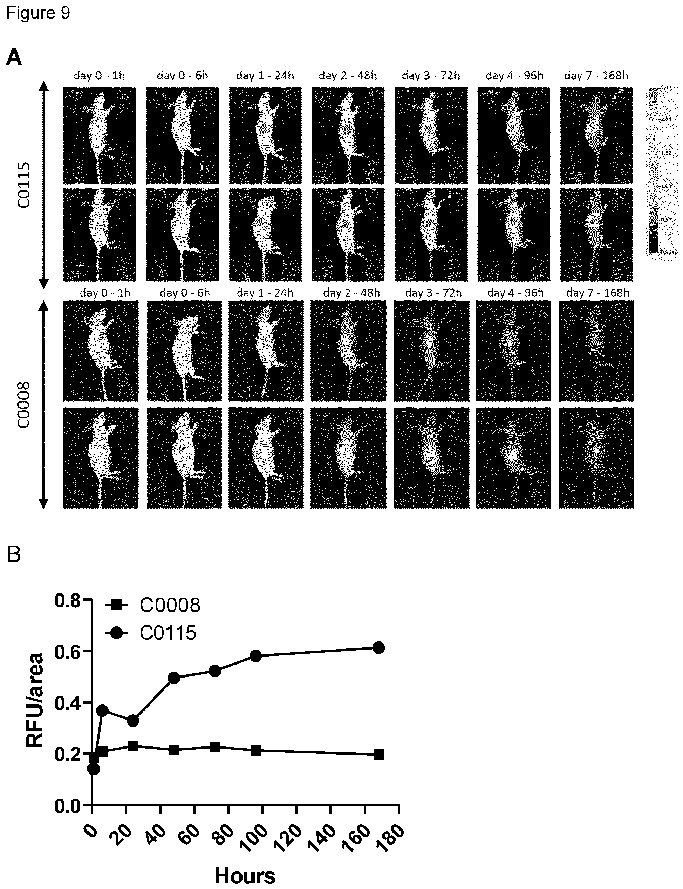

**Figure 9****:** Tumor penetration and retention of the newly designed Fc-silenced anti-oxMIF antibody C0115 and the reference antibody C0008 by infra-red *in vivo* imaging of mice carrying subcutaneous HCT116 tumors. (A) Infra-red images of mice were taken 1h, 6h, 24h, 48h, 72h, 96h and 168h post injection of the IRDye 800CW labeled antibodies C0115 (upper panel) and C0008 (lower panel) dosed at 5 mg/kg; (B) tumor penetration and retention of C0115 and C0008 quantified by digital image analysis. Mean of 3 mice is show.

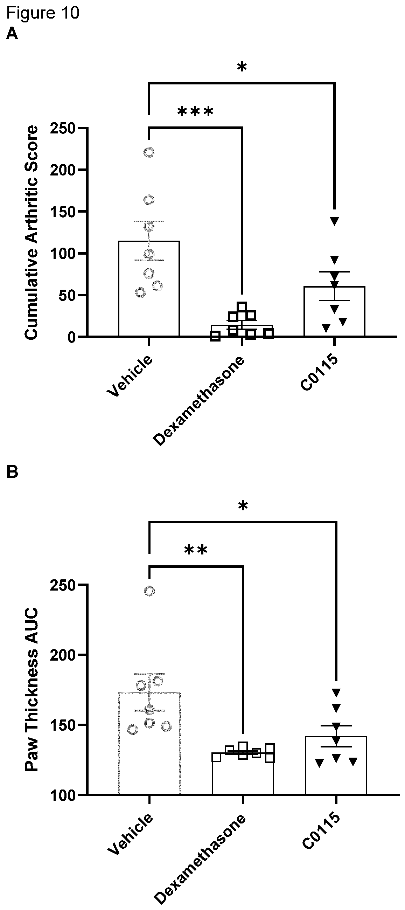

**Figure 10****:** The newly designed Fc-silenced anti-oxMIF antibody C0115 ameliorates disease severity in a collagen II-induced DBA/1j mouse arthritis model. Cumulative disease score **(A)** and paw thickness **(B)** were assessed in a mouse arthritis model upon the treatment with C0115 (20 mg/ml), high dose dexamethasone (0.3 mg/kg) as a standard-of-care corticosteroid drug, or vehicle. Mean ± SEM is shown. Ordinary One-way ANOVA followed by Fisher's LSD test was used for statistical analysis in GraphPad Prism V 9.4 (*p<0.05; **p< 0.01; ***p< 0.001).

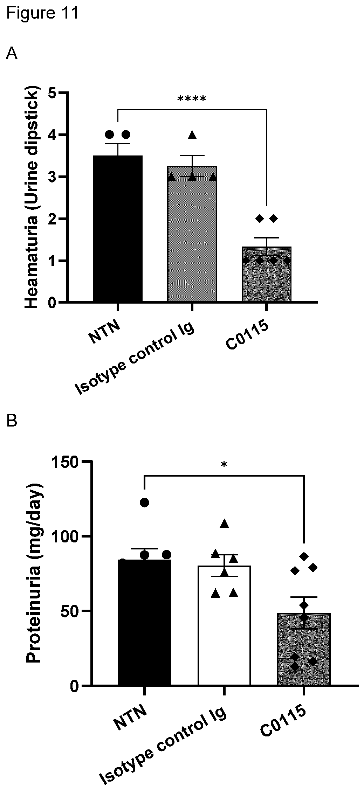

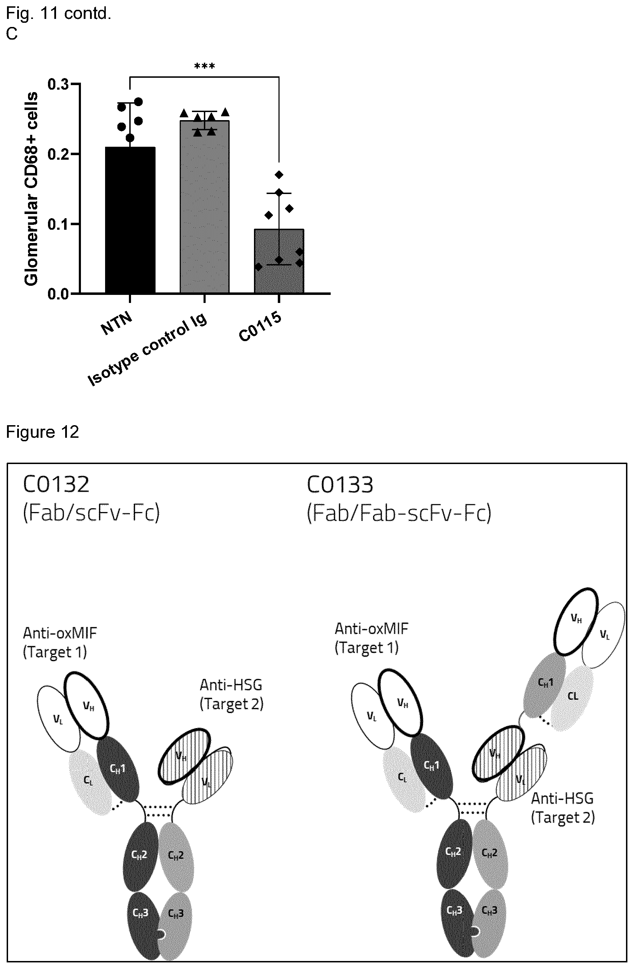

**Figure 11****:** The newly designed Fc-silenced anti-oxMIF antibody C0115 ameliorates disease severity in a nephrotoxic serum (NTS)-induced rat glomerulonephritis model. Haematuria (dipstick test) (A), proteinuria (B) and glomerular macrophage infiltration were assessed a in a rat glomerulonephritis model upon the treatment of diseased rats with the anti-oxMIF antibody C0115, Isotype control IgG1 or vehicle. Mean ± SEM is shown, Ordinary One-way ANOVA followed by Dunnett's correction for multiple testing was used for statistical analysis in GraphPad Prism V 9.4 (*p<0.05, ***p< 0.001, ****p< 0.0001).
**Figure 12****:** Schematic drawing of the newly designed Fc silenced anti-oxMIF x anti-HSG bispecific antibodies (bsMabs) C0132 (Fab/scFv-Fc) and C0133 (Fab/Fab-scFv-Fc).

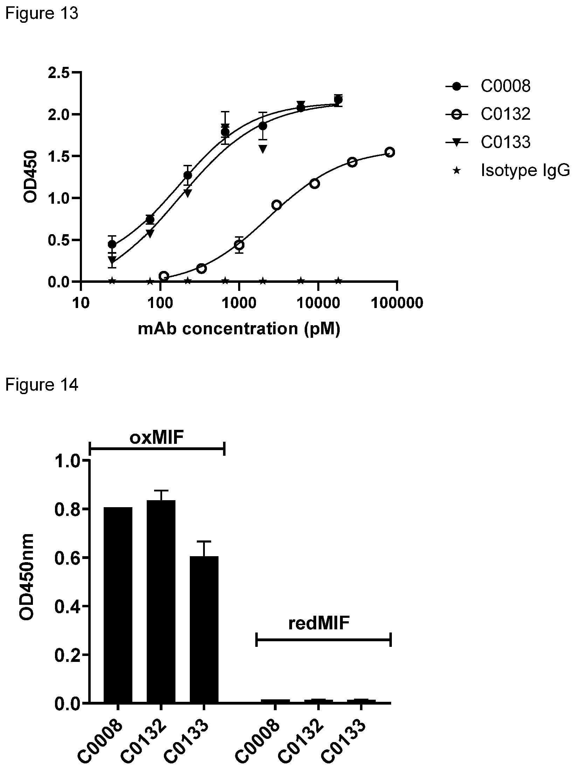

   **Figure 13****:** Binding curves of the newly designed Fc silenced anti-oxMIF x anti-HSG bsMabs C0132 and C0133 to immobilized oxMIF. Bound antibodies were detected by an anti-human-lgG (Fc)-HRP conjugate, C0008 was used as reference antibody for bivalent binding to oxMIF. Data were fitted to a sigmoidal, 4-parameter equation using GraphPad Prism (mean +/- SEM of two replicates is shown).
**Figure 14****:** Differential binding of the newly designed Fc silenced anti-oxMIF x anti-HSG bsMabs C0132 and C0133 to oxMIF vs redMIF. C0008 was used as reference anti-oxMIF antibody. Mean and SEM of three replicates is shown.

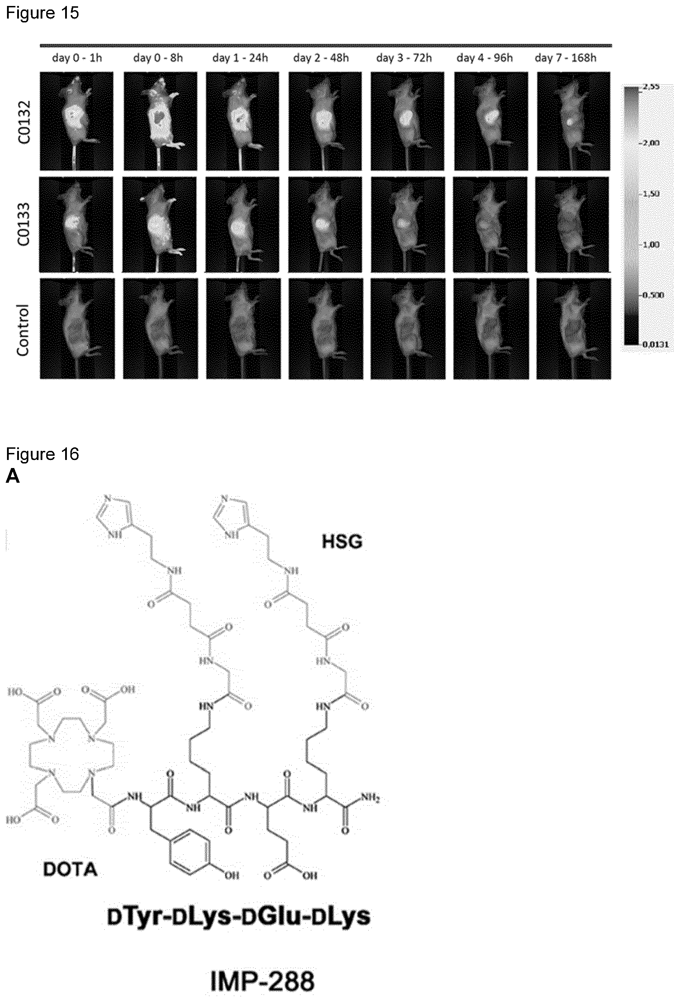

**Figure 15****:** Tumor penetration and retention of newly designed Fc silenced anti-oxMIF x anti-HSG bsMabs C0132 and C0133 assessed by infra-red *in vivo* imaging of mice carrying subcutaneous syngrafted CT26 tumors. Infra-red images of mice were taken 1h, 8h, 24h, 48h, 72h, 96h and 168h post injection of the IRDye 800CW labeled antibodies dosed at 5 mg/kg.

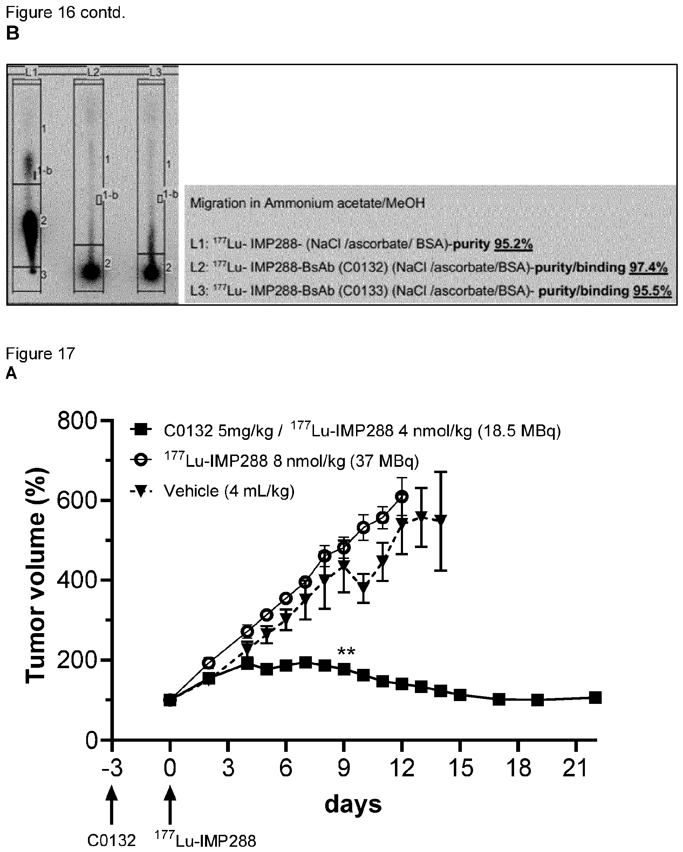

**Figure 16****:** (A) Structure of HSG hapten IMP288. (B) Binding of bsMabs C0132 and C0133 to ¹⁷⁷Lu-IMP288 peptide assessed by iTLC based on change in migration profile of ¹⁷⁷Lu-IMP288 upon the incubation with the bsMabs when compared to the migration profile of ¹⁷⁷Lu-IMP288 peptide alone.

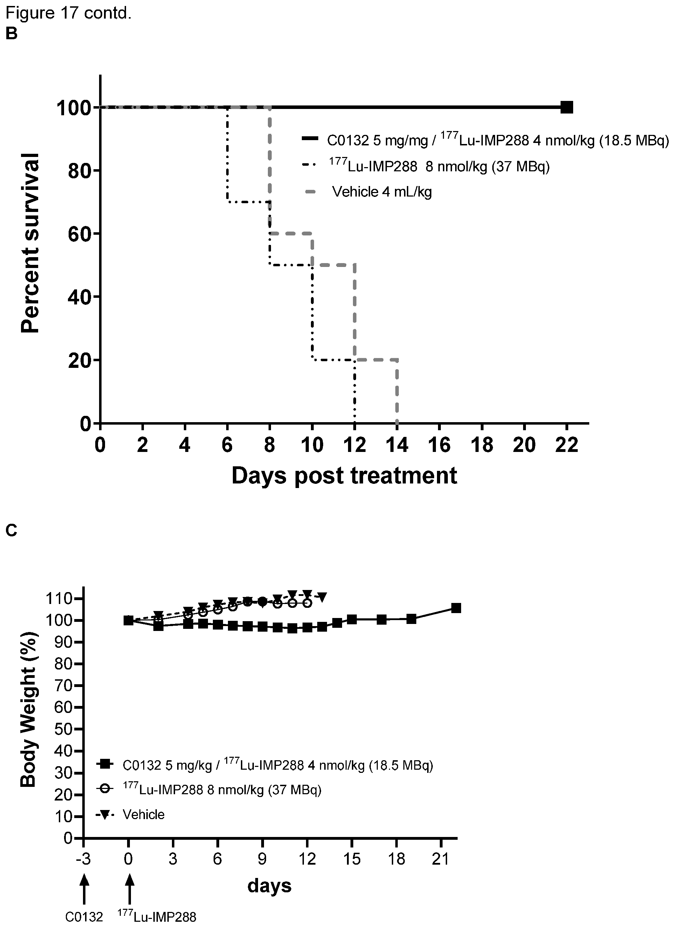

**Figure 17****:** shows PRAIT of syngrafted CT26 murine colorectal carcinomas in Balb/c mice with Fc silenced anti-oxMIF x anti-HSG bsMab C0132. C0132 was administered on day -3, followed by administration of ¹⁷⁷Lu-IMP288 3 days later (day 0). **(A)** tumour volumes in % of tumour volumes measured on day 0, Mean ± SEM is shown (n= up to 10). Statistical analysis was performed in GraphPad Prism V9.4 using Ordinary one-way ANOVA with Dunnett's correction for multiple testing; ** p<0.01 vs. ¹⁷⁷Lu-IMP288; **(B)** Kaplan-Meier survival curves (in % survival), **(C)** body weights in % to body weights measured on day 0.

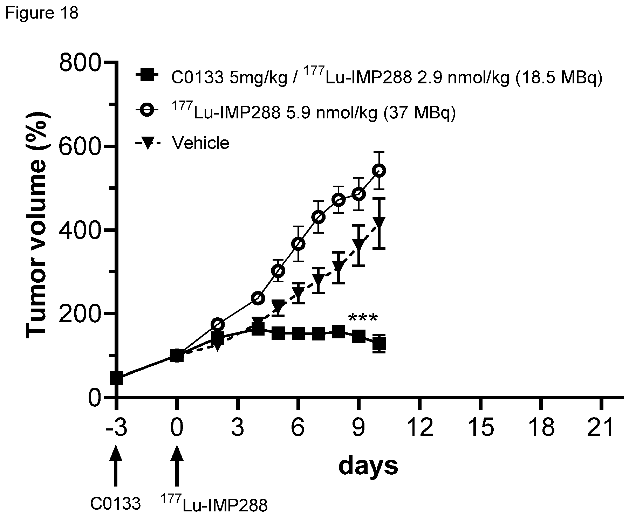

**Figure 18****:** shows PRAIT of syngrafted CT26 murine colorectal carcinomas in Balb/c mice with Fc silenced anti-oxMIFx anti-HSG bsMab C0133. C0133 was administered on day -3, followed by administration of ¹⁷⁷Lu-IMP288 3 days later (day 0). The figure shows tumour volumes in % of tumour volumes measured on day 0. Mean ± SEM is shown (n= up to 10). Statistical analysis was performed in GraphPad Prism V9.4 using Ordinary one-way ANOVA with Dunnett's correction for multiple testing; *** p<0.001 vs. ¹⁷⁷Lu-IMP288

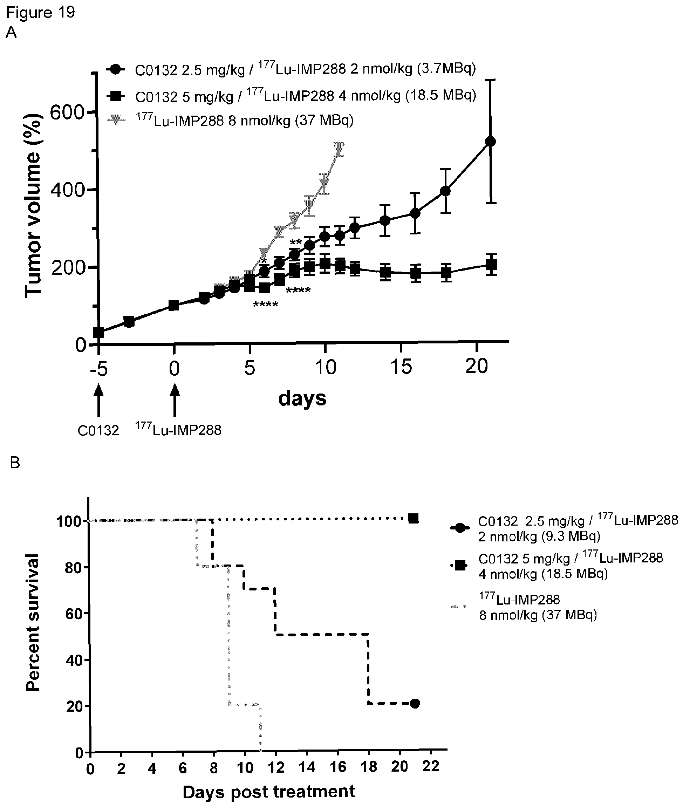

**Figure 19** Efficacy of anti-oxMIF x anti-HSG bsMab C0132 using a PRAIT approach in Balb/c mice syngrafted with CT26 murine colorectal carcinoma cells. C0132 was administered at 2.5 mg/ml, and 5 mg/ml on day -5, followed by administration of ¹⁷⁷Lu-IMP288 5 days later (day 0); Mean ± SEM is shown (n= up to 10). Statistical analysis was performed in GraphPad Prism V9.4 using Ordinary one-way ANOVA with Dunnett's correction for multiple testing; *** p<0.001 vs. ¹⁷⁷Lu-IMP288. **(A)** Tumour volumes in % of tumour volumes measured on day 0, **(B)** Kaplan-Meier survival curves (in % survival).

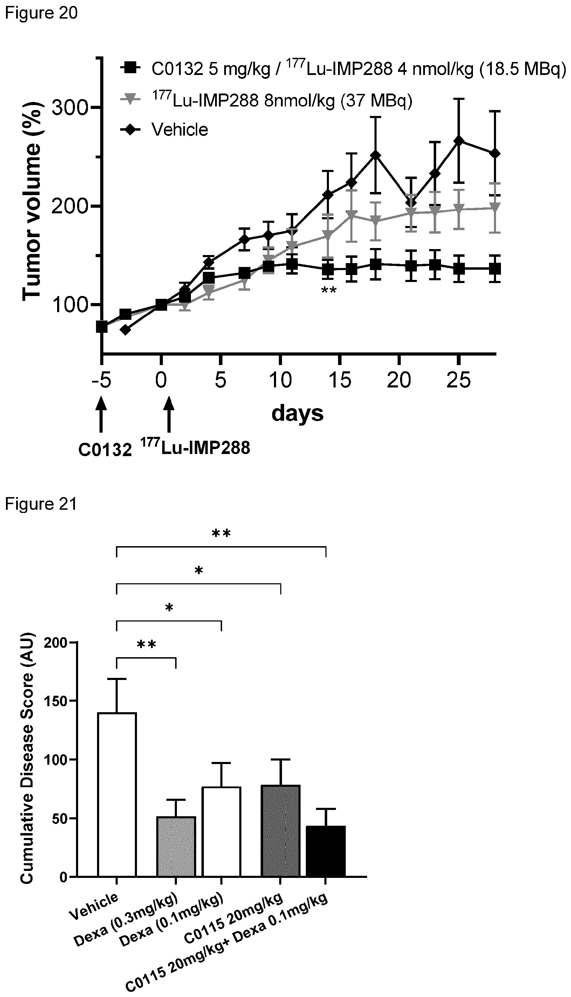

**Figure 20** Efficacy of anti-oxMIF x anti-HSG bsMab C0132 using a PRAIT approach in Balb/c nude mice xenografted with CFPAC-1 pancreatic adenocarcinoma cells. C0132 was administered at 5 mg/ml on day -5, followed by administration of ¹⁷⁷Lu-IMP288 5 days later (day 0). Tumour volumes in % of tumour volumes measured on day 0; Mean ± SEM is shown (n= up to 10). Statistical analysis was performed in GraphPad Prism V9.4 using Ordinary one-way ANOVA with Dunnett's correction for multiple testing; ** p<0.01 vs. vehicle.
**Figure 21** The newly designed Fc-silenced anti-oxMIF antibody C0115, as single agent or in combination with GCs ameliorates disease severity of collagen II-induced arthritis in DBA/1j mice. Cumulative disease score was assessed in a mouse arthritis model upon the treatment with C0115 (20 mg/kg) alone or combination with a low dose of 0.1 mg/kg of dexamethasone ("Dexa"), dexamethasone ("Dexa") at a low dose (0.1 mg/kg) and at a high dose (0.3 mg/kg) as a standard-of-care corticosteroid drug, or vehicle control. Ordinary One-way ANOVA followed by Fisher's LSD test was used for statistical analysis in GraphPad Prism V 9.4 (*p<0.05; **p< 0.01).

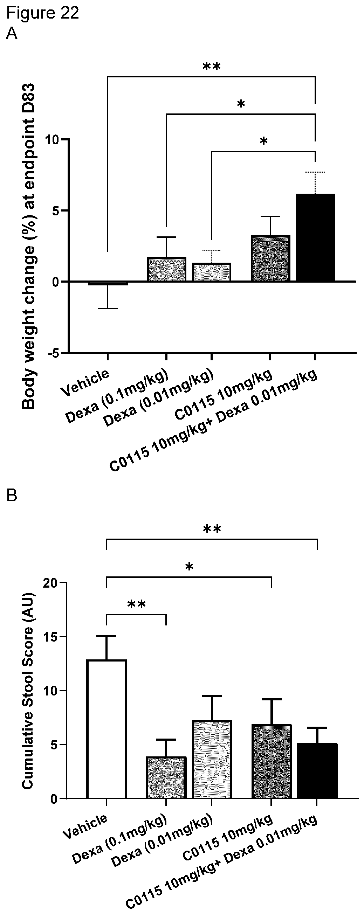

**Figure 22** The newly designed Fc-silenced anti-oxMIF antibody C0115, as single agent and in combination with GCs ameliorates disease severity in a T cell transfer mouse model of colitis. Body weight change at the end of experiment, day 83, (A) as well as cumulative stool score **(B)** were assessed upon the treatment with C0115 (10 mg/kg; alone or in combination with a low dose of 0.01 mg/kg of dexamethasone ("Dexa")), dexamethasone ("Dexa") at a low dose (0.01mg/kg) and at a high dose (0.1 mg/kg) as a standard-of-care corticosteroid drug, or vehicle control treatments. Ordinary One-way ANOVA followed by Fisher's LSD test was used for statistical analysis in GraphPad Prism V 9.4 (*p<0.05; **p< 0.01).

### DETAILED DESCRIPTION

The technical information set out below may in some respects go beyond the scope of the invention, which is defined exclusively by the appended claims. Such additional technical information which does not fall within the scope of the appended claims, is not part of the invention.

Unless indicated or defined otherwise, all terms used herein have their usual meaning in the art, which will be clear to the skilled person. Reference is for example made to the standard handbooks, such as Sambrook et al, "Molecular Cloning: A Laboratory Manual" (4th Ed.), Vols. 1 -3, Cold Spring Harbor Laboratory Press (2012); Krebs et al., "Lewin's Genes XI", Jones & Bartlett Learning, (2017), and Murphy & Weaver, "Janeway's Immunobiology" (9th Ed., or more recent editions), Taylor & Francis Inc, 2017.

The subject matter of the claims specifically refers to artificial products or methods employing or producing such artificial products, which may be variants of native (wild-type) products. Though there can be a certain degree of sequence identity to the native structure, it is well understood that the materials, methods and uses of the invention, e.g., specifically referring to isolated nucleic acid sequences, amino acid sequences, fusion constructs, expression constructs, transformed host cells and modified proteins, are "man-made" or synthetic, and are therefore not considered as a result of "laws of nature".

The terms "comprise", "contain", "have" and "include" as used herein can be used synonymously and shall be understood as an open definition, allowing further members or parts or elements. "Consisting" is considered as a closest definition without further elements of the consisting definition feature. Thus "comprising" is broader and contains the "consisting" definition.

The term "about" as used herein refers to the same value or a value differing by +/-5 % of the given value.

As used herein and in the claims, the singular form, for example "a", "an" and "the" includes the plural, unless the context clearly dictates otherwise.

As used herein, amino acids refer to twenty naturally occurring amino acids encoded by sixty-one triplet codons. These 20 amino acids can be split into those that have neutral charges, positive charges, and negative charges:
The "neutral" amino acids are shown below along with their respective three-letter and single-letter code and polarity: Alanine(Ala, A; nonpolar, neutral), Asparagine (Asn, N; polar, neutral), Cysteine (Cys, C; nonpolar, neutral), Glutamine (Gln, Q; polar, neutral), Glycine (Gly, G; nonpolar, neutral), Isoleucine (Ile, I; nonpolar, neutral), Leucine (Leu, L; nonpolar, neutral), Methionine (Met, M; nonpolar, neutral), Phenylalanine (Phe, F; nonpolar, neutral), Proline (Pro, P; nonpolar, neutral), Serine (Ser, S; polar, neutral), Threonine (Thr, T; polar, neutral), Tryptophan (Trp, W; nonpolar, neutral), Tyrosine (Tyr, Y; polar, neutral), Valine (Val, V; nonpolar, neutral), and Histidine (His, H; polar, positive (10%) neutral (90%)).
The "positively" charged amino acids are: Arginine (Arg, R; polar, positive), and Lysine (Lys, K; polar, positive).
The "negatively" charged amino acids are: Aspartic acid (Asp, D; polar, negative), and Glutamic acid (Glu, E; polar, negative).

The antibody of the invention comprises at least one binding site specifically recognizing oxMIF and exhibits reduced aggregation propensity and reduced hydrophobicity due to targeted amino acid substitutions in the variable heavy and light domains in comparison to the unmodified antibody lacking said amino acid substitutions as defined in the claims.

Reduction of aggregation potential is due to amino acid substitutions at selected positions within the variable domains of the antibody described herein.

The level of antibody aggregation can be measured using a variety of known techniques including mass spectrometry, size exclusion chromatography (SEC), hydrophobic interaction chromatography (HIC), dynamic light scattering (DLS), light obscuration (LO), dynamic imaging particle analysis (DIP A) techniques such as microflow imaging (MFI), and Coulter counter (CC), differential scanning fluorometry (DSF).

Reduced hydrophobicity and reduced aggregation potential as used herein refers to a reduction of the surface hydrophobicity and a reduced aggregation potential of the newly designed antibody compared to a reference antibody such as antibody C0008, which comprises SEQ ID NOs:44 and 2, 3, as disclosed herein. The sequence of C0008 contains the sequence of Imalumab, published in the Proposed INN List 111 (WHO Drug Information, Vol. 28, No. 2, 2014), but lacking the C-terminal lysine. Measurement can be performed using a variety of known techniques including but not limited to hydrophobic interaction chromatography (HIC) or affinity-capture self-interaction nanoparticle spectroscopy (AC-SINS, Estep P. et al., 2015).

In an embodiment, the oxMIF antibody of the invention having reduced aggregation potential and reduced hydrophobicity specifically comprises a light chain variable domain having one or more amino acid substitutions at positions M30, F49, A51, P80, W93, specifically M30L, F49Y, A51G, P80S, W93F according to the Kabat numbering, or a light chain variable domain with at least 95, specifically at least 96, 97, 98 or 99% sequence identity to SEQ ID NO:2 comprising the tyrosine at position 36, and furthermore at least one of the amino acid substitutions at positions M30, F49, A51, P80, W93, specifically M30L, F49Y, A51G, P80S, W93F, either in combination with a heavy chain variable domain comprising SEQ ID NO:3 or in combination with a heavy chain variable domain comprising the amino acid substitutions at positions L5, or W97, specifically L5Q or W97Y, according to the numbering of Kabat, or a heavy chain variable domain comprising SEQ ID NO:3 with 1, 2, 3, 4 or 5 amino acid substitutions and further comprising one or both the amino acid substitutions at positions L5 or W97, specifically L5Q or W97Y.

As described herein, the light chain variable domain comprising SEQ ID NO:2 has 1, 2, 3, 4, 5 amino acid substitutions with the proviso that the tyrosine at position 36 is preserved, and that furthermore at least one of the amino acids are substituted at positions M30, F49, A51, P80, W93.

According to a specific embodiment, amino acid W93 is replaced by F, Y, or H.

As described herein, the anti-oxMIF antibody of the invention having reduced aggregation potential and reduced hydrophobicity specifically comprises a heavy chain variable domain comprising SEQ ID NO:3 and an amino acid substitution at position W97, specifically W97Y, or an amino acid substitution at L5, specifically L5Q, or amino acid substitutions L5Q and W97Y, or a heavy chain variable domain comprising SEQ ID NO:3 with 1, 2, 3, 4 or 5 amino acid substitutions and further comprising amino acid substitution W97Y, optionally in combination with L5Q.

According to a specific embodiment, amino acid W97 is replaced by F, Y, or H.

As described herein, the heavy chain variable domain comprising SEQ ID NO:3 has 1, 2, 3, 4, 5 amino acid substitutions, and furthermore at least one of the amino acids are substituted at positions L5 and W93.

In a preferred embodiment, the anti-oxMIF antibody of the invention having reduced aggregation potential and reduced hydrophobicity specifically comprises amino acid substitutions at positions W93 and W97.

The tyrosine at position 36 of the light chain is specifically kept unmodified to preserve binding properties of the antibody described herein. Any modifications at said amino acid position may result in unwanted impaired binding properties.

Specifically, the variable light chain domain contains SEQ ID NO:5, and the variable heavy chain domain contains any one or more of SEQ ID NOs:3, 4, 9, or 43.

Specifically, the variable light chain domain contains SEQ ID NO:6, and the variable heavy chain domain is any one or more of SEQ ID NO:3, 4, 9, or 43

Specifically, the variable light chain domain contains SEQ ID NO:7, and the variable heavy chain domain is any one or more of SEQ ID NO:3, 4, 9, or 43.

Specifically, the variable light chain domain contains SEQ ID NO:8, and the variable heavy chain domain is any one or more of SEQ ID NO:3, 4, 9, or 43.

In a further embodiment, the anti-oxMIF antibody comprises SEQ ID NOs:14 and 18, in combination with any one of the variable light and heavy chain domains listed above.

Fc gamma receptors (FcγRs) are a well described family of proteins including membrane-bound surface receptors, atypical intracellular receptors and cytoplasmic glycoproteins. FcγRs control the humoral and innate immunity, which are essential for appropriate responses to infections and prevention of chronic inflammation or autoimmune diseases. Membrane-bound receptors are for example, FcγRIIa, FcyRIIb-, FcyRllla, and FcyRla receptors. Antibodies can regulate immune responses through interacting with the FcγRs.

On innate immune effector cells, activating and inhibitory FcγRs set a threshold for cell activation by immune complexes. Important examples for effector responses that are regulated by FcγRs are phagocytosis, ADCC and the release of inflammatory mediators. On dendritic cells (DCs), paired FcγR expression regulates cell maturation and antigen presentation, thereby indirectly controlling the cellular immune response. On B cells, the inhibitory FcγRIIB is essential for the maintenance of humoral tolerance. It acts as a late checkpoint at the level of class-switched memory B cells, plasmablasts or plasma cells. In addition, FcγRIIB has an important role in regulating plasma-cell homeostasis and survival. The antibody-FcγR interaction is influenced by several factors that have an impact on the expression level of activating and inhibitory FcγRs (such as cytokines) or change the affinity of the antibody-FcγR interaction (such as differential antibody glycosylation). Depending on the specific glycosylation pattern, IgG molecules can have enhanced pro- or anti-inflammatory activities. Importantly, antibody glycosylation is regulated during immune responses.

Atypical FcγRs are the neonatal Fc receptor (FcRn) and cytoplasmic glycoproteins such as complement factor C1q protein. FcRn is expressed by endothelial cells, which internalize serum components including soluble IgGs from the bloodstream by pinocytosis. IgG binding to FcRn is pH dependent; the acidic pH (pH 6.0) inside the endosomal compartment allows the IgGs to bind to FcRn. After recycling back to the cell surface, the IgG dissociates from FcRn at physiological pH (~pH 7.2), is released back into the blood circulation and thereby protected from lysosomal degradation, leading to prolonged half-life of IgGs. FcRn therefore functions as the recycling of transcytosis receptor that is responsible for maintaining IgG and albumin in the circulation. Modifications of the Fc region resulting in decreased or silenced Fc regarding FcRn binding are known in the art and are described e.g. in Kenanova V. et al., 2005 and Pyzik M. et al. 2019.

By **"effector function"** as used herein is meant a biochemical event that results from the interaction of an antibody Fc region with an Fc receptor or ligand. Effector functions include but are not limited to ADCC, ADCP, and CDC.

By **"effector cell"** as used herein is meant a cell of the immune system that expresses one or more Fc receptors and mediates one or more effector functions. Effector cells include but are not limited to monocytes, macrophages, neutrophils, dendritic cells, eosinophils, mast cells, platelets, B cells, large granular lymphocytes, Langerhans' cells, natural killer (NK) cells, and T cells, and may be from any organism including but not limited to humans, mice, rats, rabbits, and monkeys. According to the invention, the anti-oxMIF antibodies described herein have silenced effector functions due to amino acid substitutions at selected positions in the heavy chain constant region, specifically in the Fc region. Decreased or silenced effector functions of these antibodies due to reduced complement- and FcyR-mediated activities can include reduced or abolished complement dependent cytotoxicity (CDC), antibody-dependent cell-mediated cytotoxicity (ADCC) and/or antibody dependent cellular phagocytosis (ADCP).

The term **"Fc"** or **"Fc region"** (or fragment crystallizable region) as used herein refers to the polypeptide comprising the constant region of an antibody excluding the first constant region immunoglobulin domain (CH1 domain) and in some cases, part of the hinge. The Fc region refers to the C- terminal region of an antibody. The Fc region is composed of two identical protein fragments, derived from the second and third constant domains of the antibody's two heavy chains: Chain A and Chain B. The second and third constant domains are known as the CH2 domain and the CH3 domain, respectively. The CH2 domain comprises a CH2 domain sequence of Chain A and a CH2 domain sequence of Chain B. The CH3 domain comprises a CH3 domain sequence of Chain A and a CH3 domain sequence of Chain B. As used herein, the Fc region includes the hinge region or a part thereof.

The "CH2 domain" of a human IgG Fc region sequence usually extends from about amino acid 231 to about amino acid 340 according to EU numbering. The CH2 domain sequence is unique in that it is not closely paired with another domain sequence. Rather, two N-linked branched carbohydrate chains are interposed between the two CH2 domain sequences of an intact native IgG molecule.

The "CH3 domain" comprises the stretch of residues C-terminal to a CH2 domain sequence in an Fc region sequence (i.e. from about amino acid residue 341 to about amino acid residue 447 of an IgG according to EU numbering).

A **"functional Fc region"** possesses the "effector functions" and the FcRn binding of a native Fc region. Exemplary "effector functions" include C1 q binding; complement dependent cytotoxicity; Fc receptor binding; antibody-dependent cell-mediated cytotoxicity (ADCC); etc. Such effector functions generally require the Fc region to be combined with a binding domain (e.g. an antibody variable domain) and can be assessed using various assays known in the art and as herein disclosed.

A **"native Fc region"** comprises an amino acid sequence identical to the amino acid sequence of an Fc region found in nature. Native sequence human Fc regions include a native sequence human IgG1 Fc region (non-A and A allotypes); native sequence human IgG2 Fc region and native sequence human IgG3 Fc region as well as naturally occurring variants thereof.

A **"variant Fc region"** comprises an amino acid sequence which differs from that of a native Fc region sequence by virtue of "one or more amino acid substitutions". The variant Fc region sequence has at least one amino acid substitution compared to a native Fc region sequence or to the Fc region sequence of a parent polypeptide, e.g. from about one to about twenty amino acid substitutions, and preferably from about one to about seventeen amino acid substitutions in a native Fc region sequence or in the Fc region sequence of the parent polypeptide. In certain embodiments, the variant Fc region sequence herein possesses at least about 80% identity with a native Fc region sequence and/or with an Fc region sequence of a parent polypeptide, and most preferably at least about 90% identity therewith, more preferably at least about 95% identity therewith.

In a specific embodiment, the amino acid substitutions are at any one of positions E233, L234, L235, G236, I253, G237, P238, D265, S267, H268, N297, S298, T299, H310, E318, L328, P329, A330, P331 and H435 with reference to IgG1 according to EU numbering.

Modification of the Fc region resulting in decreased or silenced Fc regarding effector functions are known in the art and are described in Saunders K., 2019 and Liu R. et al., 2020.

In an alternative embodiment, the amino acid substitutions are at any one or all of positions L234F, H268Q, K274Q, Y296F, A327G, A330S, P331S in the CH2 domain and R355Q, K409R, Q419E, P445L in the CH3 domain.

Specifically, the Fc silenced anti-oxMIF antibody described herein comprises one or more of the following combinations of amino acid substitutions or deletions:
i) L235, G237 and E318, specifically L235A, G237A and E318A;
ii) L234, L235, specifically L234A, L235A;
iii) S228, L235, specifically S228P, L235E;
iv) G236, L328, specifically G236R, L328R;
v) S298, T299, specifically S298G, T299A;
vi) L234, L235, P331, specifically L234F, L235E, P331S;
vii) H268, V309, A330, P331, specifically H268Q, V309L, A330S, P331S;
viii) E233, L234, L235, G236, S267, specifically E233P, L234V, L235A, G236del, S267K;
ix) L234, L235, P329, specifically L234A, L235A, P329G;
x) V234, G237, P238, H268, A330, P331, specifically V234A, G237A, P238S, H268A, A330S, P331S;
xi) L234, L235, D265, specifically L234F, L235E, D265A;
xii) D265, specifically D265A;
xiii) G237, specifically G237A;
xiv) E318, specifically E318A;
xv) E233, specifically E233P,
xvi) G236, L328, specifically G236R, L328R;
xvii) L235, specifically L235E;
xviii) L234, L235, P331, specifically L234Q, L235F, P331S;
xix) L234, L235, G237, P238, H268, A330, P331, specifically L234A, L235A, G237A, P238S, H268A, A330S, P331S.
xx) N297, specifically N297A, N297Q or N297G, leading to an aglycosylated antibody

Glycosylation, O- and N-glycosylation, is a post-translational modification of Abs, which can be regulated by a range of B cell stimuli, including environmental factors, such as stress or disease, cytokine activity, and innate immune signaling receptors, such as Toll-like receptors. Glycosylation pattern of the parent antibody can be modified by methods well known in the art. Specifically, O-linked glycosylation sites are located in the CH2 and hinge region.

In a further embodiment, the amino acid substitutions are at any one or all of positions I253 and H310 in the CH2 domain and H435 in the CH3 domain.

Specifically, the Fc silenced anti-oxMIF antibody described herein comprises one or more of the following combinations of amino acid substitutions:
i) I253A
ii) H310A
iii) H435, specifically H435A, H345Q or H435R
iv) I253A and H310A
v) I253A and H310A and one of H435Q, H435A, andH435R
vi) H310A and one of H435Q, H435A, and H435R

The herein described silencing and further mutations in the CH domains, however, can also be introduced into the Fc of wild type IgG2, IgG3 or IgG4 at corresponding positions according to EU numbering.

The term **"aglycosylated"** indicates that the Fc region is not glycosylated. All human constant regions of the IgG isotype are known to be glycosylated at the asparagine residue at position 297, which makes up part of the N-glycosylation motif Asparagine 297-X 298-Serine 299 or Threonine 299, where X is the residue of any amino acid except proline. The glycan has a heptasaccharide core and variable extensions, such as fucose, galactose and/or sialic acid. The antibody of the invention may thus be aglycosylated by the replacement of Asparagine 297 in such a constant region with another amino acid which cannot be glycosylated or deglycosylated by enzymatic means. Any other amino acid residue can potentially be used, but alanine is the most preferred. Alternatively, glycosylation at Asparagine 297 can be prevented by altering one of the other residues of the motif, e.g. by replacing residue 298 by proline, or residue 299 by any amino acid other than serine or threonine. Techniques for performing this site directed mutagenesis are well known to those skilled in the art and may for example be performed using a commercially available site directed mutagenesis kit.

The term **"silenced Fc"** refers to an Fc region of an antibody whose effector function and/or FcRn binding is reduced or eliminated due to amino acid substitutions or modification of the glycosylation pattern resulting in modified glycan that reduce or eliminate binding of the antibody to any of FcγR, such as FcγRIIaH, FcγRIIaR, FcyRllb FcyRIllaF, FcyRIIIaV, and FcyRla and/or FcRn receptors and to complement factor C1 q protein. Such reduction or elimination of this binding results in reduction or elimination of effector functions and/or FcRn binding typically mediated by the wild-type IgG Fc region.

If FcγR binding, such as one any FcγRIIa, FcyRII FcyRllla, and FcyRla and/or FcRn receptors and to complement factor C1 q protein is completely abolished, the term "Fc null" may be used herein.

A great extent of Fc silencing can be achieved by combining mutations L234 and L235, these residues being located close to the hinge area and reducing FcyR binding when substituted by alanine. As an example, the combination of L234A and L235A with P329G can lead to a near complete inhibition of FcyR interaction for all FcyR isoforms
A Fc silenced anti-oxMIF antibody or an antigen binding fragment thereof with greatly reduced, silenced, negligible or ablated FcyR binding affinity and C1q binding affinity is one which has diminished FcyR binding activity and C1q binding activity compared to a parent polypeptide or to a polypeptide comprising a native Fc region sequence. In some embodiments, an Fc silenced anti-oxMIF antibody or an antigen binding fragment thereof with greatly reduced, silenced, negligible or ablated FcR binding affinity and C1q binding affinity has also greatly reduced, silenced, negligible or ablated ADCC, ADCP and CDC activity compared to a parent polypeptide or to a polypeptide comprising a native Fc region sequence. A Fc silenced anti-oxMIF antibody or an antigen binding fragment thereof which displays decreased or undetectable binding to FcyR may bind all FcyRs with lower affinity than the parent polypeptide. Such variants which display decreased binding to an FcyR may possess little or no appreciable binding to an FcyR. In one specific embodiment, the variant displays 0-20% binding to the FcyR compared to a native IgG Fc region, e.g. as measured by change in equilibrium constant. In one embodiment, the variant displays 0-10% binding to the FcyR compared to a native IgG Fc region. In one embodiment, the variant displays 0-5% binding to the FcyR compared to a native IgG Fc region. In one embodiment, the variant displays 0-1 % binding to the FcyR compared to a native IgG Fc region.

Antibodies described herein with silenced complement activities can be determined by cell-based CDC assays and reduced or abolished binding to C1q determined by i.e. SPR or ELISA.

Decreased or silenced CDC activity is determined to be at least 1.5-fold, specifically at least 2-fold, 5-fold, 6-fold, 7-fold, 8-fld, 9-fold, more specifically at least 10-fold downregulated compared to a reference, i.e. unmodified, wild type antibody, e.g. C0008.

Decreased ADCC or ADCP activity is determined to be at least 2-fold, 5-fold, 6-fold, 7-fold, 8-fold, 9-fold, more specifically at least 10-fold decreased potent compared to a reference antibody, i.e. unmodified, wild type antibody, e.g. C0008.

In a specific embodiment, Fc silenced anti-oxMIF antibody shows reduced binding to FcRn due to amino acid substitutions at selected positions in the heavy chain constant region. Decreased binding of the Fc silenced anti-oxMIF antibody of the present invention to the FcRn leads to reduced half-life in the circulation and faster in vivo clearance. FcRn binding and in vivo clearance/half-life determinations can also be performed using methods known in the art (see, e.g., Petkova, S.B. et al., 2006).

The Fc domain of the Fc silenced anti-oxMIF antibody, or an antigen binding fragment thereof, exhibiting decreased FcRn binding compared to an anti-oxMIF antibody or an antigen binding fragment thereof comprising the wild-type IgG Fc region may preferably comprise an Fc domain comprising one, two or three amino acid substitutions at any one of positions I253, H310, and H435.

Decreased FcRn binding (i.e. affinity) is determined to be at least 2-fold, 5-fold, 6-fold, 7-fold, 8-fold, 9-fold, more specifically at least 10-fold decreased binding (i.e. affinity) compared to a reference antibody, i.e. unmodified, wild type antibody, e.g. C0008
In a further embodiment, an even greater extent of Fc silencing can be achieved by combining mutations at the amino acid positions L234, L235, H310 and H435. These residues being are located in the Fc region and can lead to a near complete inhibition of FcyR interaction.

Specifically, the Fc silenced anti-oxMIF antibody comprises any one or more of the CDRs listed in Table 1A as defined according to Kabat, IMGT and MacCallum RM et al., 1996 (CONTACT):

| **SEQ ID NO** | **Amino acid sequence** | **L (light chain), H (heavy chain) CDR** |
|---|---|---|
| 72 | RSSQRIMTYLNWY | L-CDR1 |
| 73 | LLIFVASHSQS | L-CDR2 |
| 74 | QQSFWTPLT | L-CDR3 |
| 75 | SIYSMN | H-CDR1 |
| 76 | WVSSIGSSGGTTYYADSVKG | H-CDR2 |
| 77 | AGSQWLYGMDV | H-CDR3 |
| 78 | RSSQRI**L**TYLNWY | L-CDR1 |
| 79 | LLI**Y**V**G**SHSQS | L-CDR2 |
| 80 | LLIFV**G**SHSQS | L-CDR2 |
| 81 | LLI**Y**VASHSQS | L-CDR2 |
| 82 | QQSF**F**TPLT | L-CDR3 |
| 83 | WV**G**S**I**GSSGGTTYYADSVKG | H-CDR2 |
| 84 | AGSQ**Y**LYGMDV | H-CDR3 |

oxMIF binding specificity can be determined by any assay appropriate for determining selective binding to oxMIF, such as any competition assay against a control antibody, such as Imalumab, with respect to binding to oxMIF or various assays known in the art and as herein disclosed.

The term **"antibody"** herein is used in the broadest sense and encompasses polypeptides or proteins that consist of or comprise antibody domains, which are understood as constant and/or variable domains of the heavy and/or light chains of immunoglobulins, with or without a linker sequence. The term encompasses various antibody structures, including but not limited to monoclonal antibodies, polyclonal antibodies, multi-specific antibodies such as bispecific antibodies, trispecific antibodies, and antibody fragments as long as they exhibit the desired antigen-binding activity, i.e. binding to oxMIF. The term also encompasses fusion proteins, such as fusions with immunotoxins or antibody conjugates, such as antibody drug conjugates binding to oxMIF.

Antibody domains may be of native structure or modified by mutagenesis or derivatization, e.g. to modify the antigen binding properties or any other property, such as stability or functional properties, such as binding to the Fc receptors, such as FcRn and/or Fc-gamma receptor. Polypeptide sequences are considered to be antibody domains, if comprising a beta-barrel structure consisting of at least two beta-strands of an antibody domain structure connected by a loop sequence.

It is understood that the term "antibody" includes antigen binding derivatives, variants and fragments thereof. A derivative or variant is any combination of one or more antibody domains or antibodies of the invention and/ or a fusion protein in which any domain of the antibody of the invention may be fused at any position of one or more other proteins, such as other antibodies or antibody formats, e.g. a binding structure comprising CDR loops, a receptor polypeptide, but also ligands, scaffold proteins, enzymes, labels, toxins and the like.

The term "antibody" shall particularly refer to polypeptides or proteins that exhibit binding properties to the target antigen oxMIF.

The term **"antibody fragment, antigen-binding fragment, antigen binding variant or antibody variant"** can be used interchangeably and refers to a molecule other than an intact antibody that comprises an antigen binding portion of an intact antibody that binds the antigen to which the intact antibody binds and multispecific antibodies formed from antibody fragments or variants and further comprises a variant Fc region as described herein. Examples of antigen portions include but are not limited to Fv, Fab, Fab', Fab'-SH, single chain antibody molecules (e.g. scFvs) diabodies, cross-Fab fragments; linear antibodies. According to the invention, the antibody fragment or variant is fused to a silenced Fc-portion or silenced Fc-domains by a hinge region and/or a linker (e.g. (scFv) -Fc, (scFv)₂-Fc, scFv/scFv-Fc, Fab/scFv-Fc, Fab/(scFv)₂-Fc, Fab/Fab-scFv-Fc, IgG-scFv and IgG-(scFv)₂).

In addition, antibody fragments comprise single chain polypeptides having the characteristics of a VH domain, namely being able to assemble together with a VL domain, or of a VL domain, namely being able to assemble together with a VH domain to a functional antigen binding site and thereby providing the antigen binding property of full-length antibodies. Antibody fragments as referred herein also encompass silenced Fc domains comprising one or more structural loop regions containing antigen binding regions such as Fcab^{™} or full-length antibody formats with IgG structures in which the silenced Fc region has been replaced by an Fcab^{™} containing second distinct antigen binding site.

As used herein, **"Fab fragment or Fab"** refers to an antibody fragment comprising a light chain fragment comprising a VL domain and a constant domain of a light chain (CL), and a VH domain and a first constant domain (CH1) of a heavy chain. The antibodies of the invention can comprise at least one Fab fragment, wherein either the variable regions or the constant regions of the heavy and light chain are exchanged. Due to the exchange of either the variable regions or the constant regions, said Fab fragment is also referred to as "cross-Fab fragment" or "crossover Fab fragment". Two different chain compositions of a crossover Fab molecule are possible and comprised in the antibodies of the invention: The variable regions of the Fab heavy and light chain can be exchanged, i.e. the crossover Fab molecule comprises a peptide chain. According to the invention, the Fab is fused to a silenced Fc-portion or silenced Fc-domains by a hinge region.

As used herein, **"Fab arm"** refers to a Fab fragment fused to a silenced Fc-portion or silenced Fc-domains by a hinge region.

A **"(scFv)2"** refers to an artificial monoclonal antibody which is a fusion protein consisting of two single-chain variable fragments (scFvs) of either different antibodies or the same antibody, or amino acid sequences from four or two different genes, on a single peptide chain of about 50 kilodaltons.

A **"bs(scFv)2"** refers to an artificial monoclonal antibody which is a fusion protein consisting of two single-chain variable fragments (scFvs) of antibodies with different target antigens, or amino acid sequences from four different genes, on a single peptide chain of about 50 kilodaltons.

The term **"functional variant"** or "functionally active variant" also includes naturally occurring allelic variants, as well as mutants or any other non-naturally occurring variants. As is known in the art, an allelic variant, or also referred to as homologue, is an alternate form of a nucleic acid or peptide that is characterized as having a substitution, deletion, or addition of one or more nucleotides or amino acids that does essentially not alter the biological function of the nucleic acid or polypeptide. Specifically, a functional variant may comprise a substitution, deletion and/or addition of 1, 2, 3, 4, 5, 6, 7, 8, 9, 10, 11, 12, 13, 14, 15, 16, 17, 18, 19 or 20 amino acid residues, or a combination thereof, which substitutions, deletions and/or additions are conservative modifications and do not alter the antigen binding properties. Specifically, a functional variant as described herein comprises no more than or up to 1, 2, 3, 4, 5, 6, 7, 8, 9, 10, 11, 12, 13, 14, 15, 16, 17, 18, 19 or 20 amino acid substitutions, deletions and/or additions, which are conservative modifications and do not alter the antibody's function. Specifically, a functionally active variant as described herein comprises up to 15, preferably up to 10 or 5, amino acid substitutions, deletions and/or additions, which are conservative modifications and do not alter the antibody's function.

Functional variants may be obtained by sequence alterations in the polypeptide or the nucleotide sequence, e.g. by one or more point mutations, wherein the sequence alterations retain or improve a function of the unaltered polypeptide or the nucleotide sequence, when used in combination of the invention. Such sequence alterations can include, but are not limited to, (conservative) substitutions, additions, deletions, mutations and insertions. Conservative substitutions are those that take place within a family of amino acids that are related in their side chains and chemical properties. Examples of such families are amino acids with basic side chains, with acidic side chains, with non-polar aliphatic side chains, with non-polar aromatic side chains, with uncharged polar side chains, with small side chains, with large side chains etc.

A point mutation is particularly understood as the engineering of a polynucleotide that results in the expression of an amino acid sequence that differs from the non-engineered amino acid sequence by the deletion or insertion of one or more individual amino acids or by exchange of amino acids due to substitution.

According to a specific embodiment, the antibodies described herein may comprise one or more tags for purification and/or detection, such as but not limited to affinity tags, solubility enhancement tags and monitoring tags.

Specifically, the affinity tag is selected from the group consisting of poly-histidine tag, poly-arginine tag, peptide substrate for antibodies, chitin binding domain, RNAse S peptide, protein A, ß-galactosidase, FLAG tag, Strep II tag, streptavidin-binding peptide (SBP) tag, calmodulin-binding peptide (CBP), glutathione S-transferase (GST), maltose-binding protein (MBP), S-tag, HA tag, and c-Myc tag, specifically the tag is a His tag comprising one or more H, such as a hexahistidine tag.

By **"fused"** or **"connected"** is meant that the components (e.g. a Fab molecule and an Fc domain subunit) are linked by peptide bonds, either directly or via one or more peptide linkers.

The term **"linker"** as used herein refers to a peptide linker and is preferably a peptide with an amino acid sequence with a length of 2, 3, 4, 5, 6, 7 or more amino acids, preferably with a length of 2-10, more preferably of 3-5 amino acids.

The term **"immunoglobulin"** refers to a protein having the structure of a naturally occurring antibody. For example, immunoglobulins of the IgG class are heterotetrameric glycoproteins of about 150,000 daltons, composed of two light chains and two heavy chains that are disulfide-bonded. From N- to C-terminus, each heavy chain has a variable region (VH), also called a variable heavy domain or a heavy chain variable domain, followed by three constant domains (CH1, CH2, and CH3), also called a heavy chain constant region. Similarly, from N- to C-terminus, each light chain has a variable region (VL), also called a variable light domain or a light chain variable domain, followed by a constant light (CL) domain, also called a light chain constant region. An immunoglobulin of the IgG class essentially consists of two Fab molecules and an Fc domain, linked via the immunoglobulin hinge region. The heavy chain of an immunoglobulin may be assigned to one of five types, called α (IgA), δ (IgD), ε (IgE), γ (IgG), or µ (IgM), some of which may be further divided into subtypes, e.g. γ₁ (IgG₁), γ₂ (IgG₂), γ₃ (IgG₃), γ₄ (IgG₄), α₁ (IgA₁) and α₂ (IgA₂). The light chain of an immunoglobulin may be assigned to one of two types, called kappa (κ) and lambda (λ).

The term **"chimeric antibody"** refers to an antibody in which a portion of the heavy and/or light chain is derived from a particular source or species, while the remainder of the heavy and/or light chain is derived from a different source or species, usually prepared by recombinant DNA techniques. Chimeric antibodies may comprise a rabbit or murine variable region and a human constant region. Chimeric antibodies are the product of expressed immunoglobulin genes comprising DNA segments encoding immunoglobulin variable regions and DNA segments encoding immunoglobulin constant regions. Methods for producing chimeric antibodies involve conventional recombinant DNA and gene transfection techniques are well known in the art (Morrison, S.L., et al., 1984).

A **"human antibody"** possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues. As also mentioned for chimeric and humanized antibodies, the term "human antibody" as used herein also comprises such antibodies which are modified in the constant region e.g. by "class switching" i.e. change or mutation of Fc parts (e.g. from IgG1 to IgG4 and/or IgG1/IgG4 mutation.)

The term **"recombinant human antibody",** as used herein, is intended to include all human antibodies that are prepared, expressed, created or isolated by recombinant means, such as antibodies isolated from a host cell such as a HEK cell, NS0 or CHO cell or from an animal (e.g. a mouse) that is transgenic for human immunoglobulin genes or antibodies expressed using a recombinant expression vector transfected into a host cell. The amino acid sequences of the VH and VL regions of the recombinant antibodies are sequences that, while derived from and related to human germ line sequences, may not naturally exist within the human antibody repertoire in vivo.

A **"human consensus framework"** is a framework which represents the most commonly occurring amino acid residues in a selection of human immunoglobulin VL or VH framework sequences. Generally, the selection of human immunoglobulin VL or VH sequences is from a subgroup of variable domain sequences. Generally, the subgroup of sequences is a subgroup as described in Kabat et al., 1991.

A **"humanized"** antibody refers to a chimeric antibody comprising amino acid residues from non-human HVRs and amino acid residues from human framework regions (FRs) which has undergone humanization. In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. Specifically, forms of humanized antibodies are encompassed by the present invention in which the constant region has been additionally modified or changed from that of the original antibody to generate the new properties, i.e. in regard to reduced or abolished C1q binding and/or Fc receptor (FcR) binding.

The term **"bispecific"** as used herein shall refer to the binding reaction at least with an oxMIF antigen and a further antigen, such as but not limited to e.g. CD3 or HSG antigen. A bispecific antibody specifically can comprise at least two sites with specific binding properties, wherein two different target antigens, oxMIF and a further antigen, are recognized by the antibody. An exemplary bispecific antibody format may comprise two binding sites, wherein each of the binding sites is capable of specifically binding a different antigen, e.g. CD3 or HSG, and oxMIF. A further exemplary bispecific format may comprise more than two binding sites, wherein one or more binding sites bind to oxMIF and one or more binding sites are capable of specifically binding one or more different antigens, e.g. CD3 or HSG.

**"Bispecific antibodies"** according to the invention are antibodies which have two different binding specificities. Bispecific antibodies can be prepared as full-length antibodies or antibody fragments as described herein, but still comprising a silenced Fc region. Immunoglobulin Fc heterodimers may be engineered through modifications to the CH3 domain interface, with different mutations on each domain such that the engineered Fc fragments, carrying the CH3 variant pair, preferentially form heterodimers rather than homodimers (Ha J-H. et al., 2016). Examples of bispecific antibody formats can be, but are not limited to bispecific IgGs (BsIgG), IgGs appended with an additional antigen-binding moiety, BsAb fragments, bispecific fusion proteins, BsAb conjugates, hybrid bslgGs, modified Fc fusion proteins, appended IgGs-HC fusions, appended IgGs-LC fusions, appended IgGs-HC& LC fusions, Fc fusions, CH3 fusions, F(ab')₂ fusions, CH1/CL, modified IgGs, Fc-modified IgGs, diabodies, etc. as described in Spiess C. et al., 2015, and Brinkmann U. and Kontermann R.E., 2017.

In an alternative embodiment encompassing bispecific antibodies or antibody fragments described herein, the term **"IgG-scFv"** refers to a kind of bispecific antibodies which is engineered for bispecificity by fusing one scFv to a monospecific Immunoglobulin G (IgG). According to the invention, the bispecific antibodies are Fc silenced, i.e. they comprise a variant Fc region of a wild-type human IgG with one or more amino acid substitutions or a glycosylation modification described herein. The specificity of the IgG can be for oxMIF and the specificity of the scFv can be for CD3 or HSG or vice versa. Furthermore, either the amino terminus or the C terminus of one of the light or heavy chains can be appended with an scFv, which leads to the production of diverse types of IgG-scFv bispecific antibodies (BsAbs): (i) IgG(H)-scFv, an scFv linked to the C terminus of one of the full-length IgG HC; (ii) scFv-(H)IgG, which is same like IgG(H)-scFv, except that the scFv is linked to the HC N-terminus; (iii) IgG(L)-scFv or (iv) scFv-(L)IgG, the scFv connected to the C or N-terminus of the IgG light chain, which forms the IgG(L)-scFv or scFv-(L)IgG, respectively. Specifically, the IgG-scFv is in the range of 165 kDa to 185 kDa, specifically it is about 175 kDa.

According to an alternative embodiment, fusing recombinant variable domains such as diabodies to a silenced Fc region (e.g. scDb-Fc) can significantly increase the valency. The increased size can also prolong the half-life of diabody in serum.

The term **"diabody"** refers to a noncovalent dimer of single-chain Fv (scFv) fragment that consists of the heavy chain variable (V_{H}) and light chain variable (V_{L}) regions connected by a small peptide linker. Another form of diabody is single-chain (Fv)₂ in which two scFv fragments are covalently linked to each other. In addition, by tandem linking genes in each chain with internal linker, four VH and VL domains can be expressed in tandem and folded as single chain diabody (scDb), which is also an effective strategy for bispecific antibody production. Specifically, the diabody-CH3 is of about 125kDa.

In a specific embodiment, the term Fab/bs(scFv)2-Fc refers to bispecific antibody which is an IgG having one Fab arm replaced by a bs(scFv)2, while the second IgG arm is preserved.

In a specific embodiment, the term Fab/scFv-Fc refers to a bispecific antibody which is an IgG having one Fab arm replaced by a scFv, while the second IgG arm is preserved. Antibody C0132, as described herein and as schematically shown in Figure 12 serves as a non-limiting example of a Fab/scFv-Fc.

In a specific embodiment, the term Fab/Fab-scFv-Fc refers to bispecific antibody which is an IgG having one Fab arm replaced by a Fab-scFv, while the second IgG arm is preserved. Antibody C0133, as described herein and as schematically shown in Figure 12 serves as a non-limiting example of a Fab/Fab-scFv-Fc.

The term **"CrossMab"** (where Mab refers to monoclonal antibody) is a format of bispecific Abs derived from independent parental antibodies. Heavy chain mispairing is avoided by applying the knobs-into-holes (KIH) method. Light chain mispairing is avoided as the bispecific antibody is produced with antibody domain exchange whereas either the variable domains or the constant domains (CL and CH1) of one Fab arm are swapped between the light and heavy chains. This "crossover" keeps the antigen-binding affinity and also preserves the two different arms in order to avoid light-chain mispairing. Examples of CrossMabs can be, but are not limited to Fab, VH-VL and CH1-CL exchanged in different regions. In CrossMAbs Fabs the full VH-CH1 and VL-CL regions are exchanged; in CrossMAb VH-VL format only the VH and VL regions are exchanged; in CrossMAb CH1-CL1 format the CH1 and CL regions of bispecific antibody are exchanged. Specifically, the CrossMab is of about 150kDa.

The oxMIF antibody of the invention may further comprise at least one binding site specifically recognizing a further epitope, e.g. of CD3, specifically an epitope of human CD3, including the CD3γ (gamma) chain, CD3δ (delta) chain, and CD3ε (epsilon) chains which are present on the cell surface. Clustering of CD3 on T cells, such as by immobilized anti-CD3 antibodies leads to T cell activation similar to the engagement of the T cell receptor but independent of its clone-typical specificity. In certain embodiments, the CD3 binding domain of the antibody described herein exhibits not only potent CD3 binding affinities with human CD3, but shows also excellent cross-reactivity with the respective cynomolgus monkey CD3 proteins. In some instances, the CD3 binding domain of the antibody is cross-reactive with CD3 from cynomolgus monkey. In one embodiment, the anti-CD3 binding site comprises one or more (e.g., all three) light chain complementary determining regions of an anti-CD3 binding domain described herein, and/or one or more (e.g., all three) heavy chain complementary determining regions of an anti-CD3 binding domain described herein, e.g., an anti-CD3 binding domain comprising one or more, e.g., all three, LC CDRs and one or more, e.g., all three, HC CDRs.

According to a further embodiment, the antibody can comprise one or more additional binding sites specifically recognizing one or more antigens expressed on effector T cells, NK-cells or macrophages, specifically one or more of CD3, ADAM17, CD2, CD4, CD5, CD6, CD8, CD11a, CD11b, CD14, CD16, CD16b, CD25, CD28, CD30, CD32a, CD40, CD 40L, CD44, CD45, CD56, CD57, CD64, CD69, CD74, CD89, CD90, CD137, CD177, CEAECAM6, CEACAM8, HLA-DR alpha-chain, KIR, LSECtin or SLC44A2, or one or more hapten antigens i.e. HSG.

According to an alternative embodiment, the antibody of the invention is a bispecific antibody further comprising one or more of the CD3 variable binding domains of mosunetuzumab, pasotuxizumab, cibisatamab, otelixizumab, teplizumab, visilizumab or foralumab.

According to a specific embodiment, the anti-CD3 binding site comprises complementary determining regions (CDRs) selected from the group consisting of muromonab-CD3 (OKT3), otelixizumab (TRX4), teplizumab (MGA031), visilizumab (Nuvion), solitomab, blinatumomab, pasotuxizumab, cibisatamab, mosunetuzumab, SP34, X35, VIT3, BMA030 (BW264/56), CLB-T3/3, CRIS7, YTH12.5, F111-409, CLB-T3.4.2, TR-66, WT32, SPv-T3b, 11D8, XIII-141, XIII-46, XIII-87, 12F6, T3/RW2-8C8, T3/RW2-4B6, OKT3D, M-T301, SMC2, F101.01, UCHT-1 and WT-31 and any humanized derivatives thereof, if applicable.

Specifically, one or more CDRs of anti-CD3 variable regions or CDRs comprising or having at least 70%, specifically 80%, 90%, 95% or 99% sequence identity to any of the CDR sequences of muromonab, otelixizumab, teplizumab, visilizumab, solitomab, blinatumomab, pasotuxizumab, cibisatamab, mosunetuzumab may be used as CD3 binding domains according to the invention.

Said specific CDRs binding to CD3 are as follows:

**Table 1B: anti-CD3 heavy chain sequences**

| **Source antibody** | **VH-CDR1 (CDR1-H2)** | **VH-CDR2 (CDR2-H2)** | **VH-CDR3 (CDR3-H2)** |
|---|---|---|---|
| humanized or murine OKT3 | RYTMH SEQ ID NO:19 | YINPSRGYTNYNQKFKD SEQ ID NO:22 | YYDDHYSLDY SEQ ID NO:28 |
| Blinatumumab Muromonab Tepliuzumab | RYTMH SEQ ID NO:19 | YINPSRGYTNYNQKFKD SEQ ID NO:22 | YYDDHYCLDY SEQ ID NO:25 |
| Foralumab | GYGMH SEQ ID NO:20 | VIWYDGSKKYYVDSVKG SEQ ID NO:23 | QMGYWHFDL SEQ ID NO:26 |
| Otelixizumab | SFPMA SEQ ID NO:21 | TISTSGGRTYYRDSVKG SEQ ID NO:24 | FRQYSGGFDY SEQ ID NO:27 |
| Pasotuxizumab | GFTFNKYAMN SEQ ID NO:45 | RIRSKYNNYATYYADSVK D SEQ ID NO:46 | VRHGNFGNSYISY WAY SEQ ID NO:47 |
| Cibisatamab | GFTFSTYAMN SEQ ID NO:48 | RIRSKYNNYATYYADSVK G SEQ ID NO:49 | VRHGNFGNSYVSW FAY SEQ ID NO:50 |
| Mosunetuzumab | NYYIH SEQ ID NO:51 | WIYPGDGNTKYNEKFKG SEQ ID NO:52 | DSYSNYYFDY SEQ ID NO:53 |

**Table 2, anti-CD3 light chain sequences**

| **Source antibody** | **VL-CDR1 (CDR1-L2)** | **VL-CDR2 (CDR2-L2)** | **VL-CDR3 (CDR3-L2)** |
|---|---|---|---|
| humanized or murine OKT3 | SASSSVSYMN SEQ ID NO:30 | DTSKLAS SEQ ID NO:34 | QQWSSNP SEQ ID NO:41 |
| Blinatumumab | RASSSVSYMN SEQ ID NO 29 | DTSKVAS SEQ ID NO:33 | QQWSSNPLT SEQ ID NO:37 |
| Muromonab Tepliuzumab | SASSSVSYMN SEQ ID NO:30 | DTSKLAS SEQ ID NO:34 | QQWSSNPFT SEQ ID NO:38 |
| Foralumab | RASQSVSSYLA SEQ ID NO:31 | DASNRAT SEQ ID NO:35 | QQRSNWPPLT SEQ ID NO:39 |
| Otelixizumab | TLSSGNIENNYVH SEQ ID NO:32 | DDDKRPD SEQ ID NO:36 | HSYVSSFNV SEQ ID NO:54 |
| Pasotuxizumab | GSSTGAVTSGNYPN SEQ ID NO:55 | GTKFLAP SEQ ID NO:56 | VLWYSNRWV SEQ ID NO:57 |
| Cibisatamab | GSSTGAVTTSNYAN SEQ ID NO:58 | GTNKRAP SEQ ID NO:59 | ALWYSNLWV SEQ ID NO:60 |
| Mosunetuzumab | KSSQSLLNSRTRKNYL A SEQ ID NO:61 | WASTRES SEQ ID NO:62 | TQSFILRT SEQ ID NO:63 |

More specifically a bispecific anti-oxMIF/anti-CD3 antibody comprises 0, 1, or 2 point mutations in each of the CDR sequences listed above.

A further specific embodiment refers to an anti-oxMIF/anti-CD3 bs(scFv)2 wherein the corresponding variable heavy chain regions (VH) and the corresponding variable light chain regions (VL) regions are arranged, from N-terminus to C- terminus, in the order, VH(oxMIF)-V_{L}(oxMIF)-VH(CD3)-VL(CD3), VH(CD3)-VL(CD3)-VH(oxMIF)-VL(oxMIF) or VH(CD3)-VL(CD3)-VL(oxMIF)-VH(oxMIF). According to the invention, the bs(scFv)2 is fused to a silenced Fc-portion or silenced Fc-domains by a hinge region.

According to a further embodiment, the Fc-silenced anti-oxMIF antibody of the invention may further comprise at least one binding site specifically further recognizing the HSG (histamine-succinyl-glycine) hapten.

According to a specific embodiment, the anti-HSG binding site comprises complementary determining regions (CDRs) selected from the mouse (m) anti-HSG antibody 679 (m679) and any humanized (hz) derivatives thereof, if applicable.

Such bispecific anti-oxMIF antibody may comprise a binding site specifically recognizing HSG which comprises CDRs as listed in Tables 3 and 4 below (hz anti-HSG antibody 679 (hz679) sequences are from US 2009/0240037 A1).

**Table 3: anti-HSG heavy chain sequences**

| **Source antibody** | **VH-CDR1 (CDR1-H2)** | **VH-CDR2 (CDR2-H2)** | **VH-CDR3 (CDR3-H2)** |
|---|---|---|---|
| Hz 679 | IYTMS SEQ ID NO:64 | TLSGDGDDIYYPDSVKG SEQ ID NO:65 | VRLGDWDFDV SEQ ID NO:66 |

**Table 4, anti-HSG light chain sequences**

| **Source antibody** | **VL-CDR1 (CDR1-L2)** | **VL-CDR2 (CDR2-L2)** | **VL-CDR3 (CDR3-L2)** |
|---|---|---|---|
| Hz 679 | KSSQSLFNSRTRKNYLG SEQ ID NO:67 | WASTRES SEQ ID NO:62 | TQVYYLCT SEQ ID NO:68 |

More specifically a bispecific anti-oxMIF/anti-HSG antibody comprises 0, 1, or 2 point mutations in each of the CDR sequences listed above.

A further specific embodiment refers to an anti-oxMIF/anti-HSG bs(scFv)2 wherein the corresponding variable heavy chain regions (VH) and the corresponding variable light chain regions (VL) regions are arranged, from N-terminus to C- terminus, in the order, VH(oxMIF)-V_{L}(oxMIF)-VH(HSG)-VL(HSG), VH(HSG)-VL(HSG)-VH(oxMIF)-VL(oxMIF) or VH(HSG)-VL(HSG)-VL(oxMIF)-VH(oxMIF). According to the invention, the bs(scFv)2 is fused to a silenced Fc-portion or silenced Fc-domains by a hinge region.

In a further embodiment, the antibody is an Fc silenced bispecific antibody, specifically selected from the group consisting of bispecific IgG, IgG appended with a CD3 or HSG binding site, BsAb fragments, bispecific fusion proteins, BsAb conjugates.

Slow blood clearance and delayed tumour uptake of directly radiolabelled antibodies in solid tumours cause continuous high radiation dose exposure to healthy tissues and organs. *In vivo* pretargeted radioimmunotherapy (PRAIT) allows to overcome these limitations, although the choice and design of the bsMab format for PRAIT remains challenging. PRAIT aims at improving the therapeutic index (tumor-to-normal tissue ratios) by delivering increased absorbed doses to tumors, as compared to directly radiolabeled antibodies or antibody fragments. PRAIT involves administering a bsMab against HSG and a tumour target, followed administration of a radiolabeled bivalent HSG hapten a few days later. With this technology, a substantial amount of bsMab is accumulated in the tumour and is cleared from the circulation to a large extend and the radioactive labeled bivalent HSG hapten binds to the bsMAb accumulated in the tumour, whereas the nonbound radioactive HSG hapten clears from the circulation through the kidneys within a few hours. Thus, resulting in minimizing the exposure of normal organs to radioactivity (US2005/0025709, Sharkey RM et al., 2005, Rossi EA et al, and Karacay H. et al, 2005).

For targeted radionuclide therapy, e.g. HSG hapten IMP288 (as described in US2005/0025709) and ¹⁷⁷Lu as radionuclide may be used. IMP288 is a DOTA-conjugated D-Tyr-D-Lys-D-Glu-D-Lys-NH2 tetrapeptide in which both lysine residues are derivatized with HSG moiety via their ε-aminogroup.

The term **"antigen"** as used herein interchangeably with the term **"target"** or **"target antigen"** shall refer to a whole target molecule or a fragment of such molecule recognized by an antibody binding site. Specifically, substructures of an antigen, e.g. a polypeptide or carbohydrate structure, generally referred to as "epitopes", e.g. B-cell epitopes or T-cell epitope, which are immunologically relevant, may be recognized by such binding site.

The term **"epitope"** as used herein shall in particular refer to a molecular structure which may completely make up a specific binding partner or be part of a specific binding partner to a binding site of an antibody format of the present invention. An epitope may either be composed of a carbohydrate, a peptidic structure, a fatty acid, an organic, biochemical or inorganic substance or derivatives thereof and any combinations thereof. If an epitope is comprised in a peptidic structure, such as a peptide, a polypeptide or a protein, it will usually include at least 3 amino acids, specifically 5 to 40 amino acids, and specifically less than 10 amino acids, specifically between 4 to 10 amino acids. Epitopes can be either linear or conformational epitopes. A linear epitope is comprised of a single segment of a primary sequence of a polypeptide or carbohydrate chain. Linear epitopes can be contiguous or overlapping. Conformational epitopes are comprised of amino acids or carbohydrates brought together by folding the polypeptide to form a tertiary structure and the amino acids are not necessarily adjacent to one another in the linear sequence. Such oxMIF epitope may be sequence EPCALCS (SEQ ID NO:42) located within the central region of oxMIF.

The term **"antigen binding domain"** or "binding domain" or "binding-site" refers to the part of an antigen binding moiety that comprises the area which specifically binds to and is complementary to part or all of an antigen. Where an antigen is large, an antigen binding molecule may only bind to a particular part of the antigen, which part is termed an epitope. An antigen binding domain may be provided by, for example, one or more antibody variable domains (also called antibody variable regions). Preferably, an antigen binding domain comprises an antibody light chain variable region (VL) and an antibody heavy chain variable region (VH).

The term **"binding site"** as used herein with respect to the antibody of the present invention refers to a molecular structure capable of binding interaction with an antigen. Typically, the binding site is located within the complementary determining region (CDR) of an antibody, herein also called "a CDR binding site", which is a specific region with varying structures conferring binding function to various antigens. The varying structures can be derived from natural repertoires of antibodies, e.g. murine or human repertoires, or may be recombinantly or synthetically produced, e.g. by mutagenesis and specifically by randomization techniques. These include mutagenized CDR regions, loop regions of variable antibody domains, in particular CDR loops of antibodies, such as CDR1, CDR2 and CDR3 loops of any of VL and/or VH antibody domains. The antibody format as used according to the invention typically comprises one or more CDR binding sites, each specific to an antigen.

The oxMIF binding site of the antibody described herein is specific for the oxidized form of MIF, i.e. for animal, specifically for mammalian oxMIF, such as but not limited to mouse, rat, monkey and human, specifically for human oxMIF, but does not show substantial cross-reactivity to reduced MIF. oxMIF is the disease-related structural isoform of MIF which can be specifically and predominantly detected in the circulation of subjects with inflammatory diseases and in tumor tissue of cancer patients. In one embodiment, the humanized or human anti-oxMIF binding site comprises one or more (e.g., all three) light chain complementary determining regions of a humanized or human anti-oxMIF binding domain described herein, such as the CDRs comprised in e.g. SEQ ID NOs: 2, 5, 6, 7 or 8 and/or one or more (e.g., all three) heavy chain complementary determining regions of a humanized or human anti-oxMIF binding domain described herein, e.g. SEQ ID NOs: 3, 4, 9, or 43.

The term **"specific"** as used herein shall refer to a binding reaction which is determinative of the cognate ligand of interest in a heterogeneous population of molecules. Herein, the binding reaction is at least with an oxMIF antigen. Thus, under designated conditions, e.g. immunoassay conditions, the antibody that specifically binds to its particular target does not bind in a significant amount to other molecules present in a sample, specifically it does not show substantial cross-reactivity to reduced MIF.

A specific binding site is typically not cross-reactive with other targets. Still, the specific binding site may specifically bind to one or more epitopes, isoforms or variants of the target, or be cross-reactive to other related target antigens, e.g., homologs or analogs.

The specific binding means that binding is selective in terms of target identity, high, medium or low binding affinity or avidity, as selected. Selective binding is usually achieved if the binding constant or binding dynamics to a target antigen such as oxMIF is at least 10-fold different, preferably the difference is at least 100-fold, and more preferred a least 1000-fold compared to binding constant or binding dynamics to an antigen which is not the target antigen.

The term **"valent"** as used within the current specification denotes the presence of a specified number of binding sites in an antibody molecule. As such, the terms "bivalent", "tetravalent", and "hexavalent" denote the presence of two binding sites, four binding sites, and six binding sites, respectively, in an antibody molecule.

The term **"monovalent"** as used herein with respect to a binding site of an antibody shall refer to a molecule comprising only one binding site directed against a target antigen. The term "valency" is thus understood as the number of binding sites directed against the same target antigen, either specifically binding the same or different epitopes of an antigen.

The antibody of the present invention is understood to comprise a monovalent, bivalent, tetravalent or multivalent binding site specifically binding oxMIF

The term **"hypervariable region"** or "HVR," as used herein refers to each of the regions of an antibody variable domain which are hypervariable in sequence and/or form structurally defined loops ("hypervariable loops"). Generally, native four-chain antibodies comprise six HVRs; three in the VH (H1, H2, H3), and three in the VL (L1, L2, L3). HVRs generally comprise amino acid residues from the hypervariable loops and/or from the "complementarity determining regions" (CDRs), the latter being of highest sequence variability and/or involved in antigen recognition (Kabat et al., 1991) Hypervariable regions (HVRs) are also referred to as complementarity determining regions (CDRs), and these terms are used herein interchangeably in reference to portions of the variable region that form the antigen binding regions. The exact residue numbers which encompass a particular CDR will vary depending on the sequence and size of the CDR. Those skilled in the art can routinely determine which residues comprise a particular CDR given the variable region amino acid sequence of the antibody.

Kabat defined a numbering system for variable region sequences that is applicable to any antibody. One of ordinary skill in the art can unambiguously assign this system of "Kabat numbering" to any variable region sequence, without reliance on any experimental data beyond the sequence itself. The Kabat numbering of residues can be determined for a given antibody by alignment at regions of homology of the sequence of the antibody with a "standard" Kabat numbered sequence. As used herein, "Kabat numbering" refers to the numbering system set forth by Kabat et al., 1983, U.S. Dept. of Health and Human Services, "Sequence of Proteins of Immunological Interest". Unless otherwise specified, references to the numbering of specific amino acid residue positions in an antibody variable region are according to the Kabat numbering system. The numbering of the constant region is according to EU numbering index.

CDRs also comprise "specificity determining residues," or "SDRs," which are residues that contact antigen. SDRs are contained within regions of the CDRs called abbreviated-CDRs, or a-CDRs. Unless otherwise indicated, HVR residues and other residues in the variable domain (e.g., FR residues) are numbered herein according to Kabat et al., *supra.* CDR determination can also be performed according to IMGT (Lefranc MP. 1997). IMGT has its own definitions of the framework regions (named FR-IMGT) and CDR (named CDR-IMGT). The IMGT numbering method counts residues continuously from 1 to 128 based on the germ-line V sequence alignment.

CDRs (or SDRs) can further be determined according to MacCallum RM et al., 1996. Herein, antigen-contacting residues are analyzed and site shape in the antibody Fv and Fab crystal structures available from the Protein Data Bank are combined. Antigen-contacting propensities are presented for each antibody residue, allowing a definition for CDRs to be proposed based on observed antigen contacts. Contacts are more common at CDR residues which are located centrally within the combining site; some less central CDR residues are only contacted by large antigens. Non-contacting residues within the CDRs coincide with residues identified by Chothia and co-workers (Chothia C et al., 1987) as important in defining "canonical" conformations.

A **"point mutation"** is particularly understood as the engineering of a polynucleotide that results in the expression of an amino acid sequence that differs from the non-engineered amino acid sequence in the substitution or exchange, deletion or insertion of one or more single (non-consecutive) or doublets of amino acids for different amino acids. Preferred point mutations refer to the exchange of amino acids of the same polarity and/or charge. In this regard, amino acids refer to twenty naturally occurring amino acids encoded by sixty-one triplet codons. These 20 amino acids can be split into those that have neutral charges, positive charges, and negative charges.

**"Percent (%) sequence identity"** with respect to the polypeptide sequences identified herein is defined as the percentage of amino acid residues in a candidate sequence that are identical with the amino acid residues in the specific polypeptide sequence, after aligning the sequence and introducing gaps, if necessary, to achieve the maximum percent sequence identity, and not considering any conservative substitutions as part of the sequence identity. Those skilled in the art can determine appropriate parameters for measuring alignment, including any algorithms needed to achieve maximal alignment over the full length of the sequences being compared.

According to the present invention, sequence identity of the variable or constant region sequences is at least 95%, 96%, 97%, 98%, 99%, 99.5% or 100% with the respective sequences described herein.

A **"subject"** is a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In certain embodiments, the individual or subject is a human.

An **"isolated" nucleic acid"** refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

**"Isolated nucleic acid encoding an anti-oxMIF antibody"** refers to one or more nucleic acid molecules encoding antibody heavy and light chains (or fragments thereof), including such nucleic acid molecule(s) in a single vector or separate vectors, and such nucleic acid molecule(s) present at one or more locations in a host cell.

**"No substantial cross-reactivity"** means that a molecule (e.g., an antibody) does not recognize or specifically bind an antigen different from the actual target antigen of the molecule (e.g. an antigen closely related to the target antigen), specifically reduced MIF, particularly when compared to that target antigen. For example, an antibody may bind less than about 10% to less than about 5% to an antigen different from the actual target antigen, or may bind said antigen different from the actual target antigen at an amount consisting of less than about 10%, 9%, 8% 7%, 6%, 5%, 4%, 3%, 2%, 1%, 0.5%, 0.2%, or 0.1%, preferably less than about 2%, 1%, or 0.5%, and most preferably less than about 0.2% or 0.1% to an antigen different from the actual target antigen. Binding can be determined by any method known in the art such as, but not limited to ELISA or surface plasmon resonance.

The recombinant production of the antibody of the invention preferably employs an expression system, e.g. including expression constructs or vectors comprising a nucleotide sequence encoding the antibody format.

The term **"expression system"** refers to nucleic acid molecules containing a desired coding sequence and control sequences in operable linkage, so that hosts transformed or transfected with these sequences are capable of producing the encoded proteins. In order to effect transformation, the expression system may be included on a vector; however, the relevant DNA may then also be integrated into the host chromosome. Alternatively, an expression system can be used for *in vitro* transcription/translation.

**"Expression vectors"** used herein are defined as DNA sequences that are required for the transcription of cloned recombinant nucleotide sequences, i.e. of recombinant genes and the translation of their mRNA in a suitable host organism. Expression vectors comprise the expression cassette and additionally usually comprise an origin for autonomous replication in the host cells or a genome integration site, one or more selectable markers (e.g. an amino acid synthesis gene or a gene conferring resistance to antibiotics such as zeocin, kanamycin, G418 or hygromycin), a number of restriction enzyme cleavage sites, a suitable promoter sequence and a transcription terminator, which components are operably linked together. The terms "plasmid" and "vector" as used herein include autonomously replicating nucleotide sequences as well as genome integrating nucleotide sequences.

Specifically, the term refers to a vehicle by which a DNA or RNA sequence (e.g. a foreign gene), e.g. a nucleotide sequence encoding the antibody format of the present invention, can be introduced into a host cell, so as to transform the host and promote expression (e.g. transcription and translation) of the introduced sequence. Plasmids are preferred vectors of the invention.

Vectors typically comprise the DNA of a transmissible agent, into which foreign DNA is inserted. A common way to insert one segment of DNA into another segment of DNA involves the use of enzymes called restriction enzymes that cleave DNA at specific sites (specific groups of nucleotides) called restriction sites.

A **"cassette"** refers to a DNA coding sequence or segment of DNA that code for an expression product that can be inserted into a vector at defined restriction sites. The cassette restriction sites are designed to ensure insertion of the cassette in the proper reading frame. Generally, foreign DNA is inserted at one or more restriction sites of the vector DNA, and then is carried by the vector into a host cell along with the transmissible vector DNA. A segment or sequence of DNA having inserted or added DNA, such as an expression vector, can also be called a "DNA construct". A common type of vector is a "plasmid", which generally is a self-contained molecule of double-stranded DNA that can readily accept additional (foreign) DNA and which can readily be introduced into a suitable host cell. A vector of the invention often contains coding DNA and expression control sequences, e.g. promoter DNA, and has one or more restriction sites suitable for inserting foreign DNA. Coding DNA is a DNA sequence that encodes a particular amino acid sequence for a particular polypeptide or protein such as an antibody format of the invention. Promoter DNA is a DNA sequence which initiates, regulates, or otherwise mediates or controls the expression of the coding DNA. Promoter DNA and coding DNA may be from the same gene or from different genes, and may be from the same or different organisms. Recombinant cloning vectors of the invention will often include one or more replication systems for cloning or expression, one or more markers for selection in the host, e.g. antibiotic resistance, and one or more expression cassettes.

The procedures used to ligate DNA sequences, e.g. providing or coding for the factors of the present invention and/or the protein of interest, a promoter, a terminator and further sequences, respectively, and to insert them into suitable vectors containing the information necessary for integration or host replication, are well known to persons skilled in the art, e.g. described by Sambrook et al, 2012.

A **host cell** is specifically understood as a cell, a recombinant cell or cell line transfected with an expression construct, such as a vector according to the invention.

The term **"host cell line"** as used herein refers to an established clone of a particular cell type that has acquired the ability to proliferate over a prolonged period of time. The term host cell line refers to a cell line as used for expressing an endogenous or recombinant gene to produce polypeptides, such as the recombinant antibody format of the invention.

A **"production host cell"** or "production cell" is commonly understood to be a cell line or culture of cells ready-to-use for cultivation in a bioreactor to obtain the product of a production process, the recombinant antibody format of the invention. The host cell type according to the present invention may be any prokaryotic or eukaryotic cell.

The term **"recombinant"** as used herein shall mean "being prepared by genetic engineering" or "the result of genetic engineering", e.g. specifically employing heterologous sequences incorporated in a recombinant vector or recombinant host cell.

An antibody of the invention may be produced using any known and well-established expression system and recombinant cell culturing technology, for example, by expression in bacterial hosts (prokaryotic systems), or eukaryotic systems such as yeasts, fungi, insect cells or mammalian cells. An antibody molecule of the present invention may be produced in transgenic organisms such as a goat, a plant or a transgenic mouse, an engineered mouse strain that has large fragments of the human immunoglobulin loci and is deficient in mouse antibody production. An antibody may also be produced by chemical synthesis.

According to a specific embodiment, the host cell is a production cell line of cells selected from the group consisting of CHO, PerC6, CAP, HEK, HeLa, NS0, SP2/0, hybridoma and Jurkat. More specifically, the host cell is obtained from CHO cells.

The host cell of the invention is specifically cultivated or maintained in a serum-free culture, e.g. comprising other components, such as plasma proteins, hormones, and growth factors, as an alternative to serum.

Host cells are most preferred, when being established, adapted, and completely cultivated under serum free conditions, and optionally in media which are free of any protein/peptide of animal origin.

Anti-oxMIF antibodies of the invention can be recovered from the culture medium using standard protein purification methods.

The term **"pharmaceutical formulation"** refers to a preparation which is in such form as to permit the biological activity of an active ingredient contained therein to be effective, and which contains no additional components which are unacceptably toxic to a subject to which the formulation would be administered.

A **"pharmaceutically acceptable carrier"** refers to an ingredient in a pharmaceutical formulation, other than an active ingredient, which is nontoxic to a subject. Some examples of pharmaceutically acceptable carriers are water, saline, phosphate buffered saline, amino acids such as glycine or histidine, dextrose, glycerol, ethanol and the like, as well as combinations thereof. In many cases, it will be preferable to include isotonic agents, for example, sugars, polyalcohols such as mannitol, sorbitol, or sodium chloride in the composition. Additional examples of pharmaceutically acceptable substances are wetting agents or minor amounts of auxiliary substances such as wetting or emulsifying agents, preservatives or buffers, which enhance the shelf life or effectiveness of the antibody.

As used herein, **"treatment",** "treat" or "treating" refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, antibodies of the invention are used to delay development of a disease or to slow the progression of a disease.

The anti-oxMIF antibody of the invention and the pharmaceutical compositions comprising it, can be administered in combination with one or more other therapeutic, diagnostic or prophylactic agents. Additional therapeutic agents include other antineoplastic, anti-tumor, anti-angiogenic and chemotherapeutic agents, steroids, or checkpoint inhibitors depending on the disease to be treated.

The pharmaceutical compositions of this invention may be in a variety of forms, for example, liquid, semi-solid and solid dosage forms, such as liquid solutions (e.g., injectable and infusible solutions), dispersions or suspensions, tablets, pills, powders, liposomes and suppositories. The preferred form depends on the intended mode of administration and therapeutic application. Typical preferred compositions are in the form of injectable or infusible solutions, such as compositions similar to those used for passive immunization of humans. The preferred mode of administration is parenteral (e.g., intravenous, subcutaneous, intraperitoneal, intramuscular). In a preferred embodiment, the antibody is administered by intravenous infusion or injection. In another preferred embodiment, the antibody is administered by intramuscular or subcutaneous injection. As will be appreciated by the skilled artisan, the route and/or mode of administration will vary depending upon the desired results.

Due to the optimized and advantageous properties of the inventive antibody, high dosages of the antibody composition can be administered. Specifically, the composition comprises about 10 to 250 mg/ml of the antibody, specifically 25 to 100 mg/ml, specifically ≥ 50 mg/ml.

The anti-oxMIF antibody may be administered once, but more preferably is administered multiple times. For example, the antibody may be administered from three times daily to once every six months or longer. The administering may be on a schedule such as three times daily, twice daily, once daily, once every two days, once every three days, once weekly, once every two weeks, once every month, once every two months, once every three months and once every six months.

The invention also relates to compositions comprising an anti-oxMIF antibody or an antigen-binding portion thereof, for the treatment of a subject in need of treatment for MIF-related conditions, specifically immunological diseases such as inflammatory diseases and hyperproliferative disorders. In some embodiments, the subject in need of treatment is a human.

The term "cancer" as used herein refers to proliferative diseases, specifically to solid cancers, such as colorectal cancer, ovarian cancer, pancreas cancer, lung cancer, melanoma, squamous cell carcinoma (SCC) (e.g., head and neck, esophageal, and oral cavity), hepatocellular carcinoma, colorectal adenocarcinoma, kidney cancer, medullary thyroid cancer, papillary thyroid cancer, astrocytic tumor, neuroblastoma, Ewing's sarcoma, cervical cancer, endometrial carcinoma, breast cancer, prostate cancer, gastric cancer, and malignant seminoma, including refractory versions of any of the above cancers, or a combination of one or more of the above cancers.

Hyperproliferative disorders, such as cancerous diseases or cancer, that may be treated by anti-oxMIF antibodies of the invention can involve any tissue or organ and include but are not limited to brain, lung, squamous cell, bladder, gastric, pancreatic, breast, head, neck, liver, renal, ovarian, prostate, colorectal, esophageal, gynecological, nasopharynx, or thyroid cancers, melanomas, lymphomas, leukemias or multiple myelomas. In particular, anti-oxMIF antibodies of the invention are useful to treat carcinomas of the ovary, pancreas, colon and lung.

In a specific embodiment, the antibodies highly suitable for the treatment of cancerous diseases, specifically for the treatment of solid tumors are recombinant anti-oxMIF antibodies, bispecific anti-oxMIF/anti-CD3 antibodies, or bispecific anti-oxMIF/anti-HSG antibodies having a silenced Fc and reduced aggregation potential and reduced hydrophobicity, comprising
(a1) a light chain variable domain comprising SEQ ID NO:2 with 1, 2, 3, 4, or 5 amino acid substitutions selected from M30L, F49Y, A51G, P80S, W93F, or
(a2) a light chain variable domain comprising SEQ ID NO:2 with 1, 2, 3, 4, or 5 amino acid substitutions selected from M30L, F49Y, A51G, P80S, W93F and with 1, 2, 3, 4, or 5 further amino acid substitutions, with the proviso that the conserved tyrosine at position 36 is preserved, and
(b1) a heavy chain variable domain comprising SEQ ID NO:3; or
(b2) a heavy chain variable domain comprising SEQ ID NO:3 with 1 or 2 amino acid substitutions selected from L5Q andW97Y, or
(b3) a heavy chain variable domain comprising SEQ ID NO:3 with 1 or 2 amino acid substitutions selected from L5Q and W97Y, and 1, 2, 3, 4 or 5 further amino acid substitutions;
wherein amino acid positions are numbered according to Kabat,
wherein the variant Fc region exhibits decreased FcγR binding exhibits decreased FcγR binding compared to the wildtype IgG1 Fc region, wherein the one or more amino acid substitutions in the variant Fc region are compared to the wildtype IgG1 Fc region, wherein the one or more amino acid substitutions in the variant Fc region are at any one of positions E233, L234, L235, G236, G237, P238, D265, S267, H268, N297, S298, T299, E318, L328, P329, A330, P331 of SEQ ID NO:1, specifically the amino acid substitutions are at positions L234 and L235, according to EU numbering index, and/or wherein the Fc region is aglycosylated and wherein said antibody has reduced aggregation potential and reduced hydrophobicity compared to an antibody comprising SEQ ID NO:2 and SEQ ID NO:3 lacking the amino acid substitutions.

The invention also encompasses a pharmaceutical composition comprising the antibody of the invention for use in methods for the treatment of inflammatory diseases such as vasculitis, arthritis, sepsis, septic shock, endotoxic shock, toxic shock syndrome, acquired respiratory distress syndrome, glomerulonephritis, inflammatory bowel disease, Crohn's disease, ulcerative colitis, peritonitis, nephritis, atopic dermatitis, asthma, conjunctivitis, fever, Malaria, NASH (nonalcoholic steatosis hepatis), multiple sclerosis, acute & chronic pancreatitis, Type 1 diabetes, IgA nephropathy, interstitial cystitis, post COVID syndrome, psoriasis, glomerulonephritis, inflammatory bowel disease, nephritis and peritonitis, systemic lupus erythematosus (SLE - including lupus nephritis), asthma, rheumatoid arthritis (RA) and inflammatory bowel disease (IBD) in a subject, including a human, comprising the step of administering to said subject in need thereof a therapeutically effective amount of an anti-oxMIF antibody or antigen-binding portion thereof.

In a specific embodiment, the antibodies highly suitable for the treatment of inflammatory or infectious diseases are recombinant anti-oxMIF antibodies having a silenced Fc and reduced aggregation potential and reduced hydrophobicity, comprising the following variable domains:
(a1) a light chain variable domain comprising SEQ ID NO:2 with 1, 2, 3, 4, or 5 amino acid substitutions selected from M30L, F49Y, A51G, P80S, W93F, or
(a2) a light chain variable domain comprising SEQ ID NO:2 with 1, 2, 3, 4, or 5 amino acid substitutions selected from M30L, F49Y, A51G, P80S, W93F and with 1, 2, 3, 4, or 5 further amino acid substitutions, with the proviso that the conserved tyrosine at position 36 is preserved; and
(b1) a heavy chain variable domain comprising SEQ ID NO:3; or
(b2) a heavy chain variable domain comprising SEQ ID NO:3 with 1 or 2 amino acid substitutions selected from L5Q and W97Y, or
(b3) a heavy chain variable domain comprising SEQ ID NO:3 with 1, or 2 amino acid substitutions selected from L5Q and W97Y, and 1, 2, 3, 4 or 5 further amino acid substitutions;
wherein amino acid positions are numbered according to Kabat,
wherein the variant Fc region exhibits decreased FcγR binding compared to the wildtype IgG1 Fc region, wherein the one or more amino acid substitutions in the variant Fc region are at any one of positions E233, L234, L235, G236, G237, P238, D265, S267, H268, N297, S298, T299, E318, L328, P329, A330, P331 of SEQ ID NO:1, specifically the amino acid substitutions are at positions L234 and L235, according to EU numbering index, and/or wherein the Fc region is aglycosylated, and
wherein said antibody has reduced aggregation potential and reduced hydrophobicity compared to an antibody comprising SEQ ID NO:2 and SEQ ID NO:3 lacking the amino acid substitutions.

In a further specific embodiment, the antibodies highly suitable for the treatment of inflammatory or infectious diseases, are recombinant anti-oxMIF antibodies described herein, having also increased preferential binding to inhibitory receptor FcγRIIB, or have hyper alpha 2,6- N-linked sialylation.

### EXAMPLES

The examples described herein are illustrative of the present invention and are not intended to be limitations thereon. Accordingly, it should be understood that the examples are illustrative only and are not limiting upon the scope of the invention.

### Example 1: Variant IDs, sequence combinations and sequences of the anti-oxMIF antibodies (Table 5 A) and anti-oxMIF x anti-HSG bispecific antibodies (Table 5 B) used within the examples section.

**Table 5 A:**

| Variant ID (Sample) | VH | CH | VL | CL |
|---|---|---|---|---|
| C0008 (control/ reference antibody) | wt (SEQ ID NO:3) | wt (SEQ ID NO: 44) | wt (SEQ ID NO:2) | SEQ ID NO:18 |
| C0083 | L5Q/W97Y (SEQ ID NO:4) | wt (SEQ ID NO: 44) | F49Y/A51G/W93F (SEQ ID NO:6) | SEQ ID NO:18 |
| C0090 | L5Q/W97Y (SEQ ID NO:4) | wt (SEQ ID NO: 44) | M30L/F49Y/A51G/P80S/W93F (SEQ ID NO:8) | SEQ ID NO:18 |
| C0115 | L5Q/W97Y (SEQ ID NO:4) | L234A/L235A (SEQ ID NO:14) | F49Y/A51G/W93F (SEQ ID NO:6) | SEQ ID NO:18 |
| C0118 | L5Q/W97Y (SEQ ID NO:4) | L234A/L235A (SEQ ID NO:14) | M30L/F49Y/A51G/P80S /W93F (SEQ ID NO:8) | SEQ ID NO:18 |

**Table 5 B:**

| Variant ID (Sample) | Heavy chain 1 | Heavy chain 2 | Light chain |
|---|---|---|---|
| C0132 | (SEQ ID NO:69) | (SEQ ID NO:70) | (SEQ ID NO:10) |
| C0133 | (SEQ ID NO:69) | (SEQ ID NO:71) | (SEQ ID NO:10) |

### Example 2: Reduced aggregation propensity and reduced hydrophobicity of newly designed anti-oxMIF antibodies

To evaluate hydrophobicity and aggregation the newly designed antibodies C0115 and C0118 were analyzed by gel filtration (SEC) using two different SEC columns and running buffer conditions and by hydrophobic interaction chromatography (HIC) compared to the control anti-oxMIF antibody C0008 and their parent antibodies C0083 and C0090 without Fc silencing.

For SEC, samples were diluted to 1 mg/ml in 1x phosphate buffered saline (1xPBS) and 100 µl sample was applied to an Enrich 650 (Bio-Rad) gel-filtration column at a flow rate of 1.25 ml/min. Separations and equilibration was performed in 1xPBS at room temperature. Protein peaks were monitored using absorbance at 280 nm and spectra were analyzed using the ChromLab software package (Bio-Rad). Results are reported in retention volume (Vr, ml) for the main peak and presence of aggregates was ranked manually.

For hydrophobic interaction chromatography (HIC) analysis, all samples were diluted to a final concentration of 1 mg/ml using 50mM phosphate and 0.75 M Ammonium Sulphate, pH 6.9. Highly purified samples of antibodies (~100 µg) were loaded independently onto a 1ml HiTrap Butyl HP column. 100 µl samples were injected and the column flow rate was maintained at 1 ml/min at 22°C. Separation of peaks was carried out over a 20-column volume (CV) gradient from 0-100%B (Buffer B: 50 mM phosphate, 20% isopropanol; pH7.0). Protein peaks were monitored using absorbance at 280 nm and spectra were analyzed using the ChromLab software package (Bio-Rad).

Results: Control antibody C0008 demonstrated a retention volume close to the void volume of the size exclusion column (~16-18 ml Enrich 650), which corresponds to a molecular weight far smaller than expected for a human IgG **(****Figure 1** **A** and **B).** The unusual long retention was mainly due to hydrophobic interactions with the stationary phase surface. In addition, to the high retention volume, significant amounts of IgG dimers and aggregates were present for C0008. All newly designed antibodies showed a reduced retention volume (Vr) demonstrating reduced interaction with the column and thus reduced hydrophobicity of the molecules (Table 6, **Figure 1** **A** and **B).** The newly designed antibodies C0115 and C0118 showed a retention volume (Table 6, **Figure 1B)** corresponding to the molecular weight of a monomeric human IgG, when compared to a molecular weight standard. Additionally, antibody dimers and aggregation were significantly reduced in samples of newly designed antibodies C0115 and C0118 **(****Figure 1B)** The Fc silencing mutations of the new newly designed antibodies C0115 and C0118, did not alter their SEC profile compared to their parent antibodies C0083 and C0090 with wt Fc, respectively.

HIC column retention volumes, are a measure of hydrophobicity where antibodies with low retention volume are less hydrophobic than antibodies with high retention volume. (**Figure 1** **C**). The newly designed antibodies C0115 and C0118 demonstrated to be less hydrophobic (retention volume of 15-16 ml), compared the control antibody C0008 (retention volume of ~21 ml) showing a very high hydrophobicity. The Fc silencing mutations of the new newly designed antibodies C0115 and C0118, did not alter their HIC profile compared to their parent antibodies C0083 and C0090 with wt Fc, respectively.

Conclusion: It is evident from SEC and HIC analysis that the newly designed antibodies have improved biochemical properties, in particular reduced hydrophobicity, and aggregation propensity compared to the control anti-oxMIF antibody C0008.

**Table 6 Size Exclusion Chromatography**

| **Sample** | **Running Buffer** | **Column** | **Vr (ml)** | **Aggregates** |
|---|---|---|---|---|
| **C0008** | 1xPBS | Enrich 650 | 18.06 | + |
| **C0083** | 1xPBS | Enrich 650 | 12.39 | - |
| **C0090** | 1xPBS | Enrich 650 | 12.38 | - |
| **C0115** | 1xPBS | Enrich 650 | 11.96 | - |
| **C0118** | 1xPBS | Enrich 650 | 11.95 | - |

**Fig. 1** shows the Chromatography profiles demonstrating reduced aggregation and hydrophobicity of the newly designed anti-oxMIF antibodies. (**A**) Comparison of elution profiles of C0008 (control antibody, gray area) and the parent antibodies (C0083 and C0090, without Fc silencing) of the newly designed antibodies C0115 and C0118 on a Enrich 650 gel filtration column using 1xPBS as mobile phase; (**B**) Comparison of elution profiles of C0008 (control antibody, gray area) and newly designed antibodies C0115 and C0118 on a Enrich 650 gel filtration column using 1xPBS as mobile phase (**C**) Comparison of elution profiles of C0008 (control antibody, gray area) and newly designed antibodies C0115 and C0118 and their parent antibodies C0083 and C0090 (without Fc silencing) on a HiTrap Butyl HP HIC column.

### Example 3: Binding of newly designed anti-oxMIF antibodies to immobilized MIF (K_{D} determination)

Recombinant human MIF diluted in PBS at 1 µg/ml was immobilized into ELISA plates overnight at 4°C (transforming MIF to oxMIF according to Thiele et al., 2015). After blocking, serial dilutions of anti-oxMIF antibodies were added to the plates. Finally, bound antibodies were detected using a Goat anti-human IgG (Fc)-HRP conjugate and tetramethylbenzidine (TMB) as substrate. The chromogenic reaction was stopped with 3M H₂SO₄ and OD was measured at 450 nm. Data from different experiments were normalized to the maximal OD of the anti-oxMIF antibody C0008 (=100%) of the respective experiment and EC₅₀ values were determined by 4-parameter fit using GraphPad Prism (and the mean +/- SEM of two experiments is shown).

Results and conclusion: The binding of the newly designed antibodies towards immobilized MIF (oxMIF) was measured over a broad range of concentrations and the resulting binding curves are illustrated in **Figure 2****.** The anti-oxMIF antibody C0008 was used as reference for oxMIF binding. The binding curves and the calculated EC₅₀ values, representing the apparent K_{D}, clearly showed that the newly designed antibodies C0115 and C0118 retained their low nanomolar affinity to oxMIF, compared to C0008 (**Figure 2****,** Table 7).

**Figure 2** shows the binding curves of newly designed anti-oxMIF antibodies to immobilized oxMIF (K_{D} determination). Anti-oxMIF antibodies were detected by an anti-human-IgG (Fc)-HRP conjugate, C0008 was used as reference antibody. EC₅₀ values were determined by sigmoidal, 4-parameter equation using GraphPad Prism (mean +/-SEM of two experiments is shown)

**Table 7: KD values of anti-oxMIF antibodies**

| **Sample** | **K_{D} (nM)** |
|---|---|
| C0008 | 0.19 |
| C0115 | 0.28 |
| C0118 | 0.26 |

### Example 4: Differential binding of newly designed anti-oxMIF antibodies to oxMIF vs. redMIF.

Anti-oxMIF antibodies and a human IgG isotype control were immobilized into microplates over night at 4°C at a concentration of 15 nM. After blocking, wells were incubated with 50 ng/ml of either redMIF or the oxMIF surrogate NTB-MIF (Schinagl et al., 2018). Captured oxMIF was detected with a polyclonal rabbit anti-MIF antibody and goat anti-rabbit IgG-HRP. Plates were stained with tetramethylbenzidine (TMB), and chromogenic reaction was stopped by addition of 30% H₂SO₄. OD was measured at 450 nm. Data from different experiments were normalized to the maximal OD of the anti-oxMIF antibody C0008 (=100%) of the respective experiment, and the mean +/- SEM of two to three experiments is shown.

Results and conclusion: The binding of the newly designed antibodies towards soluble oxMIF and redMIF are illustrated in **Figure 3****.** The results clearly showed that the newly designed antibodies C0115 and C0118 demonstrated strong binding to oxMIF but no binding to redMIF. The newly designed antibodies showed very comparable ODs to the reference antibody C0008, which has been described to discriminate between oxMIF and redMIF (Thiele et al., 2015). No binding was observed for the isotype IgG. Thus, the newly designed antibodies with reduced hydrophobicity and aggregation and Fc silencing retained their ability to discriminate between oxMIF and redMIF.

**Figure 3** shows the differential binding of the newly designed antibodies to oxMIF vs. redMIF. C0008 was used as reference antibody and an Isotype IgG as negative control. Mean +/- SEM of two to three experiments is shown

### Example 5: Strongly reduced effector functions of the newly designed anti-oxMIF antibodies C0115 and C0118 determined by reporter assays.

As described above, efforts can be made to decrease ADCC and ADCP potential of antibodies by point mutations in the Fc portion, i.e. L234A/L235A. When the Fc portion of target-bound antibodies also binds to Fc receptors on the cell surface of effector cells, multiple cross-linking of the two cell types occurs, leading to pathway activation of ADCC or ADCP, which are not desired for target neutralizing antibodies to prevent functional Fc-related adverse events.

Using engineered Jurkat cells as effector cells either stably expressing the human FcγRIIIa V158 (high affinity genotype) to elucidate ADCC, or the human FcγRIIa-H131 to elucidate ADCP, and a NFAT response element driving expression of firefly luciferase, antibody biological activity is quantified through the luciferase produced as a result of NF-AT pathway activation in effector cells.

The ADCC or ADCP reporter assays were essentially performed as recommended by the manufacturer (Promega #G7010 & #G9991).

To generate highly responsive target cells, HCT116 and A2780 cells were transfected with a huMIF-pDisplay plasmid (Invitrogen), selected with geneticin, and sorted by FACS to generate cell lines stably expressing membrane-anchored monomeric human MIF (HCT116-pMIF or A2780-pMIF), i.e. MIF is displayed as monomeric protein in which an oxMIF epitope is accessible to anti-oxMIF antibodies (Schinagl et al., Biochemistry 2018). These cell lines show increased presentation of oxMIF at the cellular surface and are therefore a more sensitive tool for *in vitro* analysis. In brief, 1x10⁴ cells/well of HCT116-pMIF (**Figure 4** **A** and **C**) or A2780-pMIF (**Figure 4** **B**) cells in 100 µl culture medium (RPMI 1640 medium supplemented with Pen/Strept/L-Glutamine and 4% low IgG FBS) were seeded into 96-well plates and left to adhere overnight at 37°C/5% CO₂ in a humidified incubator. On the day after, culture medium was removed and replaced with 25 µl of the fresh culture medium. 25 µl of serial dilutions of the newly designed anti-oxMIF antibodies C0115 & C0118 with silenced Fc or their parent antibodies C0083, C0090 and the control antibody C0008 with wt Fc (final concentration 0.01-100 nM) and 25 µl of Jurkat effector cells (**Figure 4A-B,** FcγRIIIa receptor effector cells of high responder genotype V158; **Figure 4** **C,** FcγRIIa receptor effector cells) at a effector to target cell ratio of ~6:1 were added, and cells incubated with antibodies and effector cells for 6h at 37°C / 5°CO₂ in a humidified incubator. Finally, the assay plates were equilibrated to room temperature and 75 µl of Bio-Glo Luciferase reagent was added. Luminescence (RLU) was measured after 10-20 min incubation using a Tecan multiplate reader (0.5 s integration time). Data (where appropriate) were fitted to a sigmoidal, 4-parameter equation using GraphPad Prism (mean +/- SD of two replicates is shown).

Results: It is evident from Figure 4 A-C that the newly designed antibodies C0115 & C0118 harboring Fc silencing mutations did not show any activation of the reporter cells in the ADCC (**Figure 4A-B** ) or ADCP (**Figure 4** **C**) reporter assays, whereas the control antibody C0008 (**Figure 4** **B**) and their parent antibodies with wt Fc, C0083 and C0090 (**Figure 4** **A**), induced strong FcyRllla- (ADCC, **Figure 4A-B** ) or FcyRlla- (ADCP, **Figure 4** **C**) mediated activation of effector cells.

Conclusion: The newly designed variants C0115 & C0118 harboring Fc silencing mutations (L234A/L235A) did not show any initiation of either ADCC or ADCP in reporter bioassays. Thus, they show strongly reduced ADCC and ADCP effector functions.

**Figure 4** shows the strongly reduced effector functions of the newly designed Fc silenced antibodies C0115 & C0118 determined by reporter assays. (**A-B**) ADCC reporter bioassay with the newly designed Fc silenced antibodies C0115 and/or C0118 using engineered Jurkat effector cells stably expressing FcyRllla and HCT116-pMIF (**A**) or A2780-pMIF (**B**) target cells compared either to the anti-oxMIF control antibody C0008 (**B**) or their parent antibodies C0083 and C0090 with wt Fc (**A**); (**C**) ADCP reporter bioassay with the newly designed Fc silenced antibodies C0115 and/or C0118 using engineered Jurkat effector cells stably expressing FcyRlla and HCT116-pMIF target cells compared to their parent antibodies C0083 and C0090 with wt Fc. Data were fitted to a sigmoidal, 4-parameter equation using GraphPad Prism (mean +/- SD of two replicates is shown).

### Example 6: Strongly reduced CDC function of the newly designed anti-oxMIF antibody C0115 determined by a complement-dependent cytotoxicity (CDC) assay.

This assay was performed to determine lysed cells resulting from the antibody-driven complement dependent cytotoxicity (CDC) induced by newly designed anti-oxMIF antibodies. Cytotoxicity was measured by HiBiT Detection assay (Promega) which quantifies the release of a HiBiT tagged protein from the target cells using a non-lytic detection reagent containing LgBiT (LargeBiT) and furimazine (substrate). HiBiT and LgBiT spontaneously assemble to a functional NanoBiT^{®} enzyme and emits a quantifiable luminescence signal in the presence of the substrate.

To generate highly responsive reporter target cells, HCT116 cells were transfected with the HaloTag-HiBiT plasmid (Promega #CS1956B17), selected with blasticidin and sorted by FACS as cell pools. Stable HiBiT-expressing cell pools were then transfected with a huMIF-pDisplay plasmid (Invitrogen), selected with geneticin, and sorted by FACS to generate cell pools stably expressing intracellular HiBiT and membrane-anchored monomeric human MIF (HCT116-HiBiT-pMIF), i.e. MIF is displayed as monomeric protein in which an oxMIF epitope is accessible to anti-oxMIF antibodies (Schinagl et al., Biochemistry 2018). These cell lines show increased presentation of oxMIF at the cellular surface and are therefore a more sensitive tool for in vitro analysis.

In brief, 1x10⁴ cells/well of HCT116-HiBiT-pMIF cells in 100 µl culture medium (RPMI 1640 medium supplemented with Pen/Strept/L-Glutamine and 10% FBS) were seeded into 96-well plates and left to adhere overnight at 37°C and 5% CO₂ in a humidified incubator. On the day after, culture medium was removed and 50 µl of serum-free RPMI 1640, and 50 µl of serial dilutions of the newly designed anti-oxMIF antibodies C0115 with silenced Fc or its parent antibody C0083 with wt Fc and nivolumab (human IgG4, negative control) in serum-free RPMI 1640 (final concentration 1-100 nM) were added. After incubation of antibodies with cells for 30 min at 37°C, 50 µl of baby rabbit complement (BRC, Sedarlane, diluted 1:10 in serum-free RPMI shortly prior to the assay) were added to the plates. After adding the complement, the plate was incubated overnight at 37°C and 5% CO₂ in a humidified incubator. On the next day 10 µl of Nano-Glo HiBiT Extracellular Detection Reagent (Promega #N2421) was added, and luminescence signals (RLU, integration time 0.1 s) were measured 3 min later on a Tecan plate reader. Data (where appropriate) were fitted to a sigmoidal, 4-parameter equation using GraphPad Prism (mean +/- SD of two replicates is shown).

Results & Conclusion: Figure 5 clearly shows that the newly designed antibody C0115 harboring Fc silencing mutations (L234A/L235A) did not induce any CDC activity and the measured signals were even lower than the negative control (nivolumab, IgG4). In contrast, the parent antibody C0083 with wt Fc showed complement dependent cell lysis of HCT116-pMIF cells, as expected for a human IgG1. Thus, the newly designed antibody C0115 harboring Fc silencing (L234A/L235A) mutations shows strongly reduced CDC activity.
**Figure 5** shows strongly reduced CDC activity of the newly designed Fc silenced antibody C0115 determined by a complement dependent cell toxicity bioassay. CDC bioassay with the newly designed Fc silenced antibody C0115 using BRC as source of complement and HCT116-pMIF as target cells compared to its parent anti-oxMIF antibody C0083 with a wt Fc and nivolumab as an IgG4 negative control. Data (where appropriate) were fitted to a sigmoidal, 4-parameter equation using GraphPad Prism (mean +/- SD of two replicates is shown).

### Example 7: Strongly reduced ADCC function of the newly designed anti-oxMIF antibodies C0115 and C0118 determined by a PBMC cell lysis bioassay.

This assay was performed to determine cell lysis resulting from the antibody-dependent cytotoxicity (ADCC) induced the by newly designed anti-oxMIF antibodies. Cytotoxicity was measured by HiBiT Detection assay (Promega) which quantifies the release of a HiBiT tagged protein from the target cells using a non-lytic detection reagent containing LgBiT (LargeBiT) and furimazine (substrate). HiBiT and LgBiT spontaneously assemble to a functional NanoBiT^{®} enzyme and emits a quantifiable luminescence signal in the presence of the substrate.

HCT116-HiBiT-pMIF reporter target cells were generated as described in Example 6.

In brief, 1x10⁴ cells/well of HCT116-HiBiT-pMIF target cells in 50 µl culture medium (RPMI 1640 medium supplemented with Pen/Strept/L-Glutamine and 5% ultra-low IgG FBS (Thermo Scientific) were seeded into 96-well plates and left to adhere overnight at 37°C/5% CO₂ in a humidified incubator. On the day after, 50 µl serial dilutions of the newly designed anti-oxMIF antibodies C0115 and C0118 with silenced Fc or the parent antibody of C0115 (C0083 with wt Fc) and/or the reference antibody C0008 with wt Fc in culture medium (final concentration 0.01-100 nM) and 50 µl of human PBMCs effector cells from two healthy donors (4x10⁵ cells/well; effector to target cell ratio 40:1) were added to the plates. After adding antibodies and PBMCs, the plate was incubated over night at 37°C and 5% CO₂ in a humidified incubator. On the next day, 10 µl of Nano-Glo HiBiT Extracellular Detection Reagent (Promega #N2421) was added, and luminescence signals (RLU, integration time 0.1 s) were measured 3 min later on a Tecan plate reader.

Results & Conclusion: It is obvious from **Figure 6** that the newly designed antibodies C0115 and C0118 harboring Fc silencing (L234A/L235A) mutations did not show any or strongly reduced ADCC activity. In contrast, the parent antibody of C0115 (C0083, with wt Fc) and the control anti-oxMIF antibody C0008 with wt Fc showed antibody dependent cell lysis of HCT116-HiBiT-pMIF cells, as expected for a human IgG1. Thus, the newly designed antibody C0115 harboring Fc silencing (L234A/L235A) mutations shows strongly reduced ADCC activity.
**Figure 6** shows strongly reduced ADCC activity of the newly designed Fc silenced antibodies C0115 and C0118 determined by a PBMC mediated cell toxicity bioassay. ADCC bioassay with the newly designed Fc silenced antibodies C0115 (**A**) and C0118 (**B**) using PBMCs as effector cells and HCT116-HiBiT-pMIF as target cells compared to the anti-oxMIF control antibody C0008 (**B**) and the parent anti-oxMIF antibody of C0115, namely C0083 (**A**) with wt Fc. Mean and SEM of two replicates using PBMCs from two healthy donors are shown. Data were fitted to a sigmoidal, 4-parameter equation using GraphPad Prism

### Example 8: Reduced unspecific binding of the newly designed anti-oxMIF antibodies C0115 and C0118 on A2780 MIF^{-/-} cells.

Materials and Methods. A2780 MIF ^{-/-} cell line was generated by CRISPR/Cas9 gene editing of human MIF gene in A2780 ovarian carcinoma cell line. In brief, target gene sequence was analysed and target sites were located according to the general rules of designing a targeting guidance RNA (gRNA) for GenCRISPR^{™} system. A guide RNA (gRNA) was designed to specifically recognize the 5' region of the MIF gene (TTGGTGTTTACGATGAACATCGG, SEQ ID NO:40) and the gRNA sequence was cloned into the PX459 (addgene) vector containing S. pyogenes Cas9 (SpCas9) nuclease. A2780 cells were transiently transfected by electroporation and were plated in 96-well plates by limit dilution to generate isogenic single clones. Isogenic single clones, where the endogenous MIF gene was efficiently mutated, resulting in consequential reduction (or removal) of the expression of the MIF protein were identified by Sanger sequencing screening. The final clone showed a deletion of 10 bp at position +2 after the start codon of the human MIF gene. Absence of endogenous human MIF protein in the A2780 MIF ^{-/-} cell line was confirmed by Western blotting using polyclonal anti-human MIF antibodies.

A2780 MIF ^{-/-} cells were detached with Cell Stripper (Corning, Cat# 25-056-CI), washed with staining buffer (PBS +5% BSA) and plated into 96-well U-bottom plate at 2x10⁵ cells per well. Cells were stained with fixable viability dye eFluor780 (ThermoFisher, diluted 1:2000 in PBS) for 20 min at 4°C and washed with staining buffer. Cells were resuspended in 50 µl of staining buffer, and 50 µl of serial dilutions of the newly designed anti-oxMIF antibodies C0115 and C0118 or their parent antibodies C0083 and C0090, respectively, or the control anti-oxMIF antibody C0008 or an isotype IgG (final concentrations 37 nM -9.4 nM) were added. After incubating for 40 min at 4°C, cells were washed with staining buffer, and resuspended in 100 µl of secondary antibody (goat anti-human IgG (H+L)-AlexaFluor 488, diluted 1:100). After incubating for 30 min at 4°C, cells were washed with staining buffer, resuspended in PBS+2% BSA and acquired on the CytoFlex-S flow cytometer (Beckman Coulter).

Data were analyzed with FlowJo (BD) and the GeoMean (mean fluorescence intensity in AF488 channel) of viable cells was plotted against antibody concentration in GraphPad Prism.

Results & Conclusion: It is apparent from **Figure 7** **A** and **B** that the newly designed anti-oxMIF antibody C0118 and its parent antibody C0090 (A) as well as C0118 and its parent antibody C0083 (B) do not bind to A2780 MIF^{-/-} cells, as their Geo Mean is very close to those of the isotype IgG negative control. In contrast, the anti-oxMIF antibody C0008 showed significant binding to A2780 MIF^{-/-} cells, although they were proven not to express MIF. Thus, reduction of hydrophobicity resulted in a strong reduction or elimination of unspecific binding of the newly designed anti-oxMIF antibodies to A2780 MIF^{-/-}, whereas the anti-oxMIF control antibody C0008 binds unspecifically to the cell surface due to its hydrophobic properties.
**Figure 7** shows the reduced unspecific binding of the newly designed anti-oxMIF antibodies on A2780 MIF^{-/-} cells determined by FACS. Staining of A2780 MIF^{-/-} cells with the newly designed anti-oxMIF antibody C0118 and its parent antibody C0090 (**A**) and C0115 and its parent antibody C0083 (**B**) and the control antibody C0008 as well as an isotype IgG as negative control; GeoMean (mean fluorescence intensity in AF488 channel) of viable cells is plotted against antibody concentration.

### Example 9: Strongly reduced unspecific cytokine release from human PBMCs by newly designed anti-oxMIF antibody C0115.

Cytokine release syndrome (CRS) is a form of systemic inflammatory response syndrome (SIRS) that can be triggered by a variety of factors such as infections. CRS is also known as an adverse effect of some monoclonal antibody medications. CRS occurs when large numbers of white blood cells, including B cells, T cells, natural killer cells, macrophages, dendritic cells, and monocytes are activated and release inflammatory cytokines, like IL-6, IFN-y, IL-8, MCP-1 amongst others, which activate more white blood cells in a positive feedback loop of pathogenic inflammation. This process, when dysregulated, can be life-threatening due to systemic hyper-inflammation, hypotensive shock, and multi-organ failure. Thus, the newly designed anti-oxMIF antibody C0115 was assessed for their potential to release inflammatory cytokines from PMBCs in an *in-vitro* assay.

Materials and Methods: The newly designed anti-oxMIF antibody C0115 and the anti-oxMIF reference antibody C0008 were incubated at 70 nM, 7 nM, 0.7 nM, 0.07 nM with freshly thawed PBMCs (4-5x10⁵ cells per well) from three healthy donors, or with medium only, in 150 µl of RPMI1640 medium supplemented with 5% ultra-low IgG serum in 96-well plates. After overnight incubation at 37°C/5% CO₂ in a humidified incubator, plates were centrifuged at 300 x g for 5 min to pellet the cells, and cleared supernatants were transferred to the 96-well V-bottom plates (BioLegend). Supernatants were analyzed by BioLegend LegendPlex cytometric bead assays for human MCP-1, human IL-6, and human TNF-α according to manufacturer's protocol. Measurement was done on a CytoFlex-S cytometer with 96-well plate loader (Beckman Coulter). Beads classification channel was APC (excitation from laser 638 nm, band pass filter 660/10 nm), whereas reporter channel was PE (excitation from yellow laser 561 nm, band pass filter 585/42 nm). Data were analyzed using LegendPlex analysis software (BioLegend) and graphs were produced in GraphPad Prism. Mean +/- SEM are shown from 3 different PMBC donors.

Results and Conclusion: It is evident from **Figure 8** that the newly designed antibody C0115 harboring manufacturability mutations in its variable regions and Fc-silencing mutations (L234A/L235A) showed undetectable or minor release of MCP-1, IL-6 and TNF-α from PBMCs at concentrations up to 70 nM. Reference anti-oxMIF antibody C0008 induced significant release of MCP-1, IL-6 and TNF-α at the highest concentration (70 nM), in line with its higher aggregation propensity and unspecific binding due to its hydrophobicity. Aggregation of antibody therapeutics, even if minor, is known to strongly potentiate cytokine release from immune cells.

**Figure 8** illustrates that the newly designed anti-oxMIF antibody C0115 shows strongly reduced cytokine release from human PBMCs compared to reference antibody C0008. The anti-oxMIF antibody C0115 harboring Fc-silencing mutations and the reference anti-oxMIF antibody C0008 were incubated at the broad concentration range (70 nM, 7 nM, 0.7 nM, 0.07 nM) with human PBMCs overnight, and supernatants were analyzed in LegendPlex cytometric bead assays (BioLegend) for human MCP-1 (**A**), human IL-6 (**B**) and human TNF-α (**C**). Mean +/- SEM for cytokine concentrations (in pg/ml) are shown from 3 different PMBC donors.

### Example 10: Biodistribution of newly designed anti-oxMIF antibody C0115 and control antibody C0008 in Balb/c nude mice bearing xenografted human HCT116 colon carcinoma tumors.

Materials and Methods: Biodistribution of newly designed anti-oxMIF antibody C0115 was investigated in female Balb/c nude mice carrying subcutaneous tumors of the human colon cancer cells HCT116 in comparison to the reference anti-oxMIF antibody C0008. Female Balb/c nude mice received unilateral, subcutaneous injections of 5x10⁶ HCT116 cells in 50% PBS and 50% matrigel in a total injection volume of 100 µl. Upon reaching individual tumor volumes of 150-300 mm³, mice were assigned to treatment groups and received a single intravenous dose of 5 mg/kg IRDye 800CW-labeled C0115 or C0008.

C0115 and C0008 were labelled with IRDye 800CW using the IRDye 800CW Protein labeling kit (high MW from LI-COR Biosciences) following the manufacturer's instructions. After the labelling process and prior to injection of labeled antibodies into mice, the protein concentration and labeling efficiency of the IRDye 800CW labeled antibodies was determined using the Nanodrop technology, and mice were dosed based on the protein concentration after labelling. *In vivo* imaging was performed in a LI-COR Pearl^{®} Trilogy imaging device upon administration of labelled antibodies at the following time-points: 1h, 6-8h, 24h, 48h, 72h, 96h, 168h after dosing. Image analysis was performed to quantify the relative fluorescence intensity (RFU) of the antibody in the tumor (RFU/area= RFU tumor area/mm² tumor area - RFU background/mm² background area).

Results and Conclusion: **Figure 9** shows a significant intra-tumoral distribution of intravenously administered IRDye 800CW-labeled C0115 and C0008, respectively, with tumor retention up to 7 days. It is evident from **Figure 9(A)** and quantitative image analysis **(****Figure 9(B)** **)** that the tumor uptake of newly designed anti-oxMIF antibody C0115 was strongly enhanced and increased over 7 days compared to the reference anti-oxMIF antibody C0008, which showed a peak at ~24h.
**Figure 9** shows the tumor penetration and retention of the newly designed anti-oxMIF antibody C0115 and the reference antibody C0008 by infra-red *in vivo* imaging of mice carrying subcutaneous HCT116 tumors. (**A**) Infra-red images of mice were taken 1h, 6h, 24h, 48h, 72h, 96h and 168h post injection of the IRDye 800CW-labeled antibodies C0115 (upper panel) and C0008 (lower panel) dosed at 5 mg/kg; (**B**) tumor penetration and retention of C0115 and C0008 quantified by digital image analysis. Mean of 3 mice is shown.

### Example 11:

**Table 8: Overview on amino acid sequences**

| SEQ ID NO | Sequence | Specification |
|---|---|---|
| 1 | APELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPI EKTISKAKGQPREPQVYTLPPSREEMTKNQVSLTCLVKGFYPSDIAVEWES NGQPENNYKTTPPVLDSDGSFFLYSKLTVDKSRWQQGNVFSCSVMHEALH NHYTQKSLSLSPG | CH2-CH3 wt |
| 2 | DIQMTQSPSSLSASVGDRVTITCRSSQRIMTYLNWYQQKPGKAPKLLIFVAS HSQSGVPSRFRGSGSETDFTLTISGLQPEDSATYYCQQSFWTPLTFGGGT KVEIK | VL Wt |
| 3 | EVQLLESGGGLVQPGGSLRLSCAASGFTFSIYSMNWVRQAPGKGLEWVSS IGSSGGTTYYADSVKGRFTISRDNSKNTLYLQMNSLRAEDTAVYYCAGSQW LYGMDVWGQGTTVTVSS | VH Wt |
| 4 | EVQLQESGGGLVQPGGSLRLSCAASGFTFSIYSMNWVRQAPGKGLEWVS SIGSSGGTTYYADSVKGRFTISRDNSKNTLYLQMNSLRAEDTAVYYCAGSQ YLYGMDVWGQGTTVTVSS | VH L5Q/W97Y |
| 5 | DIQMTQSPSSLSASVGDRVTITCRSSQRIMTYLNWYQQKPGKAPKLLIYVGS HSQSGVPSRFRGSGSETDFTLTISGLQPEDSATYYCQQSFWTPLTFGGGT KVEIK | VL F49Y/A51G |
| 6 | DIQMTQSPSSLSASVGDRVTITCRSSQRIMTYLNWYQQKPGKAPKLLIYVGS HSQSGVPSRFRGSGSETDFTLTISGLQPEDSATYYCQQSFFTPLTFGGGTK VEIK | VL F49Y/A51G/W9 3F |
| 7 | DIQMTQSPSSLSASVGDRVTITCRSSQRILTYLNWYQQKPGKAPKLLIYVGS HSQSGVPSRFRGSGSETDFTLTISGLQPEDSATYYCQQSFFTPLTFGGGTK VEIK | VL M30L/F49Y/A51 G/W93F |
| 8 | DIQMTQSPSSLSASVGDRVTITCRSSQRILTYLNWYQQKPGKAPKLLIYVGS HSQSGVPSRFRGSGSETDFTLTISGLQSEDSATYYCQQSFFTPLTFGGGTK VEIK | VL M30L/F49Y/A51 G/P80S/W93F |
| 9 | EVQLQESGGGLVQPGGSLRLSCAASGFTFSIYSMNWVRQAPGKGLEWVS SIGSSGGTTYYADSVKGRFTISRDNSKNTLYLQMNSLRAEDTAVYYCAGSQ WLYGMDVWGQGTTVTVSS | VH L5Q |
| 10 | DIQMTQSPSSLSASVGDRVTITCRSSQRIMTYLNWYQQKPGKAPKLLIYVGS HSQSGVPSRFRGSGSETDFTLTISGLQPEDSATYYCQQSFFTPLTFGGGTK VEIKRTVAAPSVFIFPPSDEQLKSGTASVVCLLNNFYPREAKVQWKVDNALQ SGNSQESVTEQDSKDSTYSLSSTLTLSKADYEKHKVYACEVTHQGLSSPVT KSFNRGEC | C0083, C0115, C0132, C0133 light chain |
| 11 | EVQLQESGGGLVQPGGSLRLSCAASGFTFSIYSMNWVRQAPGKGLEWVS SIGSSGGTTYYADSVKGRFTISRDNSKNTLYLQMNSLRAEDTAVYYCAGSQ YLYGMDVWGQGTTVTVSSASTKGPSVFPLAPSSKSTSGGTAALGCLVKDY FPEPVTVSWNSGALTSGVHTFPAVLQSSGLYSLSSVVTVPSSSLGTQTYIC NVNHKPSNTKVDKRVEPKSCDKTHTCPPCPAPELLGGPSVFLFPPKPKDTL MISRTPEVTCVVVDVSHEDPEVKFNWYVDGVEVHNAKTKPREEQYNSTYR VVSVLTVLHQDWLNGKEYKCKVSNKALPAPIEKTISKAKGQPREPQVYTLPP SREEMTKNQVSLTCLVKGFYPSDIAVEWESNGQPENNYKTTPPVLDSDGS FFLYSKLTVDKSRWQQGNVFSCSVMHEALHNHYTQKSLSLSPG | C0083 heavy chain |
| 12 | DIQMTQSPSSLSASVGDRVTITCRSSQRILTYLNWYQQKPGKAPKLLIYVGS HSQSGVPSRFRGSGSETDFTLTISGLQSEDSATYYCQQSFFTPLTFGGGTK VEIKRTVAAPSVFIFPPSDEQLKSGTASVVCLLNNFYPREAKVQWKVDNALQ SGNSQESVTEQDSKDSTYSLSSTLTLSKADYEKHKVYACEVTHQGLSSPVT KSFNRGEC | C0090 light chain |
| 13 | EVQLQESGGGLVQPGGSLRLSCAASGFTFSIYSMNWVRQAPGKGLEWVS SIGSSGGTTYYADSVKGRFTISRDNSKNTLYLQMNSLRAEDTAVYYCAGSQ YLYGMDVWGQGTTVTVSSASTKGPSVFPLAPSSKSTSGGTAALGCLVKDY FPEPVTVSWNSGALTSGVHTFPAVLQSSGLYSLSSVVTVPSSSLGTQTYIC NVNHKPSNTKVDKRVEPKSCDKTHTCPPCPAPELLGGPSVFLFPPKPKDTL MISRTPEVTCVVVDVSHEDPEVKFNWYVDGVEVHNAKTKPREEQYNSTYR VVSVLTVLHQDWLNGKEYKCKVSNKALPAPIEKTISKAKGQPREPQVYTLPP SREEMTKNQVSLTCLVKGFYPSDIAVEWESNGQPENNYKTTPPVLDSDGS FFLYSKLTVDKSRWQQGNVFSCSVMHEALHNHYTQKSLSLSPG | C0090 heavy chain |
| 14 | ASTKGPSVFPLAPSSKSTSGGTAALGCLVKDYFPEPVTVSWNSGALTSGVH TFPAVLQSSGLYSLSSVVTVPSSSLGTQTYICNVNHKPSNTKVDKRVEPKS CDKTHTCPPCPAPEAAGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVYTLPPSREEMTKNQVSLTCLVKGF YPSDIAVEWESNGQPENNYKTTPPVLDSDGSFFLYSKLTVDKSRWQQGNV FSCSVMHEALHNHYTQKSLSLSPG | C0115, C0118 constant heavy chain CH1-CH3 L234A/L235A |
| 15 | EVQLQESGGGLVQPGGSLRLSCAASGFTFSIYSMNWVRQAPGKGLEWVS SIGSSGGTTYYADSVKGRFTISRDNSKNTLYLQMNSLRAEDTAVYYCAGSQ YLYGMDVWGQGTTVTVSSASTKGPSVFPLAPSSKSTSGGTAALGCLVKDY FPEPVTVSWNSGALTSGVHTFPAVLQSSGLYSLSSVVTVPSSSLGTQTYIC NVNHKPSNTKVDKRVEPKSCDKTHTCPPCPAPEAAGGPSVFLFPPKPKDT LMISRTPEVTCVVVDVSHEDPEVKFNWYVDGVEVHNAKTKPREEQYNSTY RVVSVLTVLHQDWLNGKEYKCKVSNKALPAPIEKTISKAKGQPREPQVYTL PPSREEMTKNQVSLTCLVKGFYPSDIAVEWESNGQPENNYKTTPPVLDSD GSFFLYSKLTVDKSRWQQGNVFSCSVMHEALHNHYTQKSLSLSPG | C0115, C0118 heavy chain |
| 16 | DIQMTQSPSSLSASVGDRVTITCRSSQRIMTYLNWYQQKPGKAPKLLIYVGS HSQSGVPSRFRGSGSETDFTLTISGLQPEDSATYYCQQSFFTPLTFGGGTK VEIKRTVAAPSVFIFPPSDEQLKSGTASVVCLLNNFYPREAKVQWKVDNALQ SGNSQESVTEQDSKDSTYSLSSTLTLSKADYEKHKVYACEVTHQGLSSPVT KSFNRGEC | C0115 light chain |
| 17 | DIQMTQSPSSLSASVGDRVTITCRSSQRILTYLNWYQQKPGKAPKLLIYVGS HSQSGVPSRFRGSGSETDFTLTISGLQSEDSATYYCQQSFFTPLTFGGGTK VEIKRTVAAPSVFIFPPSDEQLKSGTASVVCLLNNFYPREAKVQWKVDNALQ SGNSQESVTEQDSKDSTYSLSSTLTLSKADYEKHKVYACEVTHQGLSSPVT KSFNRGEC | C0118 light chain |
| 18 | | CL wt |
| 19 | RYTMH | humanized or murine OKT3 Blinatumumab Muromonab Tepliuzumab VH-CDR1 |
| 20 | GYGMH | Foralumab VH-CDR1 |
| 21 | SFPMA | Otelixizumab VH-CDR1 |
| 22 | YINPSRGYTNYNQKFKD | humanized or murine OKT3 Blinatumumab Muromonab Tepliuzumab VH-CDR2 |
| 23 | VIWYDGSKKYYVDSVKG | Foralumab VH-CDR2 |
| 24 | TISTSGGRTYYRDSVKG | Otelixizumab VH-CDR2 |
| 25 | YYDDHYCLDY | Blinatumumab Muromonab Tepliuzumab VH-CDR3 |
| 26 | QMGYWHFDL | Foralumab VH-CDR3 |
| 27 | FRQYSGGFDY | Otelixizumab VH-CDR3 |
| 28 | YYDDHYSLDY | humanized or murine OKT3 VH-CDR3 |
| 29 | RASSSVSYMN | Blinatumumab VL-CDR1 |
| 30 | SASSSVSYMN, | humanized or murine OKT3, Muromonab, Tepliuzumab VL-CDR1 |
| 31 | RASQSVSSYLA | Foralumab VL-CDR1 |
| 32 | TLSSGNIENNYVH | Otelixizumab VL-CDR1 |
| 33 | DTSKVAS | Blinatumumab VL-CDR2 |
| 34 | DTSKLAS | humanized or murine OKT3, Muromonab, Tepliuzumab VL-CDR2 |
| 35 | DASNRAT | Foralumab VL-CDR2 |
| 36 | DDDKRPD | Otelixizumab VL-CDR2 |
| 37 | QQWSSNPLT | Blinatumumab VL-CDR3 |
| 38 | QQWSSNPFT | Muromonab, Tepliuzumab VL-CDR3 |
| 39 | QQRSNWPPLT | Foralumab VL-CDR3 |
| 40 | TTGGTGTTTACGATGAACATCGG | gRNA to huMIF |
| 41 | QQWSSNP | humanized or murine OKT3 VL-CDR3 |
| 42 | EPCALCS | oxMIF epitope |
| 43 | EVQLLESGGGLVQPGGSLRLSCAASGFTFSIYSMNWVRQAPGKGLEWVSS IGSSGGTTYYADSVKGRFTISRDNSKNTLYLQMNSLRAEDTAVYYCAGSQY LYGMDVWGQGTTVTVSS | VH W97Y |
| 44 | ASTKGPSVFPLAPSSKSTSGGTAALGCLVKDYFPEPVTVSWNSGALTSGVH TFPAVLQSSGLYSLSSVVTVPSSSLGTQTYICNVNHKPSNTKVDKRVEPKS CDKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVYTLPPSREEMTKNQVSLTCLVKGF YPSDIAVEWESNGQPENNYKTTPPVLDSDGSFFLYSKLTVDKSRWQQGNV FSCSVMHEALHNHYTQKSLSLSPG | CH1-CH3 wt |
| 45 | GFTFNKYAMN | Pasotuxizumab, VH-CDR1 |
| 46 | RIRSKYNNYATYYADSVKD | Pasotuxizumab VH-CDR2 |
| 47 | VRHGNFGNSYISYWAY | Pasotuxizumab HC-CDR3 |
| 48 | GFTFSTYAMN | Cibisatamab VH-CDR1 |
| 49 | RIRSKYNNYATYYADSVKG | Cibisatamab VH-CDR2 |
| 50 | VRHGNFGNSYVSWFAY | Cibisatamab VH-CDR3 |
| 51 | NYYIH | Mosunetuzumab VH-CDR1 |
| 52 | WIYPGDGNTKYNEKFKG | Mosunetuzumab VH-CDR2 |
| 53 | DSYSNYYFDY | Mosunetuzumab VH-CDR3 |
| 54 | HSYVSSFNV | Otelixizumab VL-CDR3 |
| 55 | GSSTGAVTSGNYPN | Pasotuxizumab VL-CDR1 |
| 56 | GTKFLAP | Pasotuxizumab VL-CDR2 |
| 57 | VLWYSNRWV | Pasotuxizumab VL-CDR3 |
| 58 | GSSTGAVTTSNYAN | Cibisatamab VL-CDR1 |
| 59 | GTNKRAP | Cibisatamab VL-CDR2 |
| 60 | ALWYSNLWV | Cibisatamab VL-CDR3 |
| 61 | KSSQSLLNSRTRKNYLA | Mosunetuzumab VL-CDR1 |
| 62 | WASTRES | Mosunetuzumab Hz-679 VL-CDR2 |
| 63 | TQSFILRT | Mosunetuzumab VL-CDR3 |
| 64 | IYTMS | Hz-679 VH-CDR1 |
| 65 | TLSGDGDDIYYPDSVKG | Hz-679 VH-CDR2 |
| 66 | VRLGDWDFDV | Hz-679 VH-CDR3 |
| 67 | KSSQSLFNSRTRKNYLG | Hz-679 VL-CDR1 |
| 68 | TQVYYLCT | Hz-679 VL-CDR3 |
| 69 | EVQLQESGGGLVQPGGSLRLSCAASGFTFSIYSMNWVRQAPGKGLEWVS SIGSSGGTTYYADSVKGRFTISRDNSKNTLYLQMNSLRAEDTAVYYCAGSQ YLYGMDVWGQGTTVTVSSASTKGPSVFPLAPSSKSTSGGTAALGCLVKDY FPEPVTVSWNSGALTSGVHTFPAVLQSSGLYSLSSVVTVPSSSLGTQTYIC NVNHKPSNTKVDKKVEPKSCDKTHTCPPCPAPEAAGGPSVFLFPPKPKDTL MISRTPEVTCVVVDVSHEDPEVKFNWYVDGVEVHNAKTKPREEQYNSTYR VVSVLTVLHQDWLNGKEYKCKVSNKALPAPIEKTISKAKGQPREPQVYTLPP CRDELTKNQVSLWCLVKGFYPSDIAVEWESNGQPENNYKTTPPVLDSDGS FFLYSKLTVDKSRWQQGNVFSCSVMHEALHNHYTQKSLSLSPG | C0132 and C0133 heavy chain 1 |
| 70 | EVQLVESGGDLVKPGGSLKLSCAASGFTFSIYTMSWLRQTPGKGLEWVAT LSGDGDDIYYPDSVKGRFTISRDNAKNSLYLQMNSLRAEDTALYYCARVRL GDWDFDVWGQGTTVSVSSGGSGGSGGSGGSGGSDIVMTQSPSSLAVSP GERVTLTCKSSQSLFNSRTRKNYLGWYQQKPGQSPKLLIYWASTRESGVP DRFSGSGSGTDFTLTINSLQAEDVAVYYCTQVYYLCTFGAGTKLELKGGGG SDKTHTCPPCPAPEAAGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVCTLPPSRDELTKNQVSLSCAVKGF YPSDIAVEWESNGQPENNYKTTPPVLDSDGSFFLVSKLTVDKSRWQQGNV FSCSVMHEALHNHYTQKSLSLSPG | C0132 heavy chain 2 |
| 71 | EVQLQESGGGLVQPGGSLRLSCAASGFTFSIYSMNWVRQAPGKGLEWVS SIGSSGGTTYYADSVKGRFTISRDNSKNTLYLQMNSLRAEDTAVYYCAGSQ YLYGMDVWGQGTTVTVSSASTKGPSVFPLAPSSKSTSGGTAALGCLVKDY FPEPVTVSWNSGALTSGVHTFPAVLQSSGLYSLSSVVTVPSSSLGTQTYIC NVNHKPSNTKVDKKVEPKSCGGGGSGGGGSEVQLVESGGDLVKPGGSLK LSCAASGFTFSIYTMSWLRQTPGKGLEWVATLSGDGDDIYYPDSVKGRFTI SRDNAKNSLYLQMNSLRAEDTALYYCARVRLGDWDFDVWGQGTTVSVSS GGSGGSGGSGGSGGSDIVMTQSPSSLAVSPGERVTLTCKSSQSLFNSRTR KNYLGWYQQKPGQSPKLLIYWASTRESGVPDRFSGSGSGTDFTLTINSLQA EDVAVYYCTQVYYLCTFGAGTKLELKGGGGSGGGGSKTHTCPPCPAPEAA GGPSVFLFPPKPKDTLMISRTPEVTCVVVDVSHEDPEVKFNWYVDGVEVH NAKTKPREEQYNSTYRVVSVLTVLHQDWLNGKEYKCKVSNKALPAPIEKTIS KAKGQPREPQVCTLPPSRDELTKNQVSLSCAVKGFYPSDIAVEWESNGQP ENNYKTTPPVLDSDGSFFLVSKLTVDKSRWQQGNVFSCSVMHEALHNHYT QKSLSLSPG | C0133 heavy chain 2 |
| 72 | RSSQRIMTYLNWY | L-CDR1, wt |
| 73 | LLIFVASHSQS | L-CDR2, wt |
| 74 | QQSFWTPLT | L-CDR3, wt |
| 75 | SIYSMN | H-CDR1, wt |
| 76 | WVSSIGSSGGTTYYADSVKG | H-CDR2, wt |
| 77 | AGSQWLYGMDV | H-CDR3, wt |
| 78 | RSSQRILTYLNWY | L-CDR1 |
| 79 | LLI**Y**V**G**SHSQS | L-CDR2 |
| 80 | LLIFV**G**SHSQS | L-CDR2 |
| 81 | LLI**Y**VASHSQS | L-CDR2 |
| 82 | QQSF**F**TPLT | L-CDR3 |
| 83 | WV**G**SIGSSGGTTYYADSVKG | H-CDR2 |
| 84 | AGSQ**Y**LYGMDV | H-CDR3 |
| 85 | DIQMTQSPSSLSASVGDRVTITCRSSQRIMTYLNWYQQKPGKAPKLLIFVAS HSQSGVPSRFRGSGSETDFTLTISGLQPEDSATYYCQQSFFTPLTFGGGTK VEIK | VL W93F |

### Example 12: Efficacy of C0115 antibody in a collagen-II-induced arthritis (CIA) mouse model.

Material and Methods: DBA/1j (Harlan Laboratories, Italy) male mice aged between 8 to 9 weeks (weight range: 18-20 grams) were used in this study. Bovine type II Collagen (CII; Chondrex, USA) was dissolved at 2 mg/ml in 0.05M acetic acid by gently stirring overnight at 4°C. CFA (Complete Freund Adjuvant) was prepared by adding Mycobacterium tuberculosis H37Ra (Difco, Detroit, MI) at a concentration of 2 mg/ml to IFA (Incomplete Freund Adjuvant, Sigma Aldrich, Milano, Italy). Before injection CII was emulsified with an equal volume of CFA. To induce CIA, the mice were injected intradermally at the base of the tail with 100 µl (100 µg/mouse) of the obtained emulsion containing CII and CFA. On day 21, a second booster of 100 µl of CII (100 µg/mouse) in IFA was administered. At the onset of the disease (arthritic score ranging between 1 and 2), mice were treated twice a week (i.p.) for 20 days with vehicle, isotype control IgG1 (40 mg/kg), C0115 (at 20 mg/kg), or received daily injections of dexamethasone as standard-of-care drug (at 0.3 mg/kg). At the end of the treatment animals were euthanized and blood was collected, and tissues (front and hind paws) were harvested. Clinical severity of arthritis was assessed by monitoring of body weight, and paw thickness (of all four paws using a thickness gauge) two times a week. A paw thickness index was determined for each mouse by calculating the area under the curve (AUC) of the sum of the thickness for the 4 individual paws over the treatment. An arthritic score that ranges from 0 to 4 is assessed for the 4 paws of each mouse as: 0= no signs of arthritis; 1= swelling and/or redness of the paw or 1 digit; 2= involvement of 2 joints; 3= involvement of > 2 joints; 4= severe arthritis of the entire paw and digits, resulting in a maximum score per mouse of 12. A cumulative disease score was calculated for each mouse by summing the scores for the individual mice over the treatment period. Calculations were done in GraphPad Prism and Ordinary One-way ANOVA followed by Fisher's LSD test was used for statistical analysis.

Results and Conclusion: **Figure 10** reveals that treatment with C0115 at 20 mg/kg resulted in a significant amelioration of disease score (**A**) and paw oedema (**B**) compared to the vehicle-treated group. These effects (in particular, reduction of paw thickness) were comparable to the treatment with a high dose of standard-of-care corticosteroid drug dexamethasone (0.3 mg/kg). Isotype control (IgG)-treated group did not result in a reduced disease score. All treated groups had similar body weight variations during the disease course.

**Figure 10** shows that the newly designed Fc-silenced anti-oxMIF antibody C0115 ameliorates disease severity in a collagen II-induced DBA/1j mouse arthritis model. Cumulative disease score (**A**) and paw thickness (**B**) were assessed in a mouse arthritis model upon the treatment with C0115 (20 mg/ml), high dose dexamethasone (0.3 mg/kg) as a standard-of-care corticosteroid drug, or vehicle. Ordinary One-way ANOVA followed by Fisher's LSD test was used for statistical analysis in GraphPad Prism V 9.4 (*p<0.05; **p< 0.01; ***p< 0.001).

### Example 13: Efficacy of C0115 antibody in a glomerular nephritis (GN) rat model.

Material and Methods: The efficacy of the newly designed Fc silenced anti-oxMIF antibody C0115 was assessed in NTN in WKY rats. This model has a rapid onset of disease with macrophage infiltration, fibrin deposition and tissue destruction (Tam FWK. et al., 1999). The time course and morphology of this model is very similar to crescentic glomerulonephritis in human. The model is very robust, and all animals develop nephritis following induction by injection of nephrotoxic serum (NTS). Glomerular nephritis was induced by injecting WKY male rats (weight range: 190-220 grams) with 100 µl of rabbit anti-rat NTS (nephrotoxic serum) intravenously. At day 4 and day 6 post-NTS injection, animals were treated (i.p.) with vehicle, isotype control IgG1, or C0115 (at 30 mg/kg). Urine was collected at day 0 (baseline), 4 (onset of disease) and 7 (after treatment). At the end of the study (day 8) animals were euthanized and blood as well as tissues (kidneys, liver, spleen, and lung) were collected. Histological (Crescentic counts; ED-1 macrophage staining, rat and rabbit IgG deposit) and biochemical analysis (Proteinuria and Haematuria) were performed to assess the severity of the disease. Ordinary One-way ANOVA followed by Dunnett's correction for multiple testing was used for statistical analysis in GraphPad Prism V 9.4 (*p<0.05, **p< 0.01; ***p< 0.001, ****p< 0.0001).

Results and Conclusion: Treatment with C0115 (30 mg/kg) significantly ameliorated the disease, evidenced by reduced haematuria, proteinuria, and glomerular macrophage infiltration compared to vehicle-treated group (**Figure 11**). Treatment with isotype control IgG did not reduce haematuria when compared to the vehicle group.

**Figure 11** shows that the newly designed Fc-silenced anti-oxMIF antibody C0115 ameliorates disease severity in a nephrotoxic serum (NTS)-induced rat glomerulonephritis model. Haematuria in the urine (dipstick) (**A**), proteinuria (**B**), and glomerular macrophage infiltration (C) were assessed in a rat glomerulonephritis model upon the treatment of diseased rats with the anti-oxMIF antibody C0115, Isotype control IgG1 or vehicle. Mean ± SEM is shown, Ordinary One-way ANOVA followed by Dunnett's correction for multiple testing was used for statistical analysis in GraphPad Prism V 9.4 (*p<0.05, ***p< 0.001, ****p< 0.0001).

### Example 14: Binding of bispecific antibodies (bsMabs) C0132 and C0133 (anti-oxMIF x anti-HSG) to oxMIF.

Materials and Methods: Recombinant human MIF diluted in PBS at 1 µg/ml was immobilized into ELISA plates overnight at 4°C (transforming MIF to oxMIF according to Thiele et al., 2015). After blocking, serial dilutions of antibodies were added to the plates, and ELISA was performed as described in the Example 3.

Results and conclusion: The binding of C0132 and C0133 antibodies towards immobilized MIF (oxMIF) was measured over a broad range of concentrations and the resulting binding curves are illustrated in **Figure 13****.** The anti-oxMIF antibody C0008 was used as reference antibody for bivalent oxMIF binding. The binding curves and the calculated EC₅₀ values clearly showed that the bsMab C0133 having two anti-oxMIF arms, binds to oxMIF with an affinity similar to the C0008 (EC₅₀ = 242 pM (C0133), and 158 pM (C0008)), whereas the bsMab C0132, having only one anti-oxMIF arm, binds to oxMIF with higher EC₅₀ value (2508 pM) due to the loss of avidity effects.

**Figure 12****:** Schematic drawing of the newly designed Fc silenced anti-oxMIF x anti-HSG bispecific antibodies (bsMabs) C0132 (Fab/scFv-Fc) and C0133 (Fab/FabscFv-Fc).
**Figure 13** shows the binding curves of the newly designed Fc silenced anti-oxMIF x anti-HSG bsMabs C0132 and C0133 to immobilized oxMIF. Bound antibodies were detected by an anti-human-IgG (Fc)-HRP conjugate, C0008 was used as reference antibody for bivalent binding to oxMIF. Data were fitted to a sigmoidal, 4-parameter equation using GraphPad Prism (mean +/- SEM of two replicates is shown)

### Example 15: Differential binding of C0132 and C0133 anti-oxMIF x anti-HSG bsMabs to oxMIF vs. redMIF.

Materials and Methods: Anti-oxMIF x anti-HSG bsMabs C0132 and C0133, reference anti-oxMIF mAb C0008, and a human IgG1 isotype control were immobilized into microplates overnight at 4°C (15 nM for oxMIF bivalent and 30 nM for oxMIF monovalent antibodies). After blocking, wells were incubated with 50 ng/ml (~1,3 nM) of either redMIF or the oxMIF surrogate NTB-MIF (Schinagl et al., 2018). Captured oxMIF was detected with a polyclonal rabbit anti-MIF antibody and goat anti-rabbit IgG-HRP. Plates were stained with tetramethylbenzidine (TMB), and chromogenic reaction was stopped by addition of 30% H₂SO₄. OD was measured at 450 nm.

Results and conclusion: The binding of C0132 and C0133 towards soluble oxMIF and redMIF are illustrated in **Figure 14****.** The results clearly showed that both C0132 and C0133 demonstrated strong binding to oxMIF but no binding to redMIF. The newly designed antibodies showed very comparable OD values to the reference antibody C0008, which has been described to discriminate between oxMIF and redMIF (Thiele et al., 2015). No binding was observed for the isotype IgG. Thus, the anti-oxMIF x anti-HSG bsMabs C0132 and C0133 retained their ability to discriminate between oxMIF and redMIF.

**Figure 14****:** shows the differential binding of the newly designed Fc silenced anti-oxMIF x anti-HSG bsMabs C0132 and C0133 to oxMIF vs redMIF. C0008 was used as reference anti-oxMIF antibody. Mean and SEM of three replicates is shown.

### Example 16: Biodistribution of the newly designed Fc silenced anti-oxMIF x anti-HSG bsMabs C0132 and C0133 in Balb/c mice bearing syngenic CT26 colon tumors.

Materials and Methods: Biodistribution of newly designed Fc silenced anti-oxMIF x anti-HSG bsMabs C0132 and C0133 was investigated in female Balb/c mice carrying subcutaneous tumours of the mouse colon carcinoma CT26 cell line. Female Balb/c mice received unilateral, subcutaneous injections of 3x10⁶ CT26 cells in PBS in a total injection volume of 100 µl. Upon reaching individual tumor volumes of 150-300 mm³, mice were assigned to treatment groups and received a single intravenous dose of 5 mg/kg IRDye 800CW-labeled C0132 or C0133 or no treatment (control group).

C0132 and C0133 were labelled with IRDye 800CW using the IRDye 800CW Protein labeling kit (high MW from LI-COR Biosciences) following the manufacturer's instructions. After the labelling process and prior to injection of labeled antibodies into mice, the protein concentration and labeling efficiency of the IRDye 800CW-labeled antibodies was determined using the Nanodrop technology, and mice were dosed based on the protein concentration after labelling. *In vivo* imaging was performed in a LI-COR Pearl^{®} Trilogy imaging device performed at 785 nm excitation and 820 nm emission wavelength upon administration of labelled antibodies at the following time-points: 1h, 8h, 24h, 48h, 72h, 96h, 168h after dosing.

Results and Conclusion: **Figure 15** shows a significant intra-tumoral distribution of intravenously administered IRDye 800CW-labeled C0132 and C0133, respectively, with tumor retention up to 7 days. It is evident from **Figure 15** that one hour after injection and at all later imagining timepoints, the observed infrared signal in the tumours exceeded that of the background, indicating preferential accumulation of the antibodies in the tumour.

**Figure 15** shows the tumor penetration and retention of newly designed Fc silenced anti-oxMIF x anti-HSG bsMabs C0132 and C0133 assessed by infra-red *in vivo* imaging of mice carrying subcutaneous syngrafted CT26 tumors. Infra-red images of mice were taken 1h, 8h, 24h, 48h, 72h, 96h and 168h post injection of the IRDye 800CW labeled antibodies dosed at 5 mg/kg.

### Example 17: Pretargeted Radioimmunotherapy (PRAIT) using anti-oxMIF x anti-HSG bispecific antibodies (bsMabs) C0132 and C0133 and ¹⁷⁷Lu labeled IMP288 in a syngenic CT26 colon cancer mouse model in Balb/c mice.

In the present example, efficacy of the newly designed Fc silenced bispecific anti-oxMIF x anti-HSG antibodies C0132 and C0133 was evaluated using a PRAIT approach together with the HSG hapten IMP288 (as described in US2005/0025709) and ¹⁷⁷Lu as radionuclide. IMP288 is a DOTA-conjugated D-Tyr-D-Lys-D-Glu-D-Lys-NH2 tetrapeptide in which both lysine residues are derivatized with HSG moiety via their ε-aminogroup (**Figure 16A**). The DOTA chelating group of IMP288 is specifically designed for use with radiometals, including ¹⁷⁷Lu.

Materials and Methods: *In vivo* efficacy of the pre-RAIT was evaluated on Balb/c mice bearing subcutaneous CT26 murine colorectal carcinoma tumors.

IMP288 (Genepep, France) was labelled with ¹⁷⁷Lu at a specific activity of approximately 220 MBq/nmol while showing a radiochemical purity (RCP) ≥ 95%. Briefly, IMP288 was diluted with sterile water to a final concentration of 1 mM (1.45 mg/ml). A 1/10 dilution of IMP288 stock solution was performed in 2-(N-morpholino) ethanesulfonic acid (MES) buffer (500 mM, pH 5.5). 5mCi (185 MBq) of ¹⁷⁷LuCl₃ (EndolucinBeta^{®}), ITG, Germany) in 0.04 N HCl was placed into a radiolabelling vial, following addition of 40 µl of MES buffer (250mM, pH 5.5) and 0.84 nmol of diluted IMP288 solution (8.4 µl). The reaction mixture was incubated for 15 min at 95°C. After that, 5 µl of diethylenepenta-diaminetetraacetic acid sodium salt (DTPA-Na) at 10 mM was added to complex non-incorporated ¹⁷⁷Lu and the solution was diluted to 370 MBq/mL in 0.9%NaCl/ 10 g/L ascorbate/ 0.05% m/v BSA. The chelation efficiency and binding to C0132 and C0133 was analysed by instant Thin Layer Chromatography (iTLC, Agilent Technology, SGI001). Briefly, 50 µl of ¹⁷⁷Lu-IMP288 solution at 220 MBq/nmol and 1.68 nmol/ml corresponding to 0.084 nmol of IMP288 were mixed with 10-fold molar excess of each bsMab (0.84 nmol). Each reaction was incubated for 30 min at 37°C under gentle stirring. ¹⁷⁷Lu-IMP288 solution as well as ¹⁷⁷Lu-IMP288-bsMab solutions were applied to an iTLC strip and elution was done with 1:1 (v/v) solution of 0.15 M ammonium acetate (pH 5.5): MeOH. After elution, iTLC plates were exposed on a phosphorus screen (MS, BAS-IP, Fujifilm 2025) and revealed using Typhoon IP (Amersham) and associated software ImageQuant TL version 8.2.

CT26 murine colorectal carcinoma (ATCC-CRL-2638) cells were expanded in RPMI1640 medium supplemented with 10% heat-inactivated FBS and 2 mM glutamine in a 37°C/ 5% CO2. A suspension of CT26 cells was prepared to 10 x 10⁶ viable cells/mL in sterile PBS. Balb/c mice were subcutaneously injected in right flank with 1x10⁶ CT26 cells suspended in 100 µL of PBS. Once tumours reached 50-100 mm³ in volume (about 7-9 days post-inoculation) mice were randomized into treatment groups of 10 mice per group. Antibodies (C0132 or C0133) were injected intravenously at 5 mg/kg on the day of randomization (day -3). ¹⁷⁷Lu-IMP288 was administered 3 days after the injection of bispecific mAbs (day 0) by intravenous route at the molar ratio of bsMAb/HSGIMP288 hapten of 10:1 (~4 nmol/kg C0132 bsMAb or ~3 nmol/kg C0133 bsMab corresponding to 18.5 MBq respectively). Additionally, control groups received either ¹⁷⁷Lu-IMP288 at 37 MBq or vehicle on day 0 without any prior treatment. Tumor volumes and body weights were measured every 2-3 days until 21 days or until mice reached the humane endpoint of 1000 mm³ tumor volume, and percentage of survival was determined. Relative tumor volumes (tumor volume on day 0 = 100%), relative body weights (body weight on day 0 = 100%), and Kaplan-Meier survival curves (in percentage of survival) of each treatment group were plotted against time using GraphPrism software. Statistical analysis was performed in GraphPad Prism V9.4 using Ordinary one-way ANOVA with Dunnett's correction for multiple testing.

Results and Conclusion: **Figure 16** **B** reveals iTLC profiles for ¹⁷⁷Lu-IMP288 and ¹⁷⁷Lu-IMP288-bsMabs. As demonstrated in **Figure 16** **B,** a change in migration profile for ¹⁷⁷Lu-IMP288 was observed after incubation with the bsMabs C0132 and C0133, confirming that ¹⁷⁷Lu-IMP288 was bound by the bsMabs. Both bsMabs bound >94% of ¹⁷⁷Lu-IMP288 peptide calculated from the pixel intensities revealed by ImageQuant TL version 8.2.

As demonstrated in the **Figure 17A-B** PRAIT using Fc silenced anti-oxMIF x anti-HSG bsMab C0132 at 5 mg/kg and IMP288 at 18.5 MBq resulted in potent and significant tumour growth-inhibition (**Figure 17** **A**) persisting throughout the whole monitoring period of 21 days (100% survival, **Figure 17** **B**). Statistical analysis (Ordinary one-way ANOVA with Dunnett's correction for multiple testing; ** p<0.01) was performed for day 8 only, comparing C0132/¹⁷⁷Lu-IMP288-treated group to the ¹⁷⁷Lu-IMP288 group, because after day 8 nearly half of the animals in the vehicle group had to be sacrificed due to excessive tumour growth. Additionally, **Figure 17** **C** shows that PRAIT using C0132 was well-tolerated as it did not cause any substantial body weight loss throughout the whole monitoring period (21 days). As evident from **Figure 18****,** PRAIT with 5 mg/kg of the bsMab C0133 and IMP288 at 18.5 MBq resulted in potent and significant tumour growth-inhibition. Statistical analysis (Ordinary one-way ANOVA with Dunnett correction for multiple testing; *** p<0.001) was performed for day 8 only, comparing C0133/¹⁷⁷Lu-IMP288-treated group to the ¹⁷⁷Lu-IMP288 group, because after day 8 nearly half of the animals in the vehicle group had to be sacrificed due to excessive tumour growth. Summarized the results highlight the potential of Fc silenced anti-oxMIF x anti-HSG bsMabs together with radiolabeled HSG hapten as potent PRAITs.

**Figure 16****:** shows the structure of HSG hapten IMP288 (**A**) and (**B**) binding of bsMabs C0132 and C0133 to ¹⁷⁷Lu-IMP288 peptide assessed by iTLC based on change in migration profile of ¹⁷⁷Lu-IMP288 upon the incubation with the bsMabs when compared to the migration profile of ¹⁷⁷Lu-IMP288 peptide alone.

**Figure 17** shows PRAIT of syngrafted CT26 murine colorectal carcinomas in Balb/c mice with Fc silenced anti-oxMIF x anti-HSG bsMab C0132. C0132 was administered on day -3, followed by administration of ¹⁷⁷Lu-IMP288 3 days later (day 0). (**A**) Tumour volumes in % of tumour volumes measured on day 0; Mean ± SEM is shown (n= up to 10). Statistical analysis was performed in GraphPad Prism V9.4 using Ordinary one-way ANOVA with Dunnett's correction for multiple testing; ** p<0.01 vs. ¹⁷⁷Lu-IMP288; (**B**) Kaplan-Meier survival curves (in % survival),(C) body weights in % to body weights measured on day 0.

**Figure 18** shows PRAIT of syngrafted CT26 murine colorectal carcinomas in Balb/c mice with Fc silenced anti-oxMIF x anti-HSG bsMab C0133. C0133 was administered on day -3, followed by administration of ¹⁷⁷Lu-IMP288 3 days later (day 0). The figure shows tumour volumes in % of tumour volumes measured on day 0. Mean ± SEM is shown (n= up to 10). Statistical analysis was performed in GraphPad Prism V9.4 using Ordinary one-way ANOVA with Dunnett's correction for multiple testing; *** p<0.001 vs. ¹⁷⁷Lu-IMP288.

### Example 18: Pretargeted Radioimmunotherapy (PRAIT) using anti-oxMIF x anti-HSG bispecific antibody (bsMab) C0132 and ¹⁷⁷Lu-labeled IMP288 5 days after C0132 application in a syngenic CT26 colon cancer mouse model in Balb/c mice

In the present example, efficacy of the newly designed Fc-silenced bispecific anti-oxMIF x anti-HSG antibody C0132 was evaluated using a PRAIT approach together with the HSG hapten IMP288 (as described in US2005/0025709) and ¹⁷⁷Lu as radionuclide.

Materials and Methods: *In vivo* efficacy of the RRAIT was evaluated on Balb/c mice bearing subcutaneous CT26 murine colorectal carcinoma tumors. Study was conducted as described in the **Example 16** with some modifications. Briefly, Balb/c mice (Balb/c ByJ, Janvier Labs, France) were subcutaneously injected in right flank with 1x10⁶ CT26 cells suspended in 100 µL of PBS. The tumor growth was controlled by visual observation and palpation until day 7 post-inoculation. *In vivo* therapeutic study was initiated when tumor size reached about 100-200 mm³ for the 3 treatment groups (10 mice per group) and about 400-500 mm³ for 1 control group (10 mice per group). BsMab C0132 was injected i.v. at 2 doses: 2.5 mg/ml, and 5 mg/ml, whereas ¹⁷⁷Lu-labeled IMP288 was injected i.v. 5 days (*versus* 3 days in the **Example 16**) after the administration of C0132 (day 0). The molar ratio of C0132/radiolabeled HSG IMP288 hapten was kept constant at 10/1 (same as in **Example 16**), meaning that the ¹⁷⁷Lu-labeled IMP288 was dosed at 2 nmol/kg (9.3 MBq), and 4 nmol/kg (18.5 MBq), respectively. Control groups consisted of one cohort in which mice received ¹⁷⁷Lu-IMP288 at 8 nmol/kg (37 MBq) on day 0 without any prior treatment with bsMab. Over a period of 21 days post-dosing, the animal's body weight and the tumor volume were assessed every two-three days. The tumor volume was determined by measuring the length, the width and the depth of the tumor with a digital caliper. Tumor volume was calculated by using the following formula: Tumor volume = (length x width x depth) x 0.5. The mice were monitored until 21 days or until mice reached the predetermined experimental endpoints: tumor volume > 1500 mm³ or body weight loss > 20%. Relative tumor volumes (tumor volume on day 0 = 100%), and Kaplan-Meier survival curves (in percentage of survival) of each treatment group were plotted against time using GraphPad Prism software. Statistical analysis (Ordinary one-way ANOVA with Dunnett's correction for multiple testing, *p<0.05, **p< 0.01, ***p<0.001) was performed in using GraphPad Prism for days 8 and 10 only, comparing each of the 2 C0132-treated group to the control group (¹⁷⁷Lu-IMP288 alone, no bsMab), because after day 11 animals in the control group (¹⁷⁷Lu-IMP288, no bsMab) had to be euthanised due to excessive tumour growth.

Results and Conclusion: It is evident from **Figure 19** that pre-targeted therapy with C0132 at 2.5 and 5 mg/ml resulted in significant tumour regression (**Figure 19** **A**), and survival benefit (**Figure 19** **B**). The best survival (100%) persisting up to 21 days of monitoring could be achieved if C0132 was administered at 5 mg/ml.

**Figure 19** shows efficacy of anti-oxMIF x anti-HSG bsMab C0132 in Balb/c using a PRAIT approach in mice syngrafted with CT26 murine colorectal carcinoma cells. C0132 was administered at 2.5 mg/ml, and 5 mg/ml on day -5, followed by administration of ¹⁷⁷Lu-IMP288 5 days later (day 0). (**A**) Tumour volumes in % of tumour volumes measured on day 0, Mean ± SEM is shown (n= up to 10). Statistical analysis was performed in GraphPad Prism V9.4 using Ordinary one-way ANOVA with Dunnett's correction for multiple testing; *** p<0.001 vs. ¹⁷⁷Lu-IMP288; (**B**) Kaplan-Meier survival curves (in % survival).

### Example 19: Pretargeted Radioimmunotherapy (PRAIT) using anti-oxMIF x anti-HSG bispecific antibody (bsMab) C0132 and ¹⁷⁷Lu-labeled IMP288 5 days after C0132 application in a xenograft CFPAC-1 pancreatic cancer mouse model in Balb/c nude mice.

In the present example, efficacy of the newly designed Fc silenced bispecific anti-oxMIF x anti-HSG antibody C0132 was evaluated using a PRAIT approach together with the HSG hapten IMP288 (as described in US2005/0025709) and ¹⁷⁷Lu as radionuclide in a xenograft model of pancreatic cancer.
Materials and Methods: *In vivo* efficacy of the PRAIT was evaluated on Balb/c nude mice bearing subcutaneous CFPAC-1 ductal pancreatic adenocarcinoma tumors. Study was essentially conducted as described in the **Example 16** and **17** with some modifications. The human pancreatic ductal adenocarcinoma cells, CFPAC-1, were provided by ATCC (Cat# CRL-1918). Cells were cultured in RPMI 1640 medium supplemented with 10% FBS and 2mM L-Glutamine. A suspension of CFPAC-1 cells was prepared to 50 x 10⁶ cells/mL in sterile PBS. Balb/c nude mice (Balb/c ByAnNRj-Foxn1^{nu/nu}) were subcutaneously injected in right flank with 5x10⁶ CFPAC-1 cells suspended in 100 µL of PBS. The tumor growth was controlled by visual observation and palpation until day 4 post-inoculation. *In vivo* therapeutic study was initiated when tumor size reached about 150-300 mm³ for the treatment group (10 mice per group) and about 350-500 mm³ for the 2 control groups (10 mice per group). The treatment group first received 5 mg/kg (day -5) dose of BsMAb C0132. Five days later (day 0), the radiolabeled IMP288 was given using a fixed molar ratio of BsMAb/HSG hapten IMP288 of 10:1 meaning that the ¹⁷⁷Lu-labeled HSG hapten IMP288 was dosed at 4 nmol/kg (18.5 MBq), respectively. The control groups consisted of 1 cohort in which the mice received ¹⁷⁷Lu-IMP288 at 8 nmol/kg (37 MBq) but not the BsMAb, and 1 cohort in which the mice only received vehicle used for the dilution of ¹⁷⁷Lu-IMP288. Over a period of 28 days post-dosing, the animal's body weight and the tumor volume were assessed every two to three days. The tumor volume was determined by measuring the length, the width and the depth of the tumor with a digital caliper. Tumor volume was calculated by using the following formula: Tumor volume = (length x width x depth) x 0.5. The mice were monitored until 28 days or until mice reached the predetermined experimental endpoints: tumor volume > 1500 mm³ or body weight loss > 20%. Relative tumor volumes (tumor volume on day 0 = 100%) of each treatment group were plotted against time using GraphPad Prism software. Statistical analysis (Ordinary one-way ANOVA with Dunnett's correction for multiple testing; **p< 0.01) was performed in GraphPad Prism V9.4 for day 14 only, comparing the C0132-treated group to the vehicle group.

Results and Conclusion: It is evident from **Figure 20** that pre-targeted therapy with C0132 at 5 mg/ml resulted in significant tumour regression (**Figure 20**).

**Figure 20** shows efficacy of anti-oxMIF x anti-HSG bsMab C0132 using a PRAIT approach in Balb/c nude mice xenografted with CFPAC-1 pancreatic adenocarcinoma cells. C0132 was administered at 5 mg/ml on day -5, followed by administration of ¹⁷⁷Lu-IMP288 5 days later (day 0). Tumour volumes in % of tumour volumes measured on day 0; Mean ± SEM is shown (n= up to 10). Statistical analysis was performed in GraphPad Prism V9.4 using Ordinary one-way ANOVA with Dunnett's correction for multiple testing; ** p<0.01 vs. vehicle.

### Example 20: Efficacy of C0115 antibody in combination with glucocorticoids (GCs) in a mouse model of type II collagen induced arthritis (CIA).

Glucocorticoids (such as Dexamethasone) are potent immunosuppressive agents commonly used as a long-term therapy to control rheumatic diseases in human patients but associated with a variety of side effects. In the present example, the efficacy of C0115 was evaluated as stand-alone treatment, or in combination with dexamethasone.

Material and Methods: DBA/1j (Harlan Laboratories, Italy) male mice aged between 8 to 9 weeks (weight range: 18-20 grams) were used in this study. Bovine type II Collagen (CII; Chondrex, USA) was dissolved at 2 mg/ml in 0.05 M acetic acid by gently stirring overnight at 4°C. CFA (Complete Freund Adjuvant) was prepared by adding Mycobacterium tuberculosis H37Ra (Difco, Detroit, MI) at a concentration of 2 mg/ml to IFA (Incomplete Freund Adjuvant, Sigma Aldrich, Milano, Italy). Before injection, CII was emulsified with an equal volume of CFA. To induce CIA, the mice were injected intradermally at the base of the tail with 100 µl (100 µg/mouse) of the obtained emulsion containing CII and CFA. On day 21, a second booster of 100 µl of CII (100 µg/mouse) in IFA was administered. At disease onset, mice were treated twice a week (i.p.) for 20 days with: vehicle, C0115 (20 mg/kg) alone, or in combination with daily injections of low dose of dexamethasone (0.1 mg/kg). A control group of mice received daily injections of a high dose of dexamethasone (0.3 mg/kg) as standard-of-care drug. At the end of the treatment animals were euthanized and blood was collected, and tissues (front and hind paws) were harvested for further histological analysis. Clinical severity of arthritis was assessed by monitoring of body weight, and paw thickness (of all four paws using a thickness gauge) two times a week. An arthritic score that ranges from 0 to 4 was assessed for the 4 paws of each mouse as: 0= no signs of arthritis; 1= swelling and/or redness of the paw or 1 digit; 2= involvement of 2 joints; 3= involvement of > 2 joints; 4= severe arthritis of the entire paw and digits, resulting in a maximum score per mouse of 12. A cumulative disease score was calculated for each mouse by summing the scores for the individual mice over the treatment period. Statistical analysis was done in GraphPad Prism using Ordinary One-way ANOVA followed by Fisher's LSD test.

Results and Conclusion: **Figure 21** reveals that treatment with C0115 (20 mg/kg) resulted in a significant amelioration of clinical arthritis signs, indicated by cumulative disease score, compared to the vehicle-treated group. Moreover, combined treatment of C0115 (20mg/kg) and low dose of dexamethasone (0.1 mg/kg) resulted in further amelioration of clinical arthritis signs, compared to the treatment as single agents, and is comparable to the efficacy of a high dose of dexamethasone (0.3 mg/kg).

### Example 21: Efficacy of C0115 antibody and in combination with glucocorticoids (GCs) in a T cell transfer mouse model of colitis.

In the present example, efficacy of C0115 antibody as singe agent or in combination with a low dose of dexamethasone was evaluated during chronic intestinal inflammation.

Material and Methods: BALB/c and C.B-17 SCID (Envigo, San Pietro al Natisone, Udine, Italy) female mice aged between 8 to 9 weeks (weight range: 18-20 grams) were used in this study.

To induce colitis, CD4+CD25- T cells from BALB/c mice were transplanted in CB-17 SCID mice (lacking B cells and T cells). Briefly, spleenocytes isolated from Balb/C mice were *in vitro* stimulated with 4 µg/ml of Concanavalin A. T cells were isolated by magnetic selection of CD4+ CD25- cells and cell preparation was stained with a viability dye (7-actynomycin-D), FITC-conjugated anti-mouse CD4-antibody (BD, Heidelberg, Germany) and APC-conjugated anti-mouse CD25-antibody (BD, Heidelberg, Germany) for purity control ( >95% of viable T cells in CD4+CD25- gate) by flow cytometry using FACS Calibur (BD Bioscience, Heidelberg,German) and CellQuest software. Isolated CD4+CD25- T cells were injected i.p. into CB-17 SCID mice at a concentration of 500,000 cells in a final volume of 0.2 ml saline. One week after T cell transfer, mice were treated (twice a week; i.p.) for 83 days with: vehicle, C0115 (10 mg/kg) as singe agent or in combination with daily injections of a low dose of dexamethasone (0.01 mg/kg). A control group of mice received daily injections of high dose of dexamethasone (0.1 mg/kg) as standard-of-care drug. At the end of the treatment blood, stools and colon tissues were collected for further analysis. Clinical severity of colitis was assessed by periodic monitoring of body weight changes and stool consistency. A cumulative stool score was determined for each mouse by summing daily stool scores (0= well-formed pellet; 1= soft stool; 2= diarrhea). One-way ANOVA followed by Fisher's LSD test or was used for statistical analysis in GraphPad Prism V9.4.

Results and Conclusion: **Figure 22** indicates that treatment with C0115 (10 mg/kg) resulted in a significant amelioration of disease severity, evidenced by a significant reduction of cumulative stool score (**A**) compared to the vehicle-treated group and comparable to the high dose of dexamethasone (0.1 mg/kg). Combined treatment of C0115 at 10 mg/kg and low dose of dexamethasone (0.01 mg/kg) further ameliorated disease severity, evidenced by a further significant reduction of cumulative stool score. Remarkably, combined treatment of C0115 at 10 mg/kg and low dose of dexamethasone (0.01 mg/kg) resulted in further amelioration of colitis assessed by a significant body weight increase at the end of treatment (day 83) compared to the vehicle-treated group and the treatment with the standard-of-care corticosteroid drug dexamethasone.

**Figure 22** shows that the newly designed Fc-silenced anti-oxMIF antibody C0115, as single agent and in combination with GCs, ameliorates disease severity in a T cell transfer mouse of model colitis. Body weight change at the end of experiment D83 (**A**) as well as cumulative stool Score (**B**) were assessed upon the treatment with C0115 (10 mg/kg) alone or in combination with a low dose of 0.01 mg/kg of dexamethasone, dexamethasone at a low dose (0.01mg/kg) and at a high dose (0.1 mg/kg) as a standard-of-care corticosteroid drug, or vehicle control treatments. Data are presented as mean ± SEM and statistical analysis done using Ordinary One-way ANOVA followed by Fisher's LSD test (*p<0.05; **p< 0.01).

### Example 22: Reduced aggregation propensity and reduced hydrophobicity of newly designed anti-oxMIF antibodies and retention of their binding immobilized oxMIF (Ko estimation)

**Table 9: Variant IDs, sequence combinations and sequences of the anti-oxMIF antibodies used within the example 22. Amino acid substitutions in the VH and VL refer to SEQ ID NO: 3 and SEQ ID NO: 2 respectively.**

| **Construc t ID** | **VH** | **VL** | **CL** | **CH** |
|---|---|---|---|---|
| C0228 | W97Y | W93F | Seq ID 18 | Seq ID 14 |
| C0229 | L5Q/W97Y/S49G/P41S/ R83K/A84T | F49Y/A51G/W93F | Seq ID 18 | Seq ID 14 |
| C0230 | L5Q/W97Y | F49Y/A51G/W93F/R63S/E68G/G 77S /S83F | Seq ID 18 | Seq ID 14 |
| C0231 | L5Q/W97Y | F49Y/A51G/W93F/D1A/Q3R/L11 F/V15T | Seq ID 18 | Seq ID 14 |
| C0232 | L5Q/W97Y/S49G/P41S/ R83K/A84T | F49Y/A51G/W93F/R63S/E68G/G 77S/S83F | Seq ID 18 | Seq ID 14 |
| C0233 | L5Q/W97Y/S49G/P41S/ R83K/A84T | F49Y/A51G/W93F/D1A/Q3R/L11 F/V15T | Seq ID 18 | Seq ID 14 |
| C0234 | W97Y/G16R/S49A | W93F | Seq ID 18 | Seq ID 14 |
| C0235 | W97Y | W93F/S83F | Seq ID 18 | Seq ID 14 |
| C0236 | W97Y/L11A | W93F/L11F/V15T | Seq ID 18 | Seq ID 14 |

To evaluate hydrophobicity and aggregation, the newly designed antibodies are analyzed by gel filtration (SEC) and by hydrophobic interaction chromatography (HIC) in a head-to-head comparison to the control anti-oxMIF antibody C0008

For SEC, antibodies are diluted to 1 mg/ml in 1x phosphate buffered saline (1xPBS) and 100 µl sample are applied to an Enrich 650 (Bio-Rad) gel-filtration column at a flow rate of 1.25 ml/min. Separations and equilibration is performed in 1xPBS at room temperature. Protein peaks are monitored using absorbance at 280 nm and spectra are analyzed using the ChromLab software package (Bio-Rad). Results are reported in retention volume (Vr, ml) for the main peak and presence of aggregates is ranked manually.

For HIC analysis, antibodies are diluted to a final concentration of 1 mg/ml using 50 mM phosphate and 0.75 M ammonium sulphate, pH 6.8. 1 mg of each antibody is injected onto a 1ml HiTrap Butyl HP column using a 1 ml loading loop and the column flow rate is maintained at 1 ml/min at room temperature. Separation of peaks is carried out over a 20-column volume (CV) gradient from 0- 100% of the Buffer B (50 mM phosphate, 20% isopropanol; pH 7.0). Protein peaks are monitored using absorbance at 280 nm and spectra are analyzed using the Unicorn emulation software package (GE Healthcare). Results are reported in Vr (ml) at peak maximum, for each peak.

To evaluate binding (apparent affinity) to oxMIF, the newly designed antibodies are analyzed are analyzed by ELISA. Briefly, recombinant human MIF diluted in PBS at 1 µg/ml is immobilized into ELISA plates overnight at 4°C (transforming MIF to oxMIF according to Thiele et al., 2015). After blocking, serial dilutions of anti-oxMIF antibodies are added to the plates. Finally, bound antibodies are detected with Goat anti-human IgG (Fc)-HRP conjugate and tetramethylbenzidine (TMB) as substrate. The chromogenic reaction is stopped with 3M H₂SO₄ and OD is measured at 450 nm. EC₅₀ values representing the apparent affinity (K_{D}) are determined by 4-parameter fit using GraphPad Prism.

### REFERENCES

Aeberli D, et al. Endogenous macrophage migration inhibitory factor modulates glucocorticoid sensitivity in macrophages via effects on MAP kinase phosphatase-1 and p38 MAP kinase. FEBS Lett. 2006;580(3):974-981
Bacher M, et al. An essential regulatory role for macrophage migration inhibitory factor in T-cell activation. Proc Natl Acad Sci U S A. 1996;93(15):7849-7854
Bacher M, et al. Migration inhibitory factor expression in experimentally induced endotoxemia. Am J Pathol. 1997;150(1):235-246.
Bando, H., et al. (2002). Expression of macrophage migration inhibitory factor in human breast cancer: association with nodal spread. Jpn J Cancer Res 93, 389-396.
Barnes PJ, Adcock IM. How do corticosteroids work in asthma? Ann Intern Med. 2003;139(5 Pt 1):359-370
Bernhagen, J. et al. (1993). MIF is a pituitary-derived cytokine that potentiates lethal endotoxaemia. Nature 365, 756-759
Bernhagen, J., et al. (1994). Purification, bioactivity, and secondary structure analysis of mouse and human macrophage migration inhibitory factor (MIF). Biochemistry 33, 14144-14155
Bernhagen, J., et al. (2007). MIF is a noncognate ligand of CXC chemokine receptors in inflammatory and atherogenic cell recruitment. Nat Med 13, 587-596
Bloom B.R. and Bennet, B., 1966, Mechanism of a reaction in vitro associated with delayed-type hypersensitivity, Science 153, 80-82
Bozza FA, et al. Macrophage migration inhibitory factor levels correlate with fatal outcome in sepsis. Shock. 2004;22(4):309-313
Brinkmann U. and Kontermann R.E., 2017, The making of bispecific antibodies, MABS, 9, 2, 182-212
Bucala, R., and Donnelly, S. C. (2007). Macrophage migration inhibitory factor: a probable link between inflammation and cancer. Immunity 26, 281-285
Calandra T, et al. The macrophage is an important and previously unrecognized source of macrophage migration inhibitory factor. J Exp Med. 1994;179(6):1895-1902
Calandra T, et al. MIF as a glucocorticoid-induced modulator of cytokine production. Nature. 1995;377(6544):68-71
Chen, R., et al. (2010). Pilot study of blood biomarker candidates for detection of pancreatic cancer. Pancreas 39, 981-988
Chothia C., et al. (1987) Canonical structures for the hypervariable regions of immunoglobulins. J.Mol.Biol., 196, 901-17
Coleman, A. M., et al (2008). Cooperative regulation of non-small cell lung carcinoma angiogenic potential by macrophage migration inhibitory factor and its homolog, D-dopachrome tautomerase. J Immunol 181, 2330-2337
Conroy, H., et al. (2010). Inflammation and cancer: macrophage migration inhibitory factor (MIF)--the potential missing link. Qjm 103, 831-836
Coussens, L. M., and Werb, Z. (2002). Inflammation and cancer. Nature 420, 860-867
David, J.R., 1966, Delayed hypersensitivity in vitro: its mediation by cell-free substances formed by lymphoid cell-antigen interaction, Proc. Natl. Acad. Sci. U.S.A. 56, 72-77
de Jong et al., (2001). Development of chronic colitis is dependent on the cytokine MIF, Nat. Immunol. (11) 1061:6
del Rincón ID, et al. High incidence of cardiovascular events in a rheumatoid arthritis cohort not explained by traditional cardiac risk factors. Arthritis Rheum. 2001;44(12):2737-2745
Ehrenmann F., et al. 2010, MGT/3Dstructure-DB and IMGT/DomainGapAlign: a database and a tool for immunoglobulins or antibodies, T cell receptors, MHC, IgSF and MhcSF, Nucleic Acids Res. 38, D301-307
El-Magadmi M, et al. Systemic lupus erythematosus: an independent risk factor for endothelial dysfunction in women. Circulation. 2004;110(4):399-404.
Emonts M, et al. Association between high levels of blood macrophage migration inhibitory factor, inappropriate adrenal response, and early death in patients with severe sepsis. Clin Infect Dis. 2007;44(10):1321-1328
Estep P. et al., 2015, An alternative assay to hydrophobic interaction chromatography for high-throughput characterization of monoclonal antibodies, MAbs, 7(3), 553-561
Ewert S. et al., 2003, Structure-based improvement of the biophysical properties of immunoglobulin VH domains with a generalizable approach, Biochemistry, 18, 42(6), 1517-28
Fingerle-Rowson G, et al. Regulation of macrophage migration inhibitory factor expression by glucocorticoids in vivo. Am J Pathol. 2003;162(1):47-56
Ha J-H. et al., 2016, Immunoglobulin Fc heterodimer platform technology: From design to applications in therapeutic antibodies and proteins, frontiers Immunol., 7, article 394
Hagemann, T., and Balkwill, F. (2005). MIFed about cancer? Gastroenterology 129, 1785-1787.
Hagemann, T., et al. (2007). Ovarian cancer cell-derived migration inhibitory factor enhances tumor growth, progression, and angiogenesis. Mol Cancer Ther 6, 1993-2002
Hong, P., et al. (2012). A review size-exclusion chromatography for the analysis of protein biotherapeutics and their aggregates. In Journal of Liquid Chromatography and Related Technologies (Vol. 35, Issue 20, pp. 2923-2950). Taylor & Francis. https://doi.org/10.1080/10826076.2012.743724
Hudson, J. D., et al. (1999). A proinflammatory cytokine inhibits p53 tumor suppressor activity. J Exp Med 190, 1375-1382.
Hussain F. et al., 2013, Human anti-macrophage migration inhibitory factor antibodies inhibit growth of human prostate cancer cells in vitro and in vivo, Mol Cancer Ther. Jul;12(7):1223-34
lanello A. and Ahmad A., 2005, Role of antibody-dependent cell-mediated cytotoxicity in the efficacy of therapeutic anti-cancer monoclonal antibodies, Cancer and Metastasis Review, 24, 487-499
Kabat et al., Sequences of Proteins of Immunological Interest, Fifth Edition, NIH Publication 91-3242, Bethesda MD (1991), vols. 1-3
Kamimura, A., et al. (2000). Intracellular distribution of macrophage migration inhibitory factor predicts the prognosis of patients with adenocarcinoma of the lung. Cancer 89, 334-341
Karacay H. et al., (2005) Pretargeting with bispecifics aCEA x aHSG, Clin Cancer Res, 11(21)
Karin, M. (2009). NF-kappaB as a critical link between inflammation and cancer. Cold Spring Harb Perspect Biol 1, a000141
Kenanova V. et al. (2005) Tailoring the Pharmacokinetics and Positron Emission Tomography Imaging Properties of Anti-Carcinoembryonic Antigen Single-Chain Fv-Fc Antibody Fragments. Cancer Res. 2005, 65:622-633
Kessenbrock, K., et al. (2010). Matrix metalloproteinases: regulators of the tumor microenvironment. Cell 141, 52-67.
Lefranc MP. Unique database numbering system for immunogenetic analysis. Immunol Today (1997) 18:509.
Liu R. et al., 2020, "Fc-Engineering for modulated effector functions-improving antibodies for cancer treatment", Antibodies, 9, 64
Lue, H., et al. (2007). Macrophage migration inhibitory factor (MIF) promotes cell survival by activation of the Akt pathway and role for CSN5/JAB1 in the control of autocrine MIF activity. Oncogene 26, 5046-5059
MacCallum R.M et al. (1996) Antibody-antigen Interactions: Contact Analysis and Binding Site Topography, J.Mol.Biol., 262, 5:732-745
Mahalingam D. et al., 2015, ASCO Abstract ID2518
Mahalingam et al. 2020, Phase I study of Imalumab (BAX69), a fully human recombinant antioxidized macrophage migration inhibitory factor antibody in advanced solid tumours, Br J Clin Pharmacol. 86(9):1836-1848
Manzi S, et al. Prevalence and risk factors of carotid plaque in women with systemic lupus erythematosus. Arthritis Rheum. 1999;42(1):51-60
Meyer-Siegler, K., and Hudson, P. B. (1996). Enhanced expression of macrophage migration inhibitory factor in prostatic adenocarcinoma metastases. Urology 48, 448-452.
Mitchell RA, et al. Sustained mitogen-activated protein kinase (MAPK) and cytoplasmic phospholipase A2 activation by macrophage migration inhibitory factor (MIF). Regulatory role in cell proliferation and glucocorticoid action. J Biol Chem. 1999;274(25):18100-18106
Mitchell RA, et al. Macrophage migration inhibitory factor (MIF) sustains macrophage proinflammatory function by inhibiting p53: regulatory role in the innate immune response. Proc Natl Acad Sci U S A. 2002;99(1):345-350
Mitchell, R. A., and Bucala, R. (2000). Tumor growth-promoting properties of macrophage migration inhibitory factor (MIF). Semin Cancer Biol 10, 359-366.
Morand EF, et al. Macrophage migration inhibitory factor in rheumatoid arthritis: clinical correlations. Rheumatology (Oxford). 2002;41(5):558-562.
Morrison, S.L., et al., 1984, Chimeric human antibody molecules: mouse antigen-binding domains with human constant region domains, Proc. Natl. Acad. Sci. 81, 6851-6855
Natsume A. et al., 2009, "Improving effector functions of antibodies for cancer treatment: Enhancing ADCC and CDC", Drug, Design, development and Therapy, 3, 7-16
Niino M., et al. Macrophage migration inhibitory factor in the cerebrospinal fluid of patients with conventional and optic-spinal forms of multiple sclerosis and neuro-Behçet's disease. J Neurol Sci. 2000;179(S 1-2):127-131
Onodera S., et al. High expression of macrophage migration inhibitory factor in the synovial tissues of rheumatoid joints. Cytokine. 1999;11(2):163-167.
Petkova, S.B., et al., 2006, Enhanced half-life of genetically engineered human IgG1 antibodies in a humanized FcRn mouse model: potential application in humorally mediated autoimmune disease, Int'l. Immunol. 18(12) 1759-1769
Pisu M., et al. The cost of glucocorticoid-associated adverse events in rheumatoid arthritis. Rheumatology (Oxford). 2005;44(6):781-788
Pyzik M. et al. 2019. The Neonatal Fc Receptor (FcRn): A Misnomer?, Frontiers in Immunol., Vol 10, 1540: 1-24
Ramsey-Goldman R. Missed opportunities in physician management of glucocorticoid-induced osteoporosis? Arthritis Rheum. 2002;46(12):3115-3120
Ren, Y., et al. (2004). Upregulation of macrophage migration inhibitory factor contributes to induced N-Myc expression by the activation of ERK signaling pathway and increased expression of interleukin-8 and VEGF in neuroblastoma. Oncogene 23, 4146-4154
Rendon, B. E., et al. (2007). Regulation of human lung adenocarcinoma cell migration and invasion by macrophage migration inhibitory factor. J Biol Chem 282, 29910-29918
Rendon, B. E., et al. (2009). Mechanisms of macrophage migration inhibitory factor (MIF)-dependent tumor microenvironmental adaptation. Exp Mol Pathol 86, 180-185.
Rinta S., et al. Apoptosis-related molecules in blood in multiple sclerosis. J Neuroimmunol. 2008;205(1-2):135-141
Rossi EA et al., (2006) Stably tethered multifunctional structures of defined composition made by the dock and lock method for use in cancer targeting, PNAS, 103, 18, 6841-6846
Sampey AV, et al. (2001) Regulation of synoviocyte phospholipase A2 and cyclooxygenase 2 by macrophage migration inhibitory factor. Arthritis Rheum. 44(6):1273-1280.
Saunders K.O., (2019) "Conceptual approaches to modulating antibody effector functions and circulation half-life, frontiers Immunol., 10, 1-20, doi: 10.3389/fimmu.2019.01296
Sambrook et al, "Molecular Cloning: A Laboratory Manual" (4th Ed.), Vols. 1 -3, Cold Spring Harbor Laboratory Press (2012).
Sharkey RM et al. (2005) Pretargeting with bispecific aCD20 x aHSG, Leukemia, 15,1064-1069
Schäcke H., et al. (2002) Mechanisms involved in the side effects of glucocorticoids. Pharmacol Ther. 96(1):23-43
Schinagl, A., et al., (2018) Role of the Cysteine 81 Residue of Macrophage Migration Inhibitory Factor as a Molecular Redox Switch. Biochemistry, 57(9), 1523-1532. https://doi.org/10.1021/acs.biochem.7b01156
Schinagl. A. et al., (2016) Oxidized macrophage migration inhibitory factor is a potential new tissue marker and drug target in cancer, Oncotarget. Nov 8;7(45):73486-73496
Shimizu, T, et al. (1999). High expression of macrophage migration inhibitory factor in human melanoma cells and its role in tumor cell growth and angiogenesis. Biochem Biophys Res Commun 264, 751-758
Shimizu T., et al. (2001) High macrophage migration inhibitory factor (MIF) serum levels associated with extended psoriasis. J Invest Dermatol. 116(6):989-990
Solomon DH, et al. (2003) Cardiovascular morbidity and mortality in women diagnosed with rheumatoid arthritis. Circulation. 107(9):1303-1307
Spiess C. et al., (2015) Alternative molecular formats and therapeutic applications for bispecific antibodies, Mol.Immunol., 67, 95-106
Sprong T, et al. Macrophage migration inhibitory factor (MIF) in meningococcal septic shock and experimental human endotoxemia. Shock. 2007;27(5):482-487.
Strohl W.R. et al. (2012) Antibody Fc engineering for optimal antibody performance, Therapeutic Antibody Engineering, Woodhead Publishing Series in Biomedicine, No. 11, pp. 225-249
Takahashi, N., et al. (1998). Involvement of macrophage migration inhibitory factor (MIF) in the mechanism of tumor cell growth. Mol Med 4, 707-714.
Tam FWK, et al., (1999) Development of scarring and renal failure in a rat model of crescentic glomerulonephritis. Nephrol Dial Transplant 14:1658-1666
Tang J., et al., (2018) Comprehensive analysis of the clinical immuno-oncology landscape, Ann Oncol.;29(1):84-91
Thiele, M. et al., (2017). Selective Targeting of a Disease-Related Conformational Isoform of Macrophage Migration Inhibitory Factor Ameliorates Inflammatory Conditions. Journal of Immunology, 195(5), 2343-2352. https://doi.org/10.4049/jimmunol.1500572
Van der Kant, R. et al., (2017) Prediction and Reduction of the Aggregation of Monoclonal Antibodies, J.Mol.Biol., 429(8), 1244-1261
Verjans, E., et al. (2009). Dual role of macrophage migration inhibitory factor (MIF) in human breast cancer. BMC Cancer 9, 230
Walsh LJ, et al. (1996) Use of oral corticosteroids in the community and the prevention of secondary osteoporosis: a cross sectional study. BMJ. 313(7053):344-346
Wang P, et al., (2008) A systematic assessment of MHC class II peptide binding predictions and evaluation of a consensus approach, PLoS Comput Biol. 4(4):e1000048;
Wang P, et al., (2010) Peptide binding predictions for HLA DR, DP and DQ molecules, BMC Bioinformatics. 11:568
Wei Li et al., (2016) Antibody Aggregation: Insights from Sequence and Structure, Antibodies, 5(3), 19
WHO Drug Information, Vol. 28, No. 2, 2014
Willuda J. et al., (1999) High thermal stability is essential for tumor targeting of antibody fragments: engineering of a humanized anti-epithelial glycoprotein-2 (epithelial cell adhesion molecule) single-chain Fv fragment, Cancer Res., 15; 59(22), 5758-67
Winner, M., et al. (2007). Amplification of Tumor Hypoxic Responses by Macrophage Migration Inhibitory Factor-Dependent Hypoxia-Inducible Factor Stabilization. Cancer Research 67, 186-193

## Claims

1. An Fc silenced anti-oxMIF antibody, comprising a variant Fc region of a wild-type human IgG comprising SEQ ID NO:1 with one or more amino acid substitutions or glycosylation modifications, and the following variable domains:
(a1) a light chain variable domain comprising SEQ ID NO:2 with 1, 2, 3, 4, or 5 amino acid substitutions selected from M30L, F49Y, A51G, P80S, W93F, or
(a2) a light chain variable domain comprising SEQ ID NO:2 with 1, 2, 3, 4, or 5 amino acid substitutions selected from M30L, F49Y, A51G, P80S, W93F and with 1, 2, 3, 4, or 5 further amino acid substitutions, with the proviso that the
- conserved tyrosine at position 36 is preserved,
and
(b1) a heavy chain variable domain comprising SEQ ID NO:3; or
(b2) a heavy chain variable domain comprising SEQ ID NO:3 with 1 or 2 amino acid substitutions selected from L5Q and W97Y, or
(b3) a heavy chain variable domain comprising SEQ ID NO:3 with 1 or 2 amino acid substitutions selected from L5Q and W97Y, and 1, 2, 3, 4 or 5 further amino acid substitutions;
wherein amino acid positions are numbered according to Kabat,
wherein the variant Fc region exhibits decreased FcγR binding compared to the wildtype IgG1 Fc region, wherein the one or more amino acid substitutions in the variant Fc region are at any one of positions E233, L234, L235, G236, G237, P238, D265, S267, H268, N297, S298, T299, E318, L328, P329, A330, P331 of SEQ ID NO:1, specifically the amino acid substitutions are at positions L234 and L235, according to EU numbering index, and/or wherein the Fc region is aglycosylated , and
wherein said antibody has reduced aggregation potential and reduced hydrophobicity compared to an antibody comprising SEQ ID NO:2 and SEQ ID NO:3 lacking the amino acid substitutions.

2. The Fc-silenced anti-oxMIF antibody of claim 1, wherein the light chain variable domain comprises the amino acid substitution W93F and the heavy chain variable region comprises the amino acid substitution W97Y.

3. The Fc silenced anti-oxMIF antibody of claim 1 or 2, comprising variable domains comprising an amino acid sequence selected from the group consisting of SEQ ID NOs:4, 5, 6, 7, 8, 9, and 43.

4. The Fc-silenced anti-oxMIF antibody of claims 1 to 3 comprising
i.) SEQ ID NOs:3 and 6,
ii.) SEQ ID NOs:9 and 6,
iii.) SEQ ID NOs:4 and 6,
iv.) SEQ ID NOs:4 and 8,
v.) SEQ ID NOs:4 and 5, or
vi.) SEQ ID NO:43 and any one of SEQ ID NOs:5, 6 or 8.

5. The Fc-silenced anti-oxMIF antibody of claim 4, further comprising SEQ ID NO:14.

6. The Fc-silenced anti-oxMIF antibody of any one of claims 1 or 2, comprising an amino acid sequence selected from the group consisting of SEQ ID NOs:15 and 16 or of SEQ ID NO 15 and 17.

7. The Fc-silenced anti-oxMIF antibody of any one of claims 1 to 6, selected from the group consisting of bispecific antibodies, scFv-Fc, (scFv)₂-Fc, scFv/scFv-Fc, Fab/scFv-Fc, Fab/(scFv)₂-Fc, Fab/Fab-scFv-Fc, Fab/Fab-crossFab-Fc, IgG-scFv and IgG-(scFv)₂.

8. The Fc-silenced anti-oxMIF antibody of any one of claims 1 to 7, wherein said antibody is a bispecific antibody, further comprising at least one binding site specifically recognizing an epitope of CD3 or histamine-succinyl-glycine (HSG).

9. The Fc-silenced antibody of any one of claims 1 to 8, for use in the preparation of a medicament.

10. A pharmaceutical composition comprising the antibody of any one of claims 1 to 8, optionally together with a pharmaceutical carrier or adjuvant.

11. The pharmaceutical composition of claim 10, wherein said pharmaceutical composition is formulated for subcutaneous administration.

12. The pharmaceutical composition of claim 10 or 11, wherein the composition is for administration as the sole substance, or for administration with further active substances, preferably selected from the group consisting of antiviral, anti-cancer, anti-inflammatory, and antibiotic substances.

13. The pharmaceutical composition of any one of claims 10 to 12 for use in the treatment of a patient suffering from inflammatory disease, infectious disease, specifically in the treatment of asthma, vasculitis, arthritis, sepsis, septic shock, endotoxic shock, toxic shock syndrome, acquired respiratory distress syndrome, glomerulonephritis, inflammatory bowel disease, Crohn's disease, ulcerative colitis, peritonitis, nephritis, NASH (nonalcoholic steatosis hepatitis), multiple sclerosis, acute and chronic pancreatitis, Type 1 diabetes, IgA nephropathy, interstitial cystitis, post COVID syndrome and psoriasis, or for use in the treatment of a patient suffering from hyperproliferative disorder or cancer, specifically in the treatment of colorectal cancer, ovarian cancer, breast cancer, prostate cancer, pancreas cancer, gastric cancer, and lung cancer.

14. An isolated nucleic acid encoding the antibody of any one of claims 1 to 8.

15. An expression vector comprising the nucleic acid of claim 14.

## Patentansprüche

1. Fc-stummgeschalteter Anti-oxMIF-Antikörper, umfassend eine Variante der Fc-Region eines menschlichen IgG vom Wildtyp, umfassend SEQ ID NO1 mit einer oder mehreren Aminosäure-Substitutionen oder Glykosylierungsmodifikationen, und die folgenden variablen Domänen
(a1) eine variable Domäne der leichten Kette umfassend SEQ ID NO:2 mit 1, 2, 3, 4 oder 5 Aminosäure-Substitutionen ausgewählt aus M30L, F49Y, A51G, P80S und W93F, oder
(a2) eine variable Domäne der leichten Kette umfassend SEQ ID NO:2 mit 1, 2, 3, 4 oder 5 Aminosäure-Substitutionen ausgewählt aus M30L, F49Y, A51G, P80S und W93F, und mit 1, 2, 3, 4 oder 5 weiteren Aminosäure-Substitutionen, mit der Maßgabe, dass das
- konservierte Tyrosin an Position 36 erhalten wird,
und
(b1) eine variable Domäne der schweren Kette umfassend SEQ ID NO:3; oder
(b2) eine variablen Domäne der schweren Kette umfassend SEQ ID NO:3 mit 1 oder 2 Aminosäure-Substitutionen ausgewählt aus L5Q und W97Y, oder (b3) einer variablen Domäne der schweren Kette umfassend SEQ ID NO:3 mit 1 oder 2 Aminosäure-Substitutionen ausgewählt aus L5Q und W97Y, und mit 1, 2, 3, 4 oder 5 weiteren Aminosäure-Substitutionen;
wobei die Aminosäure-Positionen nach Kabat nummeriert sind,
wobei die Variante der Fc-Region eine verminderte FcyR-Bindung verglichen mit der Fc-Region der Wildtyp-IgG1 zeigt, wobei die eine oder die mehreren Aminosäure-Substitutionen in der Variante der Fc-Region an einer beliebigen der Positionen E233, L234, L235, G236, G237, P238, D265, S267, H268, N297, S298, T299, E318, L328, P329, A330, P331 von SEQ ID NO1 vorliegen, im Speziellen die Aminosäure-Substitutionen an den Positionen L234 und L235 vorliegen, nach dem EU-Nummerierungs-Index, und/oder wobei die Fc-Region nicht glykosyliert ist, und wobei der Antikörper ein reduziertes Aggregationspotential und reduzierte Hydrophobizität verglichen mit einem Antikörper umfassend SEQ ID NO2 und SEQ ID NO:3 aufweist, welchen die Aminosäure-Substitutionen fehlen.

2. Fc-stummgeschalteter anti-oxMIF-Antikörper nach Anspruch 1, wobei die variable Domäne der leichten Kette die Aminosäure Substitution W93F umfasst und die variable Region der schweren Kette die Aminosäure Substitution W97Y umfasst.

3. Fc-stummgeschalteter anti-oxMIF-Antikörper nach Anspruch 1 oder 2, umfassend variable Domänen umfassend eine Aminosäuresequenz ausgewählt aus der Gruppe bestehend aus SEQ ID NO:4, 5, 6, 7, 8, 9 und 43.

4. Fc-stummgeschalteter anti-oxMIF-Antikörper nach den Ansprüchen 1 bis 3 umfassend
i.) SEQ ID NO:3 und 6,
ii.) SEQ ID NO:9 und 6,
iii.) SEQ ID NO:4 und 6,
iv.) SEQ ID NO:4 und 8,
v.) SEQ ID NO:4 und 5
vi.) SEQ ID NO:43 und eine beliebige der SEQ ID NO:5, 6 oder 8.

5. Fc-stummgeschalteter anti-oxMIF-Antikörper nach Anspruch 4, ferner umfassend SEQ ID NO:14.

6. Fc-stummgeschalteter anti-oxMIF-Antikörper nach einem der Ansprüche 1 oder 2, umfassend eine Aminosäuresequenz ausgewählt aus der Gruppe bestehend aus SEQ ID NO:15 und 16 oder aus SEQ ID NO:15 und 17.

7. Fc-stummgeschalteter anti-oxMIF-Antikörper nach einem der Ansprüche 1 bis 6, ausgewählt aus der Gruppe bestehend aus bispezifischen Antikörpern, scFv-Fc, (scFv)z-Fc, scFvscFv-Fc, FabscFv-Fc, Fab(scFv)₂-Fc, FabFab-scFv-Fc, FabFabcrossFab-Fc, IgG-scFv und IgG-(scFv)₂.

8. Fc-stummgeschalteter anti-oxMIF-Antikörper nach einem der Ansprüche 1 bis 7, wobei der Antikörper ein bispezifischer Antikörper ist, ferner umfassend zumindest eine Bindungsstelle, die spezifisch ein Epitop von CD3 oder Histamin-Succinyl-Glycin (HSG) bindet.

9. Fc-stummgeschalteter Antikörper nach einem der Ansprüche 1 bis 8 zur Verwendung bei der Herstellung eines Medikaments.

10. Pharmazeutische Zusammensetzung umfassend den Antikörper nach einem der Ansprüche 1 bis 8, gegebenenfalls zusammen mit einem pharmazeutischen Träger oder Adjuvans.

11. Pharmazeutische Zusammensetzung nach Anspruch 10, wobei die pharmazeutische Zusammensetzung für die subkutane Verabreichung formuliert ist.

12. Pharmazeutische Zusammensetzung nach Anspruch 10 oder 11, wobei die Zusammensetzung zur Verabreichung als einzige Substanz oder zur Verabreichung mit weiteren Wirkstoffen, vorzugsweise ausgewählt aus der Gruppe bestehend aus antiviralen, Antikrebs-, entzündungshemmenden und antibiotischen Substanzen, bestimmt ist.

13. Pharmazeutische Zusammensetzung nach einem der Ansprüche 10 bis 12 zur Verwendung bei der Behandlung eines Patienten, der an einer entzündlichen Krankheit, einer Infektionskrankheit leidet, insbesondere bei der Behandlung von Asthma, Vaskulitis, Arthritis, Sepsis, septischem Schock, endotoxischem Schock, toxischem Schocksyndrom, akutem Lungenversagen (ARDS), Glomerulonephritis, entzündlicher Darmerkrankung, Morbus Crohn, Colitis ulcerosa, Peritonitis, Nephritis, NASH (nichtalkoholische Steatosehepatitis), multipler Sklerose, akuter und chronischer Pankreatitis, Typ-1-Diabetes, IgA-Nephropathie, interstitieller Zystitis, Post-COVID-Syndrom und Psoriasis, oder zur Verwendung in der Behandlung eines Patienten, der an einer hyperproliferativen Störung oder Krebs leidet, im Speziellen in der Behandlung von Dickdarmkrebs, Ovarialkrebs, Brustkrebs, Prostatakrebs, Bauchspeicheldrüsenkrebs, Magenkrebs und Lungenkrebs.

14. Isolierte Nukleinsäure, die für den Antikörper nach einem der Ansprüche 1 bis 8 kodiert.

15. Expressionsvektor, umfassend die Nukleinsäure nach Anspruch 14.

## Revendications

1. Anticorps anti-oxMIF inactivé par Fc, comprenant une région Fc variante d'une IgG humaine de type sauvage comprenant la SEQ ID N° : 1 avec une ou plusieurs substitutions d'acide aminé ou modifications de glycosylation, et les domaines variables suivants :
(a1) un domaine variable de chaîne légère comprenant la SEQ ID N° : 2 avec 1, 2, 3, 4 ou 5 substitutions d'acide aminé choisies parmi M30L, F49Y, A51G, P80S, W93F, ou
(a2) un domaine variable de chaîne légère comprenant la SEQ ID N° : 2 avec 1, 2, 3, 4 ou 5 substitutions d'acide aminé choisies parmi M30L, F49Y, A51G, P80S, W93F et avec 1, 2, 3, 4 ou 5 substitutions d'acide aminé supplémentaires, à condition que la tyrosine conservée au niveau de la position 36 soit préservée,
et
(b1) un domaine variable de chaîne lourde comprenant la SEQ ID N° : 3 ; ou
(b2) un domaine variable de chaîne lourde comprenant la SEQ ID N° : 3 avec 1 ou 2 substitutions d'acide aminé choisies parmi L5Q et W97Y, ou
(b3) un domaine variable de chaîne lourde comprenant la SEQ ID N° : 3 avec 1 ou 2 substitutions d'acide aminé choisies parmi L5Q et W97Y, et 1, 2, 3, 4 ou 5 substitutions d'acide aminé supplémentaires ;
dans lequel les positions des acides aminés sont numérotées selon Kabat,
dans lequel la région Fc variante présente une liaison de FcγR réduite comparée à la région Fc de l'IgG1 de type sauvage, dans lequel les une ou plusieurs substitutions d'acide aminé dans la région Fc variante se trouvent au niveau d'une quelconque des positions E233, L234, L235, G236, G237, P238, D265, S267, H268, N297, S298, T299, E318, L328, P329, A330, P331 de la SEQ ID N° : 1, plus particulièrement les substitutions d'acide aminé se trouvent au niveau des positions L234 et L235, selon l'index de numérotation EU, et/ou dans lequel la région Fc est aglycosylée, et
dans lequel ledit anticorps présente un potentiel d'agrégation réduit et une hydrophobicité réduite comparé à un anticorps comprenant la SEQ ID N° : 2 et la SEQ ID N° : 3 dépourvues des substitutions d'acide aminé.

2. Anticorps anti-oxMIF inactivé par Fc selon la revendication 1, dans lequel le domaine variable de chaîne légère comprend la substitution d'acide aminé W93F et la région variable de chaîne lourde comprend la substitution d'acide aminé W97Y.

3. Anticorps anti-oxMIF inactivé par Fc selon la revendication 1 ou 2, comprenant des domaines variables comprenant une séquence d'acides aminés choisie dans le groupe constitué par les SEQ ID N° : 4, 5, 6, 7, 8, 9 et 43.

4. Anticorps anti-oxMIF inactivé par Fc selon les revendications 1 à 3 comprenant
i.) les SEQ ID N° : 3 et 6,
ii.) les SEQ ID N° : 9 et 6,
iii.) les SEQ ID N° : 4 et 6,
iv.) les SEQ ID N° : 4 et 8,
v.) les SEQ ID N° : 4 et 5, ou
vi.) la SEQ ID N° : 43 et l'une quelconque des SEQ ID N° : 5, 6 ou 8.

5. Anticorps anti-oxMIF inactivé par Fc selon la revendication 4, comprenant en outre la SEQ ID N° : 14.

6. Anticorps anti-oxMIF inactivé par Fc selon l'une quelconque des revendications 1 ou 2, comprenant une séquence d'acides aminés choisie dans le groupe constitué par les SEQ ID N° : 15 et 16 ou par les SEQ ID N° : 15 et 17.

7. Anticorps anti-oxMIF inactivé par Fc selon l'une quelconque des revendications 1 à 6, choisi dans le groupe constitué par les anticorps bispécifiques, scFv-Fc, (scFv)₂-Fc, scFv/scFv-Fc, Fab/scFv-Fc, Fab/(scFv)₂-Fc, Fab/Fab-scFv-Fc, Fab/Fab-crossFab-Fc, IgG-scFv et IgG-(scFv)₂.

8. Anticorps anti-oxMIF inactivé par Fc selon l'une quelconque des revendications 1 à 7, dans lequel ledit anticorps est un anticorps bispécifique, comprenant en outre au moins un site de liaison reconnaissant spécifiquement un épitope de CD3 ou une histamine-succinyl-glycine (HSG).

9. Anticorps inactivé par Fc selon l'une quelconque des revendications 1 à 8, destiné à être utilisé dans la préparation d'un médicament.

10. Composition pharmaceutique comprenant l'anticorps selon l'une quelconque des revendications 1 à 8, éventuellement conjointement avec un véhicule ou excipient pharmaceutique.

11. Composition pharmaceutique selon la revendication 10, dans laquelle ladite composition pharmaceutique est formulée pour une administration sous-cutanée.

12. Composition pharmaceutique selon la revendication 10 ou 11, dans laquelle la composition est destinée à être administrée en tant que seule substance, ou à être administrée avec des substances actives supplémentaires, de préférence choisies dans le groupe constitué par les substances antivirales, anti-cancéreuses, anti-inflammatoires et antibiotiques.

13. Composition pharmaceutique selon l'une quelconque des revendications 10 à 12 destinée à être utilisée dans le traitement d'un patient souffrant d'une maladie inflammatoire, d'une maladie infectieuse, plus particulièrement dans le traitement de l'asthme, la vasculite, l'arthrite, la septicémie, un choc septique, un choc endotoxique, le syndrome de choc toxique, le syndrome de détresse respiratoire acquise, la glomérulonéphrite, une maladie inflammatoire de l'intestin, la maladie de Crohn, la colite ulcéreuse, la péritonite, la néphrite, la NASH (stéato-hépatite non alcoolique), la sclérose en plaques, la pancréatite aiguë et chronique, le diabète de type 1, la néphropathie à IgA, la cystite interstitielle, le syndrome post-COVID et le psoriasis, ou destinée à être utilisée dans le traitement d'un patient souffrant d'un trouble d'hyperprolifération ou d'un cancer, plus particulièrement dans le traitement du cancer colorectal, du cancer de l'ovaire, du cancer du sein, du cancer de la prostate, du cancer du pancréas, du cancer de l'estomac et du cancer du poumon.

14. Acide nucléique isolé codant l'anticorps selon l'une quelconque des revendications 1 à 8.

15. Vecteur d'expression comprenant l'acide nucléique selon la revendication 14.
